# EUROPEAN PATENT APPLICATION

(11) **EP 4 273 158 A1**
(43) Date of publication of application: **08.11.2023**
(21) Application number: 21909464.6
(22) Date of filing: 22.12.2021
(51) Int. Cl.: C07K 14/54, A61K 47/60, C12P 21/02

(54) **INTERLEUKIN-21 MUTANT AND USE THEREOF**

(30) Priority: 23.12.2020 CN 202011538746
(71) Applicant: Innovent Biologics (Suzhou) Co., Ltd., Suzhou, Jiangsu 215123 (CN)
(72) Inventor: FU, Fenggen, Suzhou, Jiangsu 215123 (CN); ZHOU, Shuaixiang, Suzhou, Jiangsu 215123 (CN); HAN, Hongya, Suzhou, Jiangsu 215123 (CN)
(74) Representative: Sagittarius IP
(86) International application number: PCT/CN2021/140451
(87) International publication number: WO 2022/135469

(57) **Abstract**

The present invention relates to a novel interleukin-21 (IL-21) mutant protein and use thereof. In particular, the present invention relates to an IL-21 mutant protein that has improved properties, such as a reduced binding property to an IL-21 receptor and improved druggability, compared to wild-type IL-21. The present invention also provides a fusion comprising the IL-21 mutant protein, a nucleic acid encoding the IL-21 mutant protein or the fusion, and a vector and a host cell comprising the nucleic acid. The present invention further provides a method for preparing the IL-21 mutant protein or the fusion, a pharmaceutical composition comprising the same, and therapeutic use.

## Description

The present invention relates to a novel interleukin-21 (IL-21) mutant protein and use thereof. In particular, the present invention relates to an IL-21 mutant protein that has improved properties, such as a reduced binding property to an IL-21 receptor and improved druggability, compared to wild-type IL-21. The present invention also provides a fusion comprising the IL-21 mutant protein, a nucleic acid encoding the IL-21 mutant protein or the fusion, and a vector and a host cell comprising the nucleic acid. The present invention further provides a method for preparing the IL-21 mutant protein or the fusion, a pharmaceutical composition comprising the same, and therapeutic use.

### BACKGROUND

Interleukin-21 (IL-21) is a T cell-derived pleiotropic cytokine that regulates the activity of innate and adaptive immune cells, which is a type I cytokine and a member of the common cytokine receptor γ chain (cg chain) family of cytokines.

IL-21 has a four-helix bundle structure and exists as a monomer. In humans, two isomers of IL-21 are known, each derived from a precursor molecule. The first IL-21 isomer contains 162 amino acids (aa), of which the first 29 amino acids constitute a signal peptide; and the second IL-21 isomer contains 153aa, of which the first 29 amino acids constitute a signal peptide as in the first isomer.

IL-21 is produced by activated CD4 T cells and natural killer T (NKT) cells. IL-21 binds to a heterodimeric IL-21 receptor (IL-21R) expressed on the surface of T cells, B cells, and NK cells. When IL-21 binds to IL-21R, the Jak/STAT signaling pathway is activated, thereby activating a target gene. The structure of IL-21R is similar to that of the IL-2 and IL-15 receptors in that each of these cytokine receptors contains a common γ chain (γc). In addition to γc, IL-21R contains an α chain that is important for binding to IL-21. IL-21R is widely expressed in hematopoietic cells including T and B lymphocytes, natural killer (NK) cells, and bone marrow cells.

In dendritic cells (DCs), IL-21 can inhibit DC maturation and activation, induce apoptosis in conventional DCs, potently inhibit activation of T cells in mixed cultures, and play a role in the induction of tolerance. IL-21 is a potent mitogen and survival factor for NK cells and activated T cells, but is not an essential growth or differentiation factor. IL-21 can also support the differentiation of CD4(+) T helper 17 (Th17) cells and follicular helper T (Tfh) cells and counteract the differentiation of regulatory T (Treg) cells. In addition, IL-21 can enhance the survival of CD8 T cells and protect a less activated but more durable T cell phenotype, allowing for enhanced tumor and viral control.

Since IL-21 plays a key role in anti-tumor and anti-viral responses, it has become an attractive target for several therapies, in addition to playing a major role in inflammatory responses that lead to the development of autoimmune and inflammatory diseases.

The pleiotropic nature of IL-21 makes it a potential therapeutic target. However, since the IL-21 receptor (IL-21R) is widely expressed on a variety of cells including T cells, B cells, NK cells, and bone marrow cells, the molecule is not selective for cells such as T cells and can activate T cells in large numbers in the periphery, resulting in high molecular toxicity. Thus, its toxicity must be carefully controlled.

IL-21 itself is difficult to purify, and the purity of the wild-type molecule by one-step SEC purification is only 61%. Furthermore, since IL-21 has high affinity for IL-21R, up to 8.29×10⁻¹⁰ M, it has a very short half-life *in vivo,* and requires effective engineering to increase the half-life. In addition, the wild-type IL-21 is expressed after being fused to Fc, with very poor druggability.

Some engineering solutions for the IL-21 molecule have been proposed in the art. For example, WO2019028316A1 proposes an engineered interleukin-21 mutant protein with reduced affinity, and a fusion thereof with a PD-1 antibody. The weakened IL-21 has a significantly prolonged half-life in mice, but there is no mention of improving the druggability of the molecule.

In view of the above problems associated with IL-21 immunotherapy as well as production and purification, there remains a need in the art to further develop a novel IL-21 molecule with improved properties, particularly an IL-21 molecule that is advantageous to production and purification and has improved pharmacokinetic and pharmacodynamic properties, and a fusion thereof in combination with an antibody.

### SUMMARY

The present invention relates to a novel interleukin-21 (IL-21) mutant protein and use thereof. In particular, the present invention relates to an IL-21 mutant protein that has improved properties, such as a reduced binding property to an IL-21 receptor and improved druggability, compared to wild-type IL-21. The present invention also provides a fusion comprising the IL-21 mutant protein, a nucleic acid encoding the IL-21 mutant protein or the fusion, and a vector and a host cell comprising the nucleic acid. The present invention further provides a method for preparing the IL-21 mutant protein or the fusion, a pharmaceutical composition comprising the same, and therapeutic use.

Specifically, the present invention relates to the following embodiments:
1. An IL-21 mutant protein, wherein the mutant protein comprises one or more of the following mutations compared to wild-type IL-21 (preferably human IL-21, and more preferably IL-21 comprising a sequence of SEQ ID NO: 74):
   (i) replacement of amino acids at positions 1-15 of IL-21 with amino acids at positions 1-15 or amino acids comprising CS3 at positions 1-15 of IL-4 (preferably human IL-4); replacement of amino acids at positions 1-14 of IL-21 with amino acids at positions 1-14 or amino acids comprising CS3 at positions 1-14 of IL-4; replacement of amino acids at positions 1-13 of IL-21 with amino acids at positions 1-13 or amino acids comprising CS3 at positions 1-13 of IL-4; replacement of amino acids at positions 1-12 of IL-21 with amino acids at positions 1-12 or amino acids comprising CS3 at positions 1-12 of IL-4; replacement of amino acids at positions 1-11 of IL-21 with amino acids at positions 1-11 or amino acids comprising CS3 at positions 1-11 of IL-4; replacement of amino acids at positions 1-10 of IL-21 with amino acids at positions 1-10 or amino acids comprising CS3 at positions 1-10 of IL-4; replacement of amino acids at positions 1-9 of IL-21 with amino acids at positions 1-9 or amino acids comprising CS3 at positions 1-9 of IL-4; replacement of amino acids at positions 1-8 of IL-21 with amino acids at positions 1-8 or amino acids comprising CS3 at positions 1-8 of IL-4; replacement of amino acids at positions 1-7 of IL-21 with amino acids at positions 1-7 or amino acids comprising CS3 at positions 1-7 of IL-4; replacement of amino acids at positions 1-6 of IL-21 with amino acids at positions 1-6 or amino acids comprising CS3 at positions 1-6 of IL-4; replacement of amino acids at positions 1-5 of IL-21 with amino acids at positions 1-5 or amino acids comprising CS3 at positions 1-5 of IL-4; replacement of amino acids at positions 1-4 of IL-21 with amino acids at positions 1-4 or amino acids comprising CS3 at positions 1-4 of IL-4; or replacement of amino acids at positions 1-9 of IL-21 with amino acids at positions 1-11 or amino acids comprising CS3 at positions 1-11 of IL-4;
   (ii) mutations in at least 1, 2, 3, 4, or 5 positions selected from the following, resulting in glycosylation at the positions: D4, R5, H6, M7, D15, V17, Q19, K21, D37, E39, R76, K77, P78, P79, S80, N82, G84, H120, and/or H122;
   (iii) substitutions at 1-5 positions, e.g., 1 or 2 positions, selected from the following: 18, K72, K77, P78, and/or P79, wherein the K can be substituted with D or E, the P can be substituted with E or A, or the I can be substituted with Q; and
   (iv) a deletion of 1, 2, 3, 4, 5, 6, 7, 8, or 9 amino acids or a deletion of a segment comprising 1, 2, 3, 4, 5, 6, 7, 8, or 9 amino acids at the N-terminus of Helix A (e.g., positions 1-15), CD loop (e.g., positions 82-95 or 84-91), or C loop (e.g., positions 76-81);
   wherein the amino acid positions are amino acid positions numbered according to SEQ ID NO: 74.
2. The mutant protein according to embodiment 1, wherein the amino acids at positions 1-15 of the N-terminus of IL-4 used for substitution in the mutation (i) are HKSDITLQEIIKTLN or HKCDITLQEIIKTLN;
   more preferably, the mutation in the mutation (i) is selected from:
   replacement of 1-5 (QGQDR) of IL-21 with 1-5 & C3S (HKSDI) of IL-4;
   replacement of 1-9 (QGQDRHMIR) of IL-21 with 1-9 & C3S (HKSDITLQE) of IL-4;
   replacement of 1-15 (QGQDRHMIRMRQLID) of IL-21 with 1-15 & C3S (HKSDITLQEIIKTLN) of IL-4;
   replacement of 1-12 (QGQDRHMIRMRQ) of IL-21 with 1-12 & C3S (HKSDITLQEIIK) of IL-4;
   replacement of 1-11 (QGQDRHMIRMR) of IL-21 with 1-11 & C3S (HKSDITLQEII) of IL-4; and
   replacement of 1-9 (QGQDRHMIR) of IL-21 with 1-11 & C3S (HKSDITLQEII) of IL-4.
3. The IL-21 mutant protein according to embodiment 1, wherein the mutation in the mutation (ii) is substitutions of amino acids at the positions with N or T or S; preferably, the mutation (ii) comprises 1, 2, 3, 4, or 5 substitutions selected from the following:
   D4N, R5N, H6T, M7T, D15N, V17T, Q19N, K21T, D37N, E39T, R76N, K77N, P78T/S, P79T/N, S80N, N82T, G82T, G84T, H120N, and/or H122T;
   more preferably, the mutation (ii) is selected from a substitution or a combination of substitutions at the following position:
      D4 & H6;
      R5 & M7;
      D15 & V17;
      IQ19 & K21;
      R76 & P78;
      K77 & P78 & P79;
      P79;
      S80 & N82;
      G84;
      H120 & H122; or
      D37 & E39;
   and most preferably, the mutation (ii) is selected from the following substitutions or combinations of substitutions:
      D4N & H6T;
      R5N & M7T;
      D15N & V17T;
      IQ19N & K21T;
      R76N & P78T;
      K77N & P78S & P79T;
      P79N;
      S80N & N82T;
      G84T;
      H120N & H122T; and
      D37N & E39T.
4. The IL-21 mutant protein according to embodiment 1, wherein the mutation in the mutation (iii) comprises 1-5, e.g., 1-2, of substitutions selected from the following:
   I8Q, K72E, K77D, P78A, and/or P79E;
   preferably, the mutation in the mutation (iii) is a substitution at the following position or combination of positions:
      K72;
      18 & P79; or
      K77 & P78, wherein the K can be substituted with D or E, the P can be substituted with E or A, or the I can be substituted with Q;
   preferably, the mutation in the mutation (iii) is selected from
      K72E;
      I8Q & P79E; and
      K77D & P78A.
5. The IL-21 mutant protein according to embodiment 1, wherein the mutation in the mutation (iv) comprises a deletion of an amino acid segment at positions selected from the following: deletions at positions 1-9, positions 78-79, positions 85-87, and positions 84-91;
   preferably, the mutation in the mutation (iv) is a deletion of an amino acid segment at the following position or combination of positions:
      truncation 85-87;
      truncation 78-79;
      truncation 1-9;
      truncation 84-91; or
      truncation 1-9 & truncation 78-79;
   preferably, the mutation in the mutation (iv) is selected from the following deletion of an amino acid segment:
      truncation 85-87 (RRQ);
      truncation 78-79 (PP);
      truncation 1-9 (QGQDRHMIR);
      truncation 84-91 (GRRQKHRL); or
      truncation 1-9 (QGQDRHMIR) & truncation 78-79 (PP).
6. The IL-21 mutant protein according to embodiment 1, wherein the mutant protein comprises mutations selected from the following:
   (i) truncation 1-9 (QGQDRHMIR) & Q19N & K21T;
   (ii) truncation 84-91 (GRRQKHRL) & C42A & C93T & Q19N & K21T;
   (iii) replacement of 1-9 (QGQDRHMIR) of IL-21 with 1-9 & C3S (HKSDITLQE) of IL-4 & truncation 78-79 (PP);
   (iv) replacement of 1-9 (QGQDRHMIR) of IL-21 with 1-9 & C3S (HKSDITLQE) of IL-4 & K117N & I119T;
   (v) replacement of 1-9 (QGQDRHMIR) of IL-21 with 1-9 & C3S (HKSDITLQE) of IL-4 & D37N & E39T;
   (vi) replacement of 1-9 (QGQDRHMIR) of IL-21 with 1-9 & C3S (HKSDITLQE) of IL-4 & D15N & V17T; and
   (vii) replacement of 1-5 (QGQDR) of IL-21 with 1-5 (HKCDI) of IL-4 & L123C.
7. The IL-21 mutant protein according to any one of embodiments 1-6, wherein the wild-type IL-21 comprises an amino acid sequence set forth in SEQ ID NO: 74 or SEQ ID NOs: 106-108, or an amino acid sequence having at least 95%-99% or more identity to the amino acid sequence or having no more than 1-10 or 1-5 amino acid conservative substitutions.
8. The IL-21 mutant protein according to any one of embodiments 1-7, wherein the mutant protein has one or more of the following improved properties compared to the wild-type protein:
   (i) lower affinity for IL-21R;
   (ii) higher stability;
   (iii) improved druggability (e.g., longer half-life and/or improved purity, e.g., SEC purity); and/or
   (iv) upon formation of a fusion protein with an antibody or an antigen binding fragment thereof directed against an antigen, increased selective activation of cells positive for the antigen.
9. The IL-21 mutant protein according to any one of embodiments 1-7, wherein the mutant protein comprises or consists of an amino acid sequence selected from SEQ ID NOs: 75-105, or comprises an amino acid sequence having at least 85%, 86%, 87%, 88%, or 89% identity, preferably 90% or more identity, preferably 95% but no more than 97%, and more preferably no more than 96% identity to the amino acid sequence selected from SEQ ID NOs: 75-105.
10. An IL-21 mutant protein fusion protein, comprising the IL2 mutant protein according to any one of embodiments 1-8.
11. The IL-21 mutant protein fusion protein according to embodiment 10, comprising the IL-21 mutant protein according to any one of embodiments 1-9 linked to an Fc fragment, or comprising the IL-21 mutant protein according to any one of embodiments 1-9 linked to an antibody or an antigen binding fragment thereof, wherein the linkage is achieved with or without a linker.
12. The mutant protein fusion protein according to embodiment 11, wherein the Fc fragment is human IgG Fc, e.g., human IgG1 Fc, human IgG2 Fc, or human IgG4 Fc, preferably, the Fc fragment is human IgG1 Fc, e.g., human IgG1 Fc comprising an L234A/L235A mutation, and more preferably, the Fc fragment comprises or consists of an amino acid sequence of SEQ ID NO: 70 or an amino acid sequence having at least 90% identity, e.g., 95%, 96%, 97%, 99% or more identity thereto.
13. The IL-21 mutant protein fusion protein according to embodiment 11, wherein an antigen against which the antibody is directed is PD-1, PD-L1, or PD-L2.
14. The IL-21 mutant protein fusion protein according to embodiment 12, wherein an antigen against which the antibody is directed is PD-1, and the antibody or the antigen binding fragment thereof comprises:
   (1) three complementarity determining regions HCDR1, HCDR2, and HCDR3 comprised in a VH set forth in SEQ ID NO: 115, and three complementarity determining regions LCDR1, LCDR2, and LCDR3 comprised in a VL set forth in SEQ ID NO: 116; or
   (2) HCDR1, HCDR2, and HCDR3 set forth in amino acid sequences of SEQ ID NOs: 109, 110, and 111, respectively, and LCDR1, LCDR2, and LCDR3 set forth in amino acid sequences of SEQ ID NOs: 112, 113, and 114, respectively.
15. The IL-21 mutant protein fusion protein according to embodiment 14, wherein the antibody or the antigen binding fragment thereof comprises:
   a VH comprising or consisting of an amino acid sequence set forth in SEQ ID NO: 115 or an amino acid sequence having at least 90%, 91%, 92%, 93%, 94%, 95%, 96%, 97%, 98%, or 99% identity thereto, and a VL comprising or consisting of an amino acid sequence set forth in SEQ ID NO: 116 or an amino acid sequence having at least 90%, 91%, 92%, 93%, 94%, 95%, 96%, 97%, 98%, or 99% identity thereto.
16. The IL-21 mutant protein fusion protein according to any one of embodiments 10-12, comprising
   chain A: the IL-21 mutant protein linked to the N-terminus of the Fc fragment via a linker or directly;
   wherein preferably, the fusion protein comprises two identical chains A.
17. The mutant protein fusion protein according to embodiment 16, wherein the chain A comprises or consists of an amino acid sequence set forth in any one of SEQ ID NOs: 1-32.
18. The IL-21 mutant protein fusion protein according to any one of embodiments 10, 11, and 13-15, comprising the following structures:
   chain A: a light chain of the antibody; and
   chain B: the IL-21 mutant protein linked to the C-terminus of a heavy chain of the antibody;
   wherein preferably, the mutant protein fusion protein comprises two identical chains A and two identical chains B.
19. The IL-21 mutant protein fusion protein according to embodiment 15, wherein chain A comprises an amino acid sequence set forth in SEQ ID NO: 34, or an amino acid sequence having at least 85%, 90%, 91%, 92%, 93%, 94%, 95%, 96%, 97%, 98%, 99%, or 100% identity thereto, and/or chain B comprises an amino acid sequence set forth in any one of SEQ ID NOs: 35-54, or an amino acid sequence having at least 85%, 90%, 91%, 92%, 93%, 94%, 95%, 96%, 97%, 98%, 99%, or 100% identity thereto.
20. The IL-21 mutant protein fusion protein according to any one of embodiments 10, 11, and 13-15, comprising the following structures:
   chain A: a light chain of the antibody;
   chain B1: the IL-21 mutant protein linked to the C-terminus of one heavy chain of the antibody; and
   chain B2: a heavy chain of the antibody;
   wherein preferably, the fusion protein comprises two identical chains A, one chain B1, and one chain B2; and preferably, the chain B1 comprises a knob mutation, e.g., S354C & T366W, and the chain B2 comprises a hole mutation, e.g., Y349C & T366S & L368A & Y407V
21. The IL-21 mutant protein fusion protein according to embodiment 20, wherein the chain A comprises an amino acid sequence set forth in SEQ ID NO: 34, or an amino acid sequence having at least 85%, 90%, 91%, 92%, 93%, 94%, 95%, 96%, 97%, 98%, 99%, or 100% identity thereto; and/or the chain B1 comprises or consists of an amino acid sequence set forth in any one of SEQ ID NOs: 55-67, or an amino acid sequence having at least 85%, 90%, 91%, 92%, 93%, 94%, 95%, 96%, 97%, 98%, 99%, or 100% identity thereto; and/or the chain B2 comprises an amino acid sequence set forth in SEQ ID NO: 33, or an amino acid sequence having at least 85%, 90%, 91%, 92%, 93%, 94%, 95%, 96%, 97%, 98%, 99%, or 100% identity thereto.
22. The IL-21 mutant protein fusion protein according to any one of embodiments 10-21, wherein the linker comprises a linker sequence selected from the following: (GS)n, (GSGGS)n, (GGGGS)n, and (GGGS)n, wherein n is an integer of at least 1, preferably, the linker comprises (G4S)₂ or (G4S)₃.
23. An isolated polynucleotide, encoding a chain in the IL-21 mutant protein according to any one of embodiments 1-9 or the IL-21 mutant protein fusion protein according to any one of embodiments 10-22.
24. An expression vector, comprising the polynucleotide according to embodiment 23.
25. A host cell, comprising the polynucleotide according to embodiment 23 or the vector according to embodiment 24, wherein preferably, the host cell is a yeast cell or a mammalian cell, particularly an HEK293 cell or a CHO cell.
26. A method for producing the IL-21 mutant protein according to any one of embodiments 1-9 or the IL-21 mutant protein fusion protein according to any one of embodiments 10-22, comprising culturing the host cell according to embodiment 25 under conditions suitable for expression of the IL-21 mutant protein or the fusion protein.
27. A pharmaceutical composition, comprising the IL-21 mutant protein according to any one of embodiments 1-9 or the fusion protein according to any one of embodiments 10-12, and optionally a pharmaceutical auxiliary material.
28. Use of the IL-21 mutant protein according to any one of embodiments 1-9 or the fusion protein according to any one of embodiments 10-21 or the pharmaceutical composition according to embodiment 27 in preparing a medicament for the prevention and/or treatment of cancer, wherein preferably, the cancer is a solid tumor or a hematological tumor.
29. The use according to embodiment 28, wherein the pharmaceutical composition also comprises a second therapeutic agent.
30. A method for preventing and/or treating cancer in a subject, comprising administering to the subject the IL-21 mutant protein according to any one of embodiments 1-9 or the fusion protein according to any one of embodiments 10-21 or the pharmaceutical composition according to embodiment 27, wherein preferably, the cancer is a solid tumor or a hematological tumor.
31. The method according to embodiment 30, wherein the mutant protein, the fusion protein, or the pharmaceutical composition is administered in a combination therapy with a second therapeutic agent.

### BRIEF DESCRIPTION OF THE DRAWINGS

FIG. 1 shows a crystal structure diagram of IL-21 from PDB: 3TGX (FIG. 1A), the comparison of the structure of IL-21 with that of IL-4 (FIG. 1B), and a schematic crystal diagram of IL-21 binding to its receptor (FIG. 1C).
FIG. 2 shows three formats (Format 1, Format 2, and Format 3) of an IL-21 fusion protein.
FIG. 3 shows the stimulatory activity of rhIL-21 and various fusion protein molecules for the STAT signaling pathway in PD1-negative HuT78 cells and PD1-positive HuT78-GFP-PD1 cells.
FIG. 4 shows the stimulatory activity of rhIL-21, the fusion protein molecule 053 of the present invention, and a control molecule 106 for the STAT signaling pathway in PD1-negative HuT78 cells and PD1-positive HuT78-GFP-PD1 cells.

### DETAILED DESCRIPTION

### I. Definitions

Before the present invention is described in detail below, it should be understood that the present invention is not limited to the particular methodology, protocols, and reagents described herein, as these may vary. It should also be understood that the terms used herein are only intended to describe specific embodiments rather than limit the scope of the present invention, which will be limited only by the appended claims. Unless otherwise defined, any technical and scientific term used herein has the same meaning as commonly understood by those of ordinary skill in the art to which the present invention belongs.

For the purpose of explaining this description, the following definitions will be used, and wherever appropriate, terms used in the singular form may also include the plural form, and vice versa. It should be understood that the terms used herein are for the purpose of describing specific embodiments only, and are not intended to be limiting. The term "about" used in combination with a numerical value is intended to encompass the numerical values in a range from a lower limit 5% less than the specified numerical value to an upper limit 5% greater than the specified numerical value.

As used herein, the term "and/or" refers to any one of the options or any two or more of the options.

As used herein, the term "comprise" or "include" is intended to mean that the elements, integers or steps are included, but not to the exclusion of any other elements, integers or steps. The term "comprise" or "include" used herein, unless otherwise indicated, also encompasses the situation where the entirety consists of the described elements, integers or steps. For example, when an IL-21 mutant protein "comprising" or "including" a certain mutation or combination of mutations is mentioned, it is also intended to encompass IL-21 mutant proteins having the mutation or combination of mutations only.

Herein, wild-type "interleukin-21" or "IL-21" refers to a parent IL-21 protein, preferably a naturally occurring IL-21 protein, e.g., a native IL-21 protein derived from a human, mouse, rat, or non-human primate, serving as a template to which the mutation or the combination of mutations of the present invention is introduced, including both unprocessed (e.g., without the removal of the signal peptide) and processed (e.g., with the removal of the signal peptide) forms. A full-length native human IL-21 sequence (Q9HBE4) comprising a signal peptide is set forth in SEQ ID NO: 106, and the sequence of its mature protein is set forth in SEQ ID NO: 74. In addition, this expression also includes naturally occurring allelic and splice variants, isotypes, homologs, and species homologs of IL-21. For example, the IL-21 isotype (Q9HBE4-2) that results from alternative splicing is also encompassed within this expression, wherein the full-length native human IL-21 sequence comprising the signal peptide is set forth in SEQ ID NO: 108, and the sequence of its mature protein is set forth in SEQ ID NO: 107. This expression also includes variants of native IL-21. For example, the variants may have at least 95%-99% or more identity to native IL-21 or have no more than 1-10 or 1-5 amino acid mutations (e.g., conservative substitutions), and preferably have substantially the same binding affinity for IL-21R as the native IL-21 protein. In some embodiments, the wild-type IL-21 sequence may have at least 85% or 95%, or even at least 96%, 97%, 98%, or 99% or more amino acid sequence identity to an amino acid sequence set forth in SEQ ID NO: 74, 106, 107, or 108.

Herein, the amino acid mutation may be a substitution, deletion/truncation, insertion, and addition of an amino acid or an amino acid sequence segment. Any combination of substitution, deletion/truncation, insertion, and addition may be made to obtain a final mutant protein construct with the desired properties, such as reduced binding affinity for IL-21R and/or improved druggability. Amino acid deletions and insertions include deletions/truncations and insertions at the amino terminus and/or carboxyl terminus of a polypeptide sequence, as well as deletions/truncations and insertions within the polypeptide sequence. In some embodiments, the preferred amino acid mutation is an amino acid substitution (e.g., a single amino acid substitution or a combination of several amino acid substitutions) or the replacement of amino acid sequence segments. For example, it is possible to introduce one or several glycosylation sites by substitutions to promote the glycosylation of the IL-21 protein. It is also possible to obtain mutant proteins with reduced binding affinity for IL-21R by substitution of amino acids at a binding interface with IL-21R. It is also possible to replace 1 or several amino acids or amino acid sequence segments of the N-terminus of wild-type IL-21 with the N-terminal amino acids of different other cytokines (e.g., IL-4) to form chimeras with other cytokines. In some embodiments, the preferred amino acid mutation also includes a deletion mutation or a truncation mutation. The deletion/truncation mutation encompasses the deletion of one or several amino acids or amino acid sequence segments in a certain domain (e.g. the CD loop region or the N-terminus) of the wild-type IL-21 to obtain a truncated variant of IL-21.

In the present invention, when an amino acid position in the IL-21 protein or IL-21 sequence segment is mentioned, it is determined by reference to an amino acid sequence set forth in SEQ ID NO: 74 of the wild-type human IL-21 protein (also referred to as IL-21^{WT}). The corresponding amino acid positions on other IL-21 proteins or polypeptides (including splice variants) may be identified by amino acid sequence alignment with SEQ ID NO: 74. Thus, in the present invention, unless otherwise stated, an amino acid position in an IL-21 protein or polypeptide is an amino acid position numbered according to SEQ ID NO: 74. For example, when "D4N" is mentioned, it refers to a substitution of an aspartic acid residue D at position 4 of SEQ ID NO: 74 with N, or a substitution of an amino acid residue at a corresponding position (which may or may not be D at that position) in another IL-21 polypeptide sequence with N by alignment. To perform a sequence alignment for determining an amino acid position, Basic Local Alignment Search Tool available at https://blast.ncbi.nlm.nih.gov/Blast.cgi can be used with default parameters.

Herein, when an IL-21 mutant protein is mentioned, a single amino acid substitution is described as [original amino acid residue/position/amino acid residue for substitution]. For example, a substitution of aspartic acid at position 4 with asparagine can be denoted as D4N. When there are multiple optional amino acid substitutions (e.g., N and T) at a given position (e.g., D37), the amino acid substitutions can be denoted as D37N/T.

Herein, when an IL-21 mutant protein is mentioned, an amino acid sequence segment substitution/replacement is described as [(original amino acid sequence segment)/(position)/(amino acid sequence segment for substitution)]. For example, a substitution of a sequence segment at positions 1-9 "(QGQDR)" with "(HKSDI)" can be denoted as (QGQDR)/(1-5)/(HKCDI). Alternatively, the substitution or replacement is described as "replacement of 1-5 (QGQDR) of IL-21 with 1-5 (HKCDI) of IL-4". If the amino acid sequence segment of IL-4 contains a substitution, for example, a C3S substitution, it may be described as "replacement of 1-5 (QGQDR) of IL-21 with 1-5 & C3S (HKSDI) of IL-4".

Herein, when an IL-21 mutant protein is mentioned, a deletion/truncation of amino acid sequence segments is described as [truncation position (amino acid sequence segment for truncation)]. For example, a deletion/truncation of a sequence segment at positions 85-87 "(RRQ)" is denoted as "truncation 85-87 (RRQ)".

The mutations may be linked by a plus sign "&" or "-" to denote a combinatorial mutation at a plurality of given positions. For example, the combinatorial mutation at positions D37N and E39T can be denoted as: D37N & E39T or D37N-E39T.

It should be noted that when applying the above description, the amino acid at the corresponding position in another parent IL-21 polypeptide sequence may differ from the amino acid at the corresponding position of SEQ ID NO: 74 by alignment, but the denoted mutation can still be performed.

Herein, the "percent sequence identity" can be determined by comparing two optimally aligned sequences over a comparison window. Preferably, sequence identity is determined over the full length of a reference sequence (e.g., SEQ ID NO: 74). Methods of sequence alignment for comparison are well known in the art. Algorithms suitable for determining the percent sequence identity include, for example, BLAST and BLAST 2.0 algorithms (see Altschul et al., Nuc. Acids Res. 25: 3389-402, 1977 and Altschul et al., J. Mol. Biol. 215: 403-10, 1990). Software for performing BLAST analysis is publicly available from the National Center for Biotechnology Information. For the purpose of the present application, the percent identity can be determined by using Basic Local Alignment Search Tool available at https://blast.ncbi.nlm.nih.gov/Blast.cgi with default parameters.

As used herein, the term "conservative substitution" refers to an amino acid substitution that does not adversely affect or change the biological function of a protein/polypeptide containing an amino acid sequence. For example, a conservative substitution may be introduced by standard techniques known in the art, such as site-directed mutagenesis and PCR-mediated mutagenesis. A typical conservative amino acid substitution refers to a substitution of an amino acid with another amino acid having similar chemical properties (*e.g.,* charge or hydrophobicity). Conservative replacement tables of functionally similar amino acids are well known in the art. In the present invention, residues for conservative substitutions are from the conservative substitution table X below, particularly from the preferred residues for conservative amino acid substitutions in Table X.

**Table X**

| Original residues | Exemplary substitution | Preferred conservative amino acid substitution |
|---|---|---|
| Ala (A) | Val; Leu; Ile | Val |
| Arg (R) | Lys; Gln; Asn | Lys |
| Asn (N) | Gln; His; Asp; Lys; Arg | Gin |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gin; Lys; Arg | Arg |
| Ile (I) | Leu; Val; Met; Ala; Phe; Nle | Leu |
| Leu (L) | Nle; Ile; Ual; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Nle | Leu |

For example, relative to one of SEQ ID NOs: 74 and 106-108, the wild-type IL-21 protein may have conservative amino acid substitutions, or only have conservative amino acid substitutions; and in one preferred embodiment, the conservative substitutions involve no more than 10 amino acid residues, e.g., 1, 2, 3, 4, 5, 6, 7, 8, 9, or 10 residues. For another example, relative to the IL-21 mutant protein sequences specifically given herein (e.g., any one of SEQ ID NOs: 75-105), the IL-21 mutant protein of the present invention may have conservative amino acid substitutions, or only have conservative amino acid substitutions; and in one preferred embodiment, the conservative substitutions involve no more than 10 amino acid residues, e.g., 1, 2, 3, 4, 5, 6, 7, 8, 9, or 10 residues.

"Affinity" or "binding affinity" refers to the inherent binding ability that reflects the interaction between members of a binding pair. The affinity of molecule X for its binding partner Y can be represented by an equilibrium dissociation constant (K_{D}), which is the ratio of a dissociation rate constant (k_{dis}) to an association rate constant (kₒₙ). The binding affinity can be measured by common methods known in the art. One specific method for measuring the affinity is the ForteBio affinity assay technique or BLI assay technique described herein.

Herein, an antigen binding molecule is a polypeptide molecule that can specifically bind to an antigen, e.g., an immunoglobulin molecule, an antibody, or an antibody fragment (e.g., a Fab fragment and an scFv fragment). In one embodiment, the antigen binding molecule of the present invention is a binding molecule, such as an antibody, e.g., a monoclonal antibody, directed against an immune checkpoint molecule as an antigen. In one embodiment, the immune checkpoint molecule is PD-1, PD-L1, or PD-L2.

Herein, an antibody Fc fragment refers to a C-terminus region of an immunoglobulin heavy chain that contains at least a portion of the constant region, and may include Fc fragments of native sequences and variant Fc fragments. Fc fragments of native sequences encompass various naturally occurring Fc sequences of immunoglobulins, such as the Fc regions of various Ig subclasses or allotypes thereof (Gestur Vidarsson et al., IgG subclasses and allotypes: from structure to effector functions, 20 October 2014, doi: 10.3389/fimmu.2014.00520.). In one embodiment, the heavy chain Fc fragment of human IgG extends from Cys226 or Pro230 of the heavy chain to the carboxyl terminus. In another embodiment, the C-terminus lysine (Lys447) of the Fc fragment may or may not be present. In some other embodiments, the Fc fragment is a variant Fc fragment comprising a mutation, for example, an L234A-L235A mutation (LALA mutation). In one embodiment, the Fc fragment is from IgG1. In one embodiment, the Fc used in the present invention is from IgG1 and has an LALA mutation. In one embodiment, the Fc used in the present invention contains a sequence set forth in SEQ ID NO: 70 (from IgG1 with an LALA mutation) or a sequence having at least 85%, 90%, 95%, 96%, 97%, 98%, or 99% identity to the sequence set forth in SEQ ID NO: 70. Unless otherwise indicated herein, amino acid residues in the Fc fragment are numbered according to the EU numbering system, also referred to as the EU index, as described in Kabat, E.A. et al., Sequences of Proteins of Immunological Interest, 5th Ed., Public Health Service, National Institutes of Health, Bethesda, MD (1991), NIH Publication 91-3242. In some embodiments, the antibody Fc fragment may carry an IgG1 hinge sequence or a portion of the IgG1 hinge sequence at the N-terminus, e.g., the sequence of E216 to T225 or the sequence of D221 to T225 according to the EU numbering. Mutations may be contained in the hinge sequence.

"Antigen binding fragment" refers to a molecule different from an intact antibody, which contains a portion of the intact antibody and binds to an antigen to which the intact antibody binds. Examples of the antibody fragments include, but are not limited to, Fv, Fab, Fab', Fab'-SH, F(ab')₂, a domain antibody (dAb), a linear antibody, a single-chain antibody (e.g., scFv), a single-domain antibody (e.g., VHH), a bi-valent antibody or a fragment thereof, or a camelid antibody.

The term "antigen" refers to a molecule that induces an immune response. Such an immune response may involve antibody production or activation of specific immune cells, or both. Those skilled will understand that any macromolecules, including substantially all proteins or peptides, may be used as antigens. In addition, an antigen may be derived from recombinant or genomic DNA. In some embodiments, the antigen as described herein is a tumor-associated antigen, i.e., an antigen associated with the occurrence, development, or progression of a tumor, e.g., PD-1, PD-L1, or PD-L2.

"Complementarity determining region" or "CDR region" or "CDR" is a region in an antibody variable domain that is highly variable in sequence and forms a structurally defined loop ("hypervariable loop") and/or contains antigen-contacting residues ("antigen-contacting sites"). CDRs are primarily responsible for binding to antigen epitopes. The CDRs of the heavy and light chains are generally referred to as CDR1, CDR2, and CDR3, and are numbered sequentially from the N-terminus. The CDRs located in the heavy chain variable domain of the antibody are referred to as HCDR1, HCDR2, and HCDR3, while the CDRs located in the light chain variable domain of the antibody are referred to as LCDR1, LCDR2, and LCDR3. In a given amino acid sequence of a light chain variable region or a heavy chain variable region, the exact amino acid sequence boundary of each CDR can be determined using any one or a combination of many well-known antibody CDR assignment systems including, e.g., Chothia based on the three-dimensional structure of antibodies and the topology of the CDR loops (Chothia et al., (1989) Nature, 342: 877-883; Al-Lazikani et al., Standard conformations for the canonical structures of immunoglobulins, Journal of Molecular Biology, 273: 927-948 (1997)), Kabat based on antibody sequence variability (Kabat et al., Sequences of Proteins of Immunological Interest, 4th Ed., U.S. Department of Health and Human Services, National Institutes of Health (1987)), AbM (University of Bath), Contact (University College London), International ImMunoGeneTics database (IMGT) (imgt.cines.fr/ on the World Wide Web), and North CDR definition based on the affinity propagation clustering using a large number of crystal structures. For example, according to different CDR determination schemes, the residues of each CDR are as follows.

| CDR | Kabat scheme | AbM scheme | Chothia scheme | Contact scheme |
|---|---|---|---|---|
| LCDR1 | L24-L34 | L24-L34 | L26-L32 | L30-L36 |
| LCDR2 | L50-L56 | L50-L56 | L50-L52 | L46-L55 |
| LCDR3 | L89-L97 | L89-L97 | L91-L96 | L89-L96 |
| HCDR1 | H31-H35B | H26-H35B | H26-H32 | H30-H35B |
| (Kabat numbering system) | | | | |
| HCDR1 | H31-H35 | H26-H35 | H26-H32 | H30-H35 |
| (Chothia numbering system) | | | | |
| HCDR2 | H50-H65 | H50-H58 | H53-H55 | H47-H58 |
| HCDR3 | H95-H102 | H95-H102 | H96-H101 | H93-H101 |
| (Kabat numbering system) | | | | |

CDRs can also be determined based on having identical Kabat numbering positions as a reference CDR sequence (e.g., any one of the exemplary CDRs of the present invention).

Unless otherwise stated, in the present invention, the term "CDR" or "CDR sequence" encompasses CDR sequences determined by any one of the schemes above.

Unless otherwise stated, in the present invention, residue positions of an antibody variable region (including heavy chain variable region residues and light chain variable region residues) are numbered according to the Kabat numbering system (Kabat et al., Sequences of Proteins of Immunological Interest, 5th Ed., Public Health Service, National Institutes of Health, Bethesda, Md. (1991)).

In one embodiment, the CDRs in the heavy chain variable region and the light chain variable region of the antibody of the present invention are CDR sequences defined according to the North numbering scheme.

Herein, the term "IL-21R" refers to a receptor that binds to IL-21. The receptor is a type I cytokine receptor and has been shown to form a heterodimeric receptor complex with a common γ chain, which is a receptor subunit that is also shared by IL-2, IL-7, and IL-15. The receptor transduces the growth promoting signal of IL-21 and plays an important role in the proliferation and differentiation of T cells, B cells, and natural killer NK cells. Binding of this receptor to a ligand results in activation of a number of downstream signaling molecules, such as activation of JAK1, JAK3, STAT1, and STAT3. Herein, the term "IL-21R binding interface mutation" refers to a mutation occurred at amino acid sites where IL-21 interacts with IL-21R. These interaction sites can be determined by analyzing the crystal structure of the complex of IL-21 and its receptor (e.g., PDB:3TGX). In some embodiments, the mutation especially refers to mutations on helix A, helix C and a portion of the CD loop of IL-21, e.g., one or more of amino acid residues 8 or 72 or 77-79. Preferably, an IL-21 protein containing the mutation has reduced or eliminated binding to IL-21R compared to the corresponding protein before introduction of the mutation.

Herein, "IL-21" glycosylation refers to increasing the glycosylation level of the IL-21 mutant protein by introducing N-glycosylation sites into IL-21, thereby improving the druggability of the IL-21 mutant protein and/or providing it with reduced binding affinity for IL-21R. In some embodiments, the glycosylation site is selected from one or more of the following positions: 4-7, 15, 17, 19, 21, 37, 39, 76-80, 82, 84, 120, and 122.

The term "linker" as used herein refers to any molecule that enables a direct linkage of different portions of a fusion protein. Examples of linkers to establish covalent linkages between different portions of a fusion protein include peptide linkers and non-proteinaceous polymers including, but not limited to, polyethylene glycol (PEG), polypropylene glycol, polyalkylene oxide and copolymers of polyethylene glycol and polypropylene glycol. The term "peptide linker" according to the present invention refers to an amino acid sequence that links the amino acid sequence of a first moiety of a fusion protein to a second moiety of the fusion protein. For example, a peptide linker may link an IL-21 moiety of a fusion protein to an Fc domain or a fragment thereof. For example, a peptide linker may also link an antibody to IL-21, such as linking the C-terminus of an antibody heavy chain to IL-21. Preferably, the peptide linker has a length sufficient to link two entities in a manner that maintains their conformation relative to each other without interference with the desired activities. The peptide linker may or may not primarily include the following amino acid residues: Gly, Ser, Ala, or Thr. Useful linkers include glycine-serine polymers including, for example, (GS)n, (GSGGS)n, (GGGGS)n, (GGGS)n, and (GGGGS)nG, wherein n is an integer of at least 1 (and preferably 2, 3, 4, 5, 6, 7, 8, 9, or 10). Useful linkers also include glycine-alanine polymers, alanine-serine polymers, and other flexible linkers. In some embodiments, the linker is (GGGGS)₂ (SEQ ID NO: 69) or (GGGGS)₃ (SEQ ID NO: 72).

"Antibody in the form of IgG" refers to a heavy chain constant region of an antibody belonging to the IgG form. Heavy chain constant regions of all antibodies of the same type are identical, and heavy chain constant regions of antibodies of different types are different. For example, an antibody in the form of IgG4 means that the heavy chain constant region of the antibody is from IgG4, or an antibody in the form of IgG1 means that the heavy chain constant region of the antibody is from IgG1.

"Humanized" antibody refers to an antibody containing amino acid residues from non-human CDRs and amino acid residues from human FRs. In some embodiments, a humanized antibody will contain at least one, or generally two of substantially all variable domains in which all or substantially all CDRs (e.g., CDRs) correspond to those of a non-human antibody, and all or substantially all FRs correspond to those of a human antibody. A humanized antibody may optionally contain at least a portion of an antibody constant region derived from a human antibody. The "humanized form" of an antibody (such as a non-human antibody) refers to an antibody that has been humanized.

"Human antibody", "fully human antibody", and "fully humanized antibody" are used interchangeably, and refer to an antibody having an amino acid sequence which corresponds to the amino acid sequence of an antibody generated by a human or human cell or derived from a non-human source that utilizes human antibody libraries or other human antibody encoding sequences. This definition of a human antibody explicitly excludes humanized antibodies comprising non-human antigen binding residues.

The term "fusion" as used herein refers to a fusion formed by linking two or more initially separate proteins/genes/compounds. If the entity constituting the fusion is a protein, it is referred to as a fusion protein. The fusion protein is encompassed within the scope of the fusion of the present application. For example, IL-21 linked to an Fc dimer may constitute an IL-21 fusion protein, or IL-21 linked to an intact antibody or antibody fragment may also constitute an IL-21 fusion protein. The linkage between the two entity molecules constituting the fusion may be achieved with or without a linker.

The term "therapeutic agent" as described herein encompasses any substance that is effective in preventing or treating a tumor, e.g., cancer, including a chemotherapeutic agent, a cytokine, an angiogenesis inhibitor, a cytotoxic agent, other antibodies, a small molecule drug, or an immunomodulatory agent (e.g., an immunosuppressant). The term "effective amount" refers to an amount or dosage of the antibody, fragment, composition, or combination of the present invention which generates expected effects in a patient in need of treatment or prevention after administered to the patient in a single or multiple doses. An "effective amount" can encompass a "therapeutically effective amount" or a "prophylactically effective amount".

"Therapeutically effective amount" refers to an amount effective to achieve a desired therapeutic result at a necessary dose for a necessary period of time. The therapeutically effective amount is also such an amount that any toxic or undesired effect of the antibody, fragment thereof, composition, or combination is inferior to the therapeutically beneficial effect. The "therapeutically effective amount" preferably inhibits a measurable parameter (e.g., tumor volume) by at least about 40%, and even more preferably by at least about 50%, 55%, 60%, 65%, 70%, 75%, 80%, 85%, 90%, or even 100%, relative to untreated subjects. "Prophylactically effective amount" refers to an amount effective to achieve a desired prophylactic result at a necessary dose for a necessary period of time. Generally, since a prophylactic dose is administered in a subject before or at an earlier stage of a disease, a prophylactically effective amount will be less than a therapeutically effective amount.

The terms "host cell", "host cell line", and "host cell culture" are used interchangeably, and refer to cells into which exogenous nucleic acids are introduced, including progenies of such cells. Host cells include "transformants" and "transformed cells", which include primary transformed cells and progenies derived therefrom, regardless of the number of passages. Progenies may not be exactly the same as parent cells in terms of nucleic acid content, and may contain mutations. Mutant progenies having the same function or biological activity that are screened or selected from the initially transformed cells are included herein.

The term "label" used herein refers to a compound or composition which is directly or indirectly conjugated or fused to an agent, such as a polynucleotide probe or an antibody, and facilitates the detection of the agent to which it is conjugated or fused. The label itself can be detectable (e.g., a radioisotope label or a fluorescent label) or can catalyze a chemical change to a detectable substrate compound or composition in the case of enzymatic labeling. The term is intended to encompass direct labeling of a probe or an antibody by coupling (i.e., physical linking) a detectable substance to the probe or antibody and indirect labeling of a probe or an antibody by reacting with another reagent which is directly labeled.

"Individual" or "subject" includes mammals. The mammals include, but are not limited to, domestic animals (e.g., cattle, goats, cats, dogs, and horses), primates (e.g., human and non-human primates such as monkeys), rabbits, and rodents (e.g., mice and rats). In some embodiments, the individual or subject is a human.

The term "anti-tumor effect" refers to a biological effect that can be demonstrated by a variety of means, including but not limited to, for example, a decrease in tumor volume, a decrease in number of tumor cells, a decrease in tumor cell proliferation, or a decrease in tumor cell viability.

The terms "tumor" and "cancer" are used interchangeably herein and encompass solid and hematological tumors.

The term "cancer" refers to or describes a physiological condition in mammals characterized generally by unregulated cell growth. In certain embodiments, cancers suitable for treatment with the antibody of the present invention include solid tumors or hematological tumors, and the like, including metastatic forms of the cancers.

The term "tumor" refers to all neoplastic cell growth and proliferation, whether being malignant or benign, and all pre-cancerous and cancerous cells and tissues. The terms "cancer", "cancerous" and "tumor" are not mutually exclusive when referred to herein.

The term "pharmaceutical auxiliary material" refers to diluents, adjuvants (e.g., Freund's adjuvants (complete and incomplete)), excipients, carriers, stabilizers, or the like, which are administered with the active substance.

The term "pharmaceutical composition" refers to such a composition that exists in a form allowing effective biological activity of the active ingredient contained therein, and does not contain additional ingredients having unacceptable toxicity to a subject to which the composition is administered.

The term "pharmaceutical combination" refers to a non-fixed combination product or a fixed combination product, including but not limited to, a kit and a pharmaceutical composition. The term "non-fixed combination" means that the active ingredients (e.g., (i) the mutant protein or fusion of the present invention, and (ii) an additional therapeutic agent) are administered, either simultaneously or sequentially (without particular time limitation or at identical or different time intervals), to a patient as separate entities, wherein such administration provides two or more prophylactically or therapeutically effective active agents in the patient. The term "fixed combination" means that two or more active agents are administered to a patient simultaneously as a single entity. The doses and/or time intervals of two or more active agents are preferably selected such that the combined use of the components can result in a therapeutic effect on the disease or disorder which is greater than that achieved by the use of either component alone. The ingredients may each take a separate formulation form and such separate formulation forms may be identical or different.

The term "combination therapy" refers to the administration of two or more therapeutic agents or modalities (e.g., radiotherapy or surgery) to treat the diseases as described herein. Such administration includes co-administration of these therapeutic agents in a substantially simultaneous manner, for example, in a single capsule with a fixed proportion of active ingredients. Alternatively, such administration includes co-administration of the active ingredients in a variety of or separate containers (such as tablets, capsules, powder and liquid). The powder and/or liquid can be reconstituted or diluted to a desired dose before administration. In addition, such administration also includes using each type of the therapeutic agents at approximately the same time or in a sequential manner at different times. In any case, the therapeutic regimen will provide the beneficial effect of the pharmaceutical combination in the treatment of disorders or symptoms described herein.

As used herein, "treatment" (or "treat" or "treating") refers to slowing, interrupting, arresting, alleviating, stopping, lowering, or reversing the progression or severity of an existing symptom, disorder, condition, or disease.

As used herein, "prevention" (or "prevent" or "preventing") includes the inhibition of the development or progression of symptoms of a disease or disorder, or a particular disease or disorder. In some embodiments, subjects with family history of cancer are candidates for preventive regimens. Generally, in the context of cancer, the term "prevention" refers to the administration of a drug before the onset of signs or symptoms of cancer, particularly in subjects at risk of cancer.

The term "vector" as used herein refers to a nucleic acid molecule capable of proliferating another nucleic acid to which it is linked. The term includes vectors that serve as self-replicating nucleic acid structures as well as vectors incorporated in the genome of a host cell into which they have been introduced. Some vectors are capable of directing the expression of a nucleic acid to which they are operably linked. Such vectors are referred to as "expression vectors" herein.

"Subject/patient/individual sample" refers to a collection of cells or fluids obtained from a patient or a subject. The source of tissue or cell samples can be solid tissues, e.g., from fresh, frozen and/or preserved organ or tissue samples or biopsy samples or puncture samples; blood or any blood component; body fluids such as cerebrospinal fluids, amniotic fluids, peritoneal fluids, or interstitial fluids; and cells from a subject at any time during pregnancy or development. Tissue samples may comprise compounds which are naturally not mixed with tissues, such as preservatives, anticoagulants, buffers, fixatives, nutrients and antibiotics.

### II. IL-21 Mutant Protein and Fusion Thereof

### Advantageous biological properties of the IL-21 mutant protein of the present invention

The IL-21 protein forms a four-helix bundle (A, B, C, and D) arranged by an up-up-down-down topology, which is shared by all members of the short chain cytokine family. A ring structure connects the four helix bundles. Specifically, the domain of human IL-21 (e.g., set forth in SEQ ID NO: 74) is as follows, in which Helix is known as the N-terminus of IL-21 and is a region that binds to IL-21R, which also includes Helix C, C loop, and/or CD loop:

| Domain | Sequence* |
|---|---|
| Helix A | Q1 to D26 |
| AB loop | Q27 to E39 |
| Helix B | T40 to A53 |
| BC loop | Q54 to G61 |
| Helix C | N62 to K75 |
| C loop | R76 to T81 |
| CD loop | N82 to P104 |
| Helix D | K105 to S124 |
| C-terminus | S125 to S133 |

| | |
|---|---|
| *The position is determined with reference to the position of an amino acid sequence set forth in SEQ ID NO: 74. | |

Through long-term research, the inventors have found that the following molecular mutations and engineering can be combined and implemented to improve the stability and the druggability of the IL-21 and realize a good production performance simultaneously, and to enhance its selective activation of particular T cells:
1. IL-21/IL-4 chimera mutation: the N-terminal amino acid of IL-21 is substituted with the N-terminal amino acid of IL-4 to obtain a more stable N-terminal conformation of IL-21, thereby improving the stability of IL-21 or a fusion thereof and further improving the druggability;
2. IL-21 glycosylation mutation: the glycosylation pattern of IL-21 is changed by introducing N-glycosylation sites at a binding interface with IL-21R to reduce the binding affinity of IL-21 for its receptor IL-21R, thereby improving the stability of IL-21 or a fusion thereof and further improving the druggability;
3. IL-21 interface amino acid mutation: a substitution performed by introducing amino acids with similar charge or hydrophilicity/hydrophobicity is introduced at an interface where IL-21 binds to IL-21R to change the binding property of the IL-21 mutant protein to IL-21R and reduce the binding affinity of IL-21 for its receptor IL-21R, thereby improving the druggability of IL-21 or a fusion thereof;
4. IL-21 truncation mutation: performing truncations/deletions on the Helix A, CD loop, or C loop region of IL-21 by one or more amino acids or amino acid segments to change the local structure of IL-21 to enable a more stable local structure, thereby improving the stability of IL-21 and further improving the druggability of IL-21 or a fusion thereof.

In some embodiments, the inventors have found that one or more (e.g., 2, 3, or all 4) of the above mutations can be further combined to change the binding property of the IL-21 mutant protein to IL-21R or to change the stability of the IL-21 mutant protein or a fusion thereof, thereby conferring good druggability to the mutant protein or the fusion thereof of the present invention.

In some embodiments, the improved druggability of the mutant protein of the present invention includes an increased half-life of the mutant protein or the fusion thereof and/or an increased purity of the produced protein.

### Improved druggability

In some embodiments, the IL-21 mutant protein or the fusion thereof of the present invention has improved druggability. e.g., ease of purification to higher protein purity, when expressed in mammalian cells such as HEK293 or CHO cells, particularly when expressed as a mutant protein fusion (e.g., an Fc fusion protein or a fusion protein formed with an antibody).

In some embodiments, the IL-21 mutant protein fusion or fusion protein of the present invention exhibits greater purity relative to the fusion or fusion protein of the wild-type IL-21 protein, as shown by determining the purity of the purified protein after protein A affinity chromatography. In some embodiments, the protein purity is determined by the SEC-HPLC technique. In some preferred embodiments, after purification, the IL-21 mutant protein or the fusion thereof of the present invention can reach a purity of more than 65%, 70%, 75%, 80%, or 85%, preferably more than 85%, 86%, 87%, 88%, 89%, 90%, 91%, 92%, 93%, 94%, 95%, 98%, or 99%.

In some other embodiments, the improved druggability as described herein refers to an improved half-life of the IL-21 mutant protein or the mutant protein fusion (e.g., an Fc fusion protein or a fusion protein formed with an antibody) of the present invention. In some embodiments, the IL-21 mutant protein or the fusion thereof of the present invention may have a half-life longer than that of a known IL-21 wild-type protein or a fusion thereof, or that of a known IL-21 mutant protein or a fusion thereof.

### Reduced binding affinity for IL-21 receptor

IL-21 has a short half-life *in vivo.* One of the main reasons is that IL-21 has a high binding affinity for a cell surface receptor, which makes it susceptible to endocytosis and degradation. Thus, the IL-21 mutant protein or the fusion thereof of the present invention has introduced mutations relative to the wild-type IL-21 or the fusion thereof, which results in the IL-21 mutant protein or the fusion thereof having reduced or eliminated (undetectable) IL-21R receptor binding.

In some embodiments, the binding affinity of the IL-21 mutant protein or the fusion thereof of the present invention for the IL-21R receptor is reduced by at least 5 times, at least 10 times, or at least 25 times, particularly at least 30 times, 50 times, or 100 times or more, relative to the wild-type IL-21 (e.g., IL-21 set forth in SEQ ID NO: 74) or the fusion thereof. In a preferred embodiment, the mutant protein or the fusion thereof of the present invention binds weakly or with undetectable affinity to the IL-21 receptor. The binding affinity can be determined by measuring the equilibrium dissociation constant (K_{D}) for the binding of the IL-21 mutant protein or the fusion thereof of the present invention to the receptor IL-21R by the BLI affinity assay technique.

### Increased selective activation of antigen positive cells

The mutant protein of the present invention can be fused with an antibody, e.g., a monoclonal antibody (e.g., directed against a tumor-associated antigen, e.g., PD-1, PD-L1, or PD-L2), to produce an IL-21 mutant protein-antibody fusion that has increased selective activation ability for antigen-positive cells compared to the wild-type IL-21 mutant protein-antibody fusion.

In some embodiments, the antibody is an antibody directed against PD-1, e.g., a monoclonal antibody directed against PD-1. The fusion protein of the IL-21 mutant protein and the PD-1 antibody has weak or undetectable activity in PD-1-negative cells, while has high or very high activity in PD-1-positive cells, relative to the wild-type IL-21.

Thus, the IL-21 mutant protein or the IL-21 mutant protein fusion of the present invention has a larger therapeutic window relative to the wild-type IL-21 protein or fusion, and can selectively activate cells positive for an antigen (e.g., a tumor-associated antigen), e.g., cells expressing the tumor-associated antigen, e.g., T cells expressing the tumor-associated antigen.

In one embodiment, in a STAT3 phosphorylation assay, the ability of the IL-21 mutant protein or the antibody fusion protein thereof to activate T cells (e.g., the JAK-STAT signaling pathway) is identified by detecting the activation of STAT3 phosphorylation signals by the IL-21 mutant protein or the antibody fusion protein thereof in T cells. For example, as described in the examples of the present application, STAT3 phosphorylation in cells can be analyzed by flow cytometry to determine the half maximum effective concentration (EC₅₀), so that the activation activity of the IL-21 mutant protein or the antibody fusion protein thereof of the present invention is determined.

### The mutant protein of the present invention

### IL-21/IL-4 chimeric mutation

In one aspect, the present invention provides an IL-21 mutant protein, which comprises a substitution of the N-terminal amino acid of IL-21 with the N-terminal amino acid of IL-4 to obtain a more stable N-terminal conformation of IL-21, thereby improving the stability of IL-21 or a fusion thereof and further improving the druggability.

In some embodiments, amino acids 1-15 of the IL-21 mutant protein at the Helix A (N-terminus) can be replaced with amino acids at the N-terminus of IL-4. In one embodiment, the IL-21 mutant protein comprises the following replacement:
a substitution of amino acids at positions 1-15, 1-14, 1-13, 1-12, 1-11, 1-10, 1-9, 1-8, 1-7, 1-6, 1-5, or 1-4 of the N-terminus with amino acids at the N-terminus of IL-4.

In some embodiments, the positions of the amino acids at the N-terminus of IL-4 used for substitution are the same as the amino acid positions of IL-21 it replaces. For example, the IL-21 mutant protein of the present invention comprises:
a substitution of amino acids at positions 1-15 of IL-21 with amino acids at positions 1-15 of IL-4;
a substitution of amino acids at positions 1-14 of IL-21 with amino acids at positions 1-14 of IL-4;
a substitution of amino acids at positions 1-13 of IL-21 with amino acids at positions 1-13 of IL-4;
a substitution of amino acids at positions 1-12 of IL-21 with amino acids at positions 1-12 of IL-4;
a substitution of amino acids at positions 1-11 of IL-21 with amino acids at positions 1-11 of IL-4;
a substitution of amino acids at positions 1-10 of IL-21 with amino acids at positions 1-10 of IL-4;
a substitution of amino acids at positions 1-9 of IL-21 with amino acids at positions 1-9 of IL-4;
a substitution of amino acids at positions 1-8 of IL-21 with amino acids at positions 1-8 of IL-4;
a substitution of amino acids at positions 1-7 of IL-21 with amino acids at positions 1-7 of IL-4;
a substitution of amino acids at positions 1-6 of IL-21 with amino acids at positions 1-6 of IL-4;
a substitution of amino acids at positions 1-5 of IL-21 with amino acids at positions 1-5 of IL-4; or
a substitution of amino acids at positions 1-4 of IL-21 with amino acids at positions 1-4 of IL-4.

In some embodiments, the number of amino acids at the N-terminus of IL-4 used for substitution differs from the number of amino acids of IL-21 it replaces by 1-3 amino acids. For example, the IL-21 mutant protein may comprise substitution of amino acids at positions 1-9 of IL-21 with amino acids at positions 1-11 of IL-4.

In some embodiments, the N-terminus of IL-4 used for replacement may comprise 1 or 2 substitutions. For example, the N-terminus of IL-4 used for replacement may comprise C3S.

When IL-4 is mentioned, it may be wild-type IL-4, or naturally occurring IL-4, e.g., IL-4 derived from human (uniprot: P05112). In some embodiments, the amino acid sequence of the IL-4 of the present invention is set forth in SEQ ID NO: 117. This expression also includes naturally occurring allelic and splice variants, isotypes, homologs, and species homologs of IL-4. This expression also includes variants of native IL-4. For example, the variants may have at least 95%-99% or more identity to native IL-4 (SEQ ID NO: 117) or have no more than 1-5 amino acid mutations (e.g., conservative substitutions), and preferably have substantially the same binding affinity for IL-4R as the native IL-4 protein.

In some specific embodiments, the amino acids at positions 1-15 of the N-terminus of IL-4 of the present invention are as follows: HKSDITLQEIIKTLN or HKCDITLQEIIKTLN.

In some preferred embodiments, the IL-21 mutant protein of the present invention comprises the following substitution/replacement:
replacement of amino acids at positions 1-5 of IL-21 with amino acids at positions 1-5 or amino acids comprising C3S at positions 1-5 of IL-4;
replacement of amino acids at positions 1-9 of IL-21 with amino acids at positions 1-9 or amino acids comprising C3S at positions 1-9 of IL-4;
replacement of amino acids at positions 1-15 of IL-21 with amino acids at positions 1-15 or amino acids comprising C3S at positions 1-15 of IL-4;
replacement of amino acids at positions 1-12 of IL-21 with amino acids at positions 1-12 or amino acids comprising CS3 at positions 1-12 of IL-4;
replacement of amino acids at positions 1-11 of IL-21 with amino acids at 1-11 or amino acids comprising C3S at positions 1-11 of IL-4; or
replacement of positions 1-9 of IL-21 with amino acids at positions 1-11 or amino acids comprising C3S at positions 1-11 of IL-4.

In some further preferred embodiments, the IL-21 mutant protein of the present invention comprises the following substitution/replacement:
replacement of 1-5 (QGQDR) of IL-21 with 1-5 & C3S (HKSDI) of IL-4;
replacement of 1-9 (QGQDRHMIR) of IL-21 with 1-9 & C3S (HKSDITLQE) of IL-4;
replacement of 1-15 (QGQDRHMIRMRQLID) of IL-21 with 1-15 & C3S (HKSDITLQEIIKTLN) of IL-4;
replacement of 1-12 (QGQDRHMIRMRQ) of IL-21 with 1-12 & C3S (HKSDITLQEIIK) of IL-4;
replacement of 1-11 (QGQDRHMIRMR) of IL-21 with 1-11 & C3S (HKSDITLQEII) of IL-4; or
replacement of 1-9 (QGQDRHMIR) of IL-21 with 1-11 & C3S (HKSDITLQEII) of IL-4.

### IL-21-glycosylation mutation

In some aspects, the glycosylation site mutations of IL-21 are changed by introducing N-glycosylation sites at a binding interface with IL-21R to reduce the binding affinity of IL-21 for its receptor IL-21R, thereby improving the stability of IL-21 or a fusion thereof and further improving the druggability.

The glycosylation site mutations suitable for the present invention are performed in at least 1, 2, 3, 4, or 5 positions selected from the following, resulting in glycosylation at the positions:
D4, R5, H6, M7, D15, V17, Q19, K21, D37, E39, R76, K77, P78, P79, S80, N82, G84, H120, and/or H122.

In some embodiments, the amino acids at the positions are mutated to N or T or S.

In some embodiments, the IL-21 mutant protein comprises 1, 2, 3, 4, or 5 substitutions selected from the following:
D4N, R5N, H6T, M7T, D15N, V17T, Q19N, K21T, D37N, E39T, R76N, K77N, P78T/S, P79T/N, S80N, N82T, G82T, G84T, H120N, and/or H122T.

In some preferred embodiments, the IL-21 mutant protein comprises a substitution or a combination of substitutions at the following position:
D4 & H6
R5 & M7
D15 & V17
IQ19 & K21
R76 & P78
K77 & P78 & P79
P79
S80 & N82
G84
H120 & H122; or
D37 & E39.

In some further preferred embodiments, the IL-21 mutant protein comprises a substitution or a combination of substitutions selected from the following:
D4N & H6T
R5N & M7T
D15N & V17T
IQ19N & K21T
R76N & P78T
K77N & P78S & P79T
P79N
S80N & N82T
G84T
H120N & H122T; and
D37N & E39T.

### IL-21 binding interface amino acid mutation

A substitution performed by introducing amino acids with similar charge or hydrophilicity/hydrophobicity may be introduced at a binding interface where IL-21 binds to IL-21R to change the binding property of the IL-21 mutant protein to IL-21R and reduce the binding affinity of IL-21 for its receptor IL-21R, thereby improving the druggability of IL-21 or a fusion thereof.

Examples of such mutations include, but are not limited to, substitutions at the IL-21 binding interface, particularly in Helix A, Helix C or C loop, especially at 1-5 positions, e.g., 1 or 2 positions, selected from the following: I8, K72, K77, P78, and/or P79.

In some preferred embodiments, the K can be substituted with D or E, the P can be substituted with E or A, or the I can be substituted with Q.

In some embodiments, the IL-21 mutant protein comprises 1-5, e.g., 1-2, of substitutions selected from the following: I8Q, K72E, K77D, P78A, and/or P79E.

In some preferred embodiments, the IL21 mutant protein comprises a substitution at a position or a combination of positions selected from the following:
K72;
I8 & P79; and
K77 & P78.

In some further preferred embodiments, the IL-21 mutant protein comprises the following substitution/combination of substitutions:

| |
|---|
| K72E |
| I8Q & P79E |
| K77D & P78A |

### IL-21 truncation mutation

In some aspects, the IL-21 mutant protein of the present invention comprises a deletion/truncation of one or more amino acids or amino acid segments in Helix A, CD loop, or C loop of IL-21 to change the local structure of IL-21 to enable a more stable local structure, thereby improving the stability of IL-21 and further improving the druggability of the IL-21 mutant protein or the fusion thereof.

In some embodiments, the IL-21 mutant protein of the present invention comprises a deletion of 1, 2, 3, 4, 5, 6, 7, 8, or 9 amino acids or a deletion of a segment comprising 1, 2, 3, 4, 5, 6, 7, 8, or 9 amino acids at the N-terminus of Helix A (e.g., positions 1-15), CD loop (e.g., positions 82-95, preferably 84-91), or C loop (e.g., positions 76-81).

In some embodiments, the IL-21 mutant protein of the present invention comprises a deletion of an amino acid segment at the following position:
a deletion at positions 1-9, positions 78-79, positions 85-87, or positions 84-91.

In some preferred embodiments, the mutant protein comprises a truncation/deletion of an amino acid segment at the following position or combination of positions:
truncation 85-87;
truncation 78-79;
truncation 1-9;
truncation 84-91; or
truncation 1-9 & truncation 78-79.

In some further preferred embodiments, the mutant protein comprises the following truncation/deletion:
truncation 85-87 (RRQ);
truncation 78-79 (PP);
truncation 1-9 (QGQDRHMIR);
truncation 84-91 (GRRQKHRL); or
truncation 1-9 (QGQDRHMIR) & truncation 78-79 (PP).

In some embodiments, in addition to the truncation/deletion, the mutant protein comprises a mutation to cysteine to remove disulfide bonds and increase the flexibility of the Loop. For example, the mutant protein may also comprise C42A and/or C93T. Thus, the mutant protein may comprise both truncation 84-91 (GRRQKHRI,) and C42A and C93T.

### Other mutations

In addition to the mutations described above, the IL-21 mutant protein of the present invention may also have one or more mutations in other regions or positions, as long as it retains one or more beneficial properties described above. For example, the IL-21 mutant protein of the present invention may also comprise a mutation of an original cysteine to other amino acids to reduce disulfide bonds, or a mutation of an original amino acid to cysteine. For example, the mutation may be L123C, C42A, or C93T. Those skilled in the art know how to determine additional mutations that can be incorporated into the IL-21 mutant protein of the present invention.

### Preferred exemplary combinations of mutations

The IL-21 mutant protein of the present invention may also comprise a combination of one or more of the mutation types described herein, or a combination of one or more of the mutation types described herein with other mutations (e.g., other mutations known in the art), and have improved properties.

In a preferred embodiment, the IL-21 mutant protein of the present invention comprises, relative to the wild type, at least two of the mutations described herein. For example, it comprises the following combination of mutations described herein:
(1) an IL-21/IL-4 chimera mutation and an IL-21 glycosylation mutation;
(2) an IL-21/IL-4 chimera mutation and an IL-21 interface amino acid mutation;
(3) an IL-21/IL-4 chimera mutation and an IL-21 truncation mutation;
(4) an IL-21 glycosylation mutation and an IL-21 interface amino acid mutation;
(5) an IL-21 glycosylation mutation and an IL-21 truncation mutation; or
(6) an IL-21 interface amino acid mutation and an IL-21 truncation mutation.

Optionally, the IL-21 mutant protein of the present invention also comprises other mutations, e.g., C42A and C93T. For example, the IL-21 mutant protein of the present invention may comprise an IL-21 interface amino acid mutation, an IL-21 truncation mutation, and C42A and C93T; or comprise an IL-21/IL-4 chimera mutation and L123C.

In some embodiments, the IL-21 mutant protein of the present invention comprises, relative to the wild type, a mutation or a combination of mutations selected from the following:
IL-21 truncation 1-9 (QGQDRHMIR) & Q19N & K21T;
IL-21 truncation 84-91 (GRRQKHRL) & C42A & C93T & Q 19N & K21T;
replacement of 1-9 (QGQDRHMIR) of IL-21 with 1-9 & C3S (HKSDITLQE) of IL-4 & truncation 78-79 (PP);
replacement of 1-9 (QGQDRHMIR) of IL-21 with 1-9 & C3S (HKSDITLQE) of IL-4 & K117N & I119T;
replacement of 1-9 (QGQDRHMIR) of IL-21 with 1-9 & C3 S (HKSDITLQE) of IL-4 & D37N & E39T;
replacement of 1-9 (QGQDRHMIR) of IL-21 with 1-9 & C3S (HKSDITLQE) of IL-4 & D15N & V17T; and
replacement of 1-5 (QGQDR) of IL-21 with 1-5 (HKCDI) of IL-4 & L123C.

The sequence difference between the IL-21 mutant protein and the wild-type protein can be expressed in terms of sequence identity or in terms of the number of different amino acids between the two. In one embodiment, the IL-21 mutant protein has at least 85%, 86%, 87%, 88%, or 89% identity, preferably 90% or more identity, preferably 95% but no more than 97%, and more preferably no more than 96% identity to the wild-type protein. In another embodiment, in addition to the four types of mutations described above in the present invention, the IL-21 mutant protein may also have no more than 15, e.g., 1-10, or 1-5, e.g., 0, 1, 2, 3, or 4, mutations relative to the wild-type protein. Preferably, the other mutations may be conservative substitutions.

### The IL-21 mutant protein of the present invention

Thus, the present invention provides a mutant protein comprising or consisting of an amino acid sequence selected from SEQ ID NOs: 75-105. The IL-21 mutant protein of the present invention also comprises an amino acid sequence having at least 85%, 86%, 87%, 88%, or 89% identity, preferably 90% or more identity, preferably 95% but not more than 97%, and more preferably no more than 96% identity to the amino acid sequence selected from SEQ ID NOs: 75-105, provided that these amino acid sequences contain the particular mutations described herein and have one or more of the following improved properties compared to the wild-type protein:
(i) lower affinity;
(ii) higher stability;
(iii) improved druggability (e.g., longer half-life and/or improved purity, e.g., SEC purity); and/or
(iv) upon formation of a fusion protein with an antibody or an antigen binding fragment thereof directed against an antigen, increased selective activation of cells positive for the antigen.

### Fusion of the mutant protein of the present invention

In one aspect, the present invention also provides a fusion of the IL-21 mutant protein of the present invention, e.g., a fusion protein. In one embodiment, the IL-21 mutant protein of the present invention can be linked to an Fc fragment of an antibody or to an intact antibody or an antigen binding fragment thereof to obtain an IL-21 mutant protein fusion protein.

In one preferred embodiment, the IL-21 mutant protein of the present invention is fused to another polypeptide, e.g., an antibody Fc fragment, which can provide improved pharmacokinetic properties. Thus, in one embodiment, the present invention provides an IL-21 mutant protein-Fc fusion protein comprising the IL-21 mutant protein of the present invention linked to an antibody Fc fragment.

The Fc fragment for use in the present invention may comprise a mutation that reduces or eliminates effector functions. In one preferred embodiment, the Fc fragment has reduced Fc-mediated effector functions, e.g., reduced or eliminated ADCC or ADCP or CDC effector functions. For example, in some particular embodiments, the Fc fragment for use in the present invention has an L234A/L235A mutation that reduces the binding to the Fcγ receptor.

In some embodiments, the Fc fragment fused to the IL-21 mutant protein is human IgG Fc, e.g., human IgG1 Fc, human IgG2 Fc, or human IgG4 Fc. Preferably, the Fc fragment is human IgG1 Fc, e.g., human IgG1 Fc comprising the L234A/L235A mutation. In one embodiment, the Fc fragment comprises or consists of an amino acid sequence set forth in SEQ ID NO: 70 or an amino acid sequence having at least 90% identity, e.g., 95%, 96%, 97%, 99% or more identity thereto.

In some embodiments, the IL-21 mutant protein is fused directly to Fc. In some embodiments, the IL-21 mutant protein can be linked to the Fc via a linker.

In some embodiments, the IL-21 mutant protein-Fc fusion protein of the present invention comprises the following chain A: the IL-21 mutant protein linked to the N-terminus of the antibody Fc fragment via a linker or directly. Preferably, the fusion protein comprises two identical chains A. In some other embodiments, the fusion protein comprises two different chains A or comprises one chain A and an Fc fragment that is not linked to the IL-21 mutant protein.

In some embodiments, the IL-21 mutant protein-Fc fusion protein of the present invention comprises two different chains A or comprises one chain A and one Fc fragment that is not linked to the IL-21 mutant protein. For example, on the basis of the Knob-in-Hole technique, Knob is introduced in the Fc fragment of the first chain A and a Hole mutation is introduced in the Fc fragment of the other chain A or in the other Fc fragment, or vice versa.

In some specific embodiments, the IL-21 mutant protein fusion protein of the present invention fused to the Fc fragment has properties of the mutant protein of the present invention, e.g., reduced affinity for the Fc receptor, higher stability, and/or improved druggability (such as prolonged half-life or increased production purity), compared to the wild-type IL-21 fusion protein.

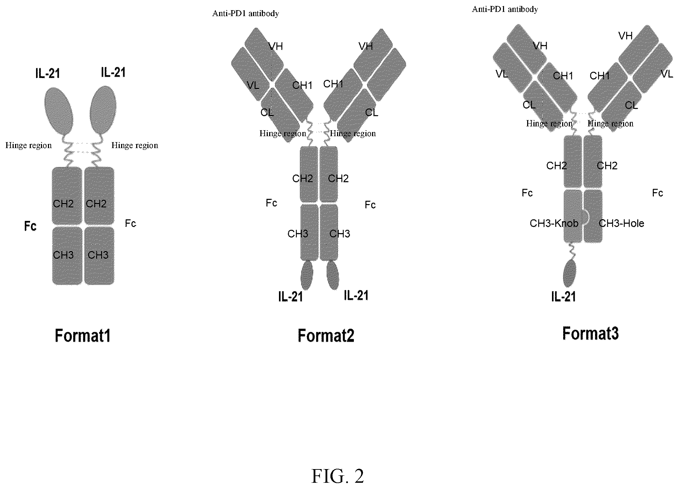

In one specific embodiment, the IL-21 mutant protein fusion protein of the present invention has a form of Format 1 as shown in FIG. 2.

As understood by those skilled in the art, the Fc fragment suitable for the fusion protein of the present invention may be any antibody Fc fragment.

In some specific embodiments, the chain A of the IL-21 mutant protein fusion protein of the present invention fused to Fc comprises or consists of an amino acid sequence set forth in any one of SEQ ID NOs: 2-32.

In another preferred embodiment, the present invention also provides an IL-21 mutant protein-antibody fusion protein comprising an IL-21 mutant protein and an antibody or an antigen binding fragment thereof linked thereto.

The IL-21 mutant protein fusion protein of the present invention formed by the IL-21 mutant protein of the present invention and an antibody or an antigen binding fragment thereof has properties of the mutant protein of the present invention, such as reduced affinity for the receptor, higher stability, improved druggability (e.g., prolonged half-life or increased production purity), and/or has increased selective activation of cells positive for an antigen against which the antibody is directed, compared to the corresponding wild-type IL-21 fusion protein.

In some embodiments, the antibody is an antibody directed against a tumor-associated antigen, e.g., PD-1, PD-L1, or PD-L2.

The IL-21 mutant protein described herein may be linked to an antibody or an antigen binding fragment thereof directly or via a linker. In some embodiments, the IL-21 mutant protein of the present invention is linked to the C-terminus of the antibody or the antigen binding fragment thereof.

The antibody suitable for linking to the IL-21 mutant protein may be an intact antibody or an antigen binding fragment thereof. In some embodiments, the antibody of the present invention is an antibody in the form of IgG1, IgG2, IgG3, or IgG4, preferably an antibody in the form of IgG1. In some embodiments, the antibody of the present invention is a monoclonal antibody. In some embodiments, the antibody of the present invention is humanized. In some embodiments, the antibody of the present invention is a human antibody. In some embodiments, the antibody of the present invention is a chimeric antibody. In one embodiment, the antigen binding fragment of the antibody of the present invention is selected from the following antibody fragments: Fab, Fab', Fab'-SH, Fv, a single-chain antibody (e.g., scFv), (Fab')₂, a single-domain antibody (e.g., VHH), a domain antibody (dAb), and a linear antibody. In one specific embodiment of the present invention, the IL-21 mutant protein described herein is linked to the C-terminus or N-terminus of one or both heavy chains of an intact antibody to form an IL-21 mutant protein-antibody fusion protein. In one embodiment of the present invention, the IL-21 mutant protein-antibody fusion protein described herein comprises an IL-21 mutant protein linked to the C-termini of the two heavy chains of an antibody. In one embodiment of the present invention, the IL-21 mutant protein-antibody fusion protein described herein comprises an IL-21 mutant protein linked to the C-terminus of one heavy chain of the antibody, and one antibody heavy chain. Preferably, a knob-into-hole structure is introduced in both heavy chains of the antibody to facilitate the formation of a heterodimer. In some embodiments, the antibody heavy chain linked to the IL-21 mutant protein comprises a Knob mutation and the antibody heavy chain not linked to the IL-21 mutant protein comprises a hole mutation, or vice versa. In some embodiments, the Knob mutation is a Knob: S354C & T366W, and/or the Hole mutation is Y349C & T366S & L368A & Y407V

In one embodiment of the present invention, the antibody or the antigen binding fragment thereof directed against PD-1 is an anti-PD-1 antibody or an antigen binding fragment thereof disclosed in CN201680040482.0. In one embodiment, the anti-PD-1 antibody or the antigen binding fragment thereof comprises one or more CDRs (preferably 3 CDRs, i.e., HCDR1, HCDR2H, and HCDR3, or LCDR1, LCDR2, and LCDR3; and more preferably 6 CDRs, i.e., HCDR1, HCDR2, HCDR3, LCDR1, LCDR2, and LCDR3) of the anti-PD-1 antibody or the antigen binding fragment thereof disclosed in CN201680040482.0, or comprises a VH and/or a VL of the anti-PD-1 antibody or the antigen binding fragment thereof disclosed in CN201680040482.0, or comprises a heavy chain and/or a light chain of the antibody. In some embodiments, the anti-PD-1 antibody or the antigen binding fragment thereof comprises 3 complementarity determining regions from a heavy chain variable region (HCDRs): HCDR1, HCDR2, and HCDR3. In some embodiments, the anti-PD-1 antibody or the antigen binding fragment thereof comprises 3 complementarity determining regions from a light chain variable region (LCDRs): LCDR1, LCDR2, and LCDR3. In some embodiments, the anti-PD-1 antibody or the antigen binding fragment thereof comprises 3 complementarity determining regions from a heavy chain variable region (HCDRs) and 3 complementarity determining regions from a light chain variable region (LCDRs).

In some aspects, the anti-PD-1 antibody or the antigen binding fragment thereof comprises a heavy chain variable region (VH). In some aspects, the anti-PD-1 antibody or the antigen binding fragment thereof comprises a light chain variable region (VH). In some aspects, the anti-PD-1 antibody or the antigen binding fragment thereof comprises a heavy chain variable region (VH) and a light chain variable region (VL). In some embodiments, the heavy chain variable region comprises 3 complementarity determining regions (CDRs) from the heavy chain variable region, HCDR1, HCDR2, and HCDR3. In some embodiments, the light chain variable region comprises 3 complementarity determining regions (CDRs) from the light chain variable region, LCDR1, LCDR2, and LCDR3.

In some embodiments, the anti-PD-1 antibody or the antigen binding fragment thereof comprises an antibody heavy chain. In some embodiments, the anti-PD-1 antibody or the antigen binding fragment thereof of the present invention comprises an antibody light chain. In some embodiments, the anti-PD-1 antibody or the antigen binding fragment thereof of the present invention also comprises a heavy chain and a light chain.

In some embodiments, the heavy chain variable region (VH)
(i) comprises or consists of an amino acid sequence having at least 90%, 91%, 92%, 93%, 94%, 95%, 96%, 97%, 98%, or 99% identity to an amino acid sequence set forth in SEQ ID NO: 115; or
(ii) comprises or consists of an amino acid sequence set forth in SEQ ID NO: 115; or
(iii) comprises or consists of an amino acid sequence having one or more (preferably no more than 10, and more preferably no more than 5, 4, 3, 2, or 1) amino acid changes (preferably amino acid replacements, and more preferably conservative amino acid replacements) compared to an amino acid sequence set forth in SEQ ID NO: 115, wherein preferably, the amino acid changes do not occur in the CDRs.

In some embodiments, the light chain variable region (VL)
(i) comprises or consists of an amino acid sequence having at least 90%, 91%, 92%, 93%, 94%, 95%, 96%, 97%, 98%, or 99% identity to an amino acid sequence set forth in SEQ ID NO: 116; or
(ii) comprises or consists of an amino acid sequence set forth in SEQ ID NO: 116; or
(iii) comprises or consists of an amino acid sequence having one or more (preferably no more than 10, and more preferably no more than 5, 4, 3, 2, or 1) amino acid changes (preferably amino acid replacements, and more preferably conservative amino acid replacements) compared to an amino acid sequence set forth in SEQ ID NO: 116, wherein preferably, the amino acid changes do not occur in the CDRs.

In some embodiments, the 3 complementarity determining regions from the heavy chain variable region (HCDRs), HCDR1, HCDR2 and HCDR3, are selected from
(i) three complementarity determining regions HCDR1, HCDR2, and HCDR3 comprised in a VH set forth in SEQ ID NO: 115, or
(ii) relative to the sequence in (i), a sequence comprising a total of at least one and no more than 5, 4, 3, 2, or 1 amino acid change (preferably amino acid replacement, and more preferably conservative replacement) in the three HCDRs.

In some embodiments, the 3 complementarity determining regions from the light chain variable region (LCDRs), LCDR1, LCDR2, and LCDR3, are selected from
(i) three complementarity determining regions LCDR1, LCDR2, and LCDR3 comprised in a VL set forth in SEQ ID NO: 116, or
(ii) relative to the sequence in (i), a sequence comprising a total of at least one and no more than 5, 4, 3, 2, or 1 amino acid change (preferably amino acid replacement, and more preferably conservative replacement) in the three LCDRs.

In some embodiments, the HCDR1 comprises or consists of an amino acid sequence set forth in SEQ ID NO: 109, or the HCDR1 comprises an amino acid sequence having one, two, or three changes (preferably amino acid replacements, and more preferably conservative replacements) compared to the amino acid sequence set forth in SEQ ID NO: 109.

In some embodiments, the HCDR2 comprises or consists of an amino acid sequence set forth in SEQ ID NO: 110, or the HCDR2 comprises an amino acid sequence having one, two, or three changes (preferably amino acid replacements, and more preferably conservative replacements) compared to the amino acid sequence set forth in SEQ ID NO: 110.

In some embodiments, the HCDR3 comprises or consists of an amino acid sequence set forth in SEQ ID NO: 111, or the HCDR3 comprises an amino acid sequence having one, two, or three changes (preferably amino acid replacements, and more preferably conservative replacements) compared to the amino acid sequence set forth in SEQ ID NO: 111.

In some embodiments, the LCDR1 comprises or consists of an amino acid sequence set forth in SEQ ID NO: 112, or the LCDR1 comprises an amino acid sequence having one, two, or three changes (preferably amino acid replacements, and more preferably conservative replacements) compared to the amino acid sequence set forth in SEQ ID NO: 112.

In some embodiments, the LCDR2 comprises or consists of an amino acid sequence set forth in SEQ ID NO: 113, or the LCDR2 comprises an amino acid sequence having one, two, or three changes (preferably amino acid replacements, and more preferably conservative replacements) compared to the amino acid sequence set forth in SEQ ID NO: 113.

In some embodiments, the LCDR3 comprises or consists of an amino acid sequence set forth in SEQ ID NO: 114, or the LCDR3 comprises an amino acid sequence having one, two, or three changes (preferably amino acid replacements, and more preferably conservative replacements) compared to the amino acid sequence set forth in SEQ ID NO: 114.

In some embodiments, the heavy chain constant region (HC) of the anti-PD-1 antibody or the antigen binding fragment thereof is a heavy chain constant region of IgG1, IgG2, IgG3, or IgG4, preferably a heavy chain constant region of IgG1, e.g., a heavy chain constant region of IgG1 with an LALA mutation. In some embodiments, a knob-into-hole mutation is introduced into the heavy chain constant region. For example, a mutation of S354C and T366W is introduced in one heavy chain constant region to obtain an antibody heavy chain comprising a knob mutation, and/or a mutation of Y349C & T366S & L368A & Y407V is introduced in another heavy chain constant region to obtain an antibody heavy chain comprising a hole mutation. In some embodiments, the light chain constant region of the anti-PD-1 antibody or the antigen binding fragment thereof is a lambda or Kappa light chain constant region.

In some embodiments, the heavy chain of the antibody or the antigen binding fragment thereof
(i) comprises or consists of an amino acid sequence having at least 85%, 90%, 91%, 92%, 93%, 94%, 95%, 96%, 97%, 98%, or 99% identity to an amino acid sequence selected from SEQ ID NO: 71;
(ii) comprises or consists of an amino acid sequence selected from SEQ ID NO: 71; or
(iii) comprises or consists of an amino acid sequence having one or more (preferably no more than 20 or 10, and more preferably no more than 5, 4, 3, 2, or 1) amino acid changes (preferably amino acid replacements, and more preferably conservative amino acid replacements) compared to an amino acid sequence selected from SEQ ID NO: 71.

In some embodiments, the heavy chain of the antibody or the antigen binding fragment thereof comprising a knob mutation
(i) comprises or consists of an amino acid sequence having at least 85%, 90%, 91%, 92%, 93%, 94%, 95%, 96%, 97%, 98%, or 99% identity to an amino acid sequence selected from SEQ ID NO: 73;
(ii) comprises or consists of an amino acid sequence selected from SEQ ID NO: 73; or
(iii) comprises or consists of an amino acid sequence having one or more (preferably no more than 20 or 10, and more preferably no more than 5, 4, 3, 2, or 1) amino acid changes (preferably amino acid replacements, and more preferably conservative amino acid replacements) compared to an amino acid sequence selected from SEQ ID NO: 73.

In some embodiments, the heavy chain of the antibody or the antigen binding fragment thereof comprising a hole mutation
(i) comprises or consists of an amino acid sequence having at least 85%, 90%, 91%, 92%, 93%, 94%, 95%, 96%, 97%, 98%, or 99% identity to an amino acid sequence selected from SEQ ID NO: 33;
(ii) comprises or consists of an amino acid sequence selected from SEQ ID NO: 33; or
(iii) comprises or consists of an amino acid sequence having one or more (preferably no more than 20 or 10, and more preferably no more than 5, 4, 3, 2, or 1) amino acid changes (preferably amino acid replacements, and more preferably conservative amino acid replacements) compared to an amino acid sequence selected from SEQ ID NO: 33.

In some embodiments, the light chain of the antibody or the antigen binding fragment thereof
(i) comprises or consists of an amino acid sequence having at least 85%, 90%, 91%, 92%, 93%, 94%, 95%, 96%, 97%, 98%, or 99% identity to an amino acid sequence selected from SEQ ID NO: 34;
(ii) comprises or consists of an amino acid sequence selected from SEQ ID NO: 34; or
(iii) comprises or consists of an amino acid sequence having one or more (preferably no more than 20 or 10, and more preferably no more than 5, 4, 3, 2, or 1) amino acid changes (preferably amino acid replacements, and more preferably conservative amino acid replacements) compared to an amino acid sequence selected from SEQ ID NO: 34.

In some specific embodiments of the present invention, the anti-PD-1 antibody or the antigen binding fragment thereof comprises:
three complementarity determining regions HCDR1, HCDR2, and HCDR3 comprised in a VH set forth in SEQ ID NO: 115, and three complementarity determining regions LCDR1, LCDR2, and LCDR3 comprised in a VL set forth in SEQ ID NO: 116.

In some specific embodiments of the present invention, the anti-PD-1 antibody or the antigen binding fragment thereof comprises:
HCDR1, HCDR2, and HCDR3 set forth in amino acid sequences of SEQ ID NOs: 109, 110, and 111, respectively, and LCDR1, LCDR2, and LCDR3 set forth in amino acid sequences of SEQ ID NOs: 112, 113, and 114, respectively.

In some specific embodiments of the present invention, the anti-PD-1 antibody or the antigen binding fragment thereof comprises:
a VH comprising or consisting of an amino acid sequence set forth in SEQ ID NO: 115 or an amino acid sequence having at least 90%, 91%, 92%, 93%, 94%, 95%, 96%, 97%, 98%, or 99% identity thereto, and a VL comprising or consisting of an amino acid sequence set forth in SEQ ID NO: 116 or an amino acid sequence having at least 90%, 91%, 92%, 93%, 94%, 95%, 96%, 97%, 98%, or 99% identity thereto.

In some specific embodiments of the present invention, the anti-PD-1 antibody or the antigen binding fragment thereof comprises:
(ii) a heavy chain comprising or consisting of an amino acid sequence set forth in SEQ ID NO: 71 or an amino acid sequence having at least 85%, 90%, 91%, 92%, 93%, 94%, 95%, 96%, 97%, 98%, or 99% identity thereto; and a light chain comprising or consisting of an amino acid sequence set forth in SEQ ID NO: 34 or an amino acid sequence having at least 85%, 90%, 91%, 92%, 93%, 94%, 95%, 96%, 97%, 98%, or 99% identity thereto.

In some preferred embodiments, the anti-PD-1 antibody or the antigen binding fragment thereof comprises two identical heavy chains and two identical light chains. In some specific embodiments of the present invention, the anti-PD-1 antibody or the antigen binding fragment thereof comprises:
a heavy chain with a knob mutation comprising or consisting of an amino acid sequence set forth in SEQ ID NO: 73 or an amino acid sequence having at least 85%, 90%, 91%, 92%, 93%, 94%, 95%, 96%, 97%, 98%, or 99% identity thereto;
a heavy chain with a hole mutation comprising or consisting of an amino acid sequence set forth in SEQ ID NO: 33 or an amino acid sequence having at least 85%, 90%, 91%, 92%, 93%, 94%, 95%, 96%, 97%, 98%, or 99% identity thereto; and
a light chain comprising or consisting of an amino acid sequence set forth in SEQ ID NO: 34 or an amino acid sequence having at least 85%, 90%, 91%, 92%, 93%, 94%, 95%, 96%, 97%, 98%, or 99% identity thereto.

In some preferred embodiments, the anti-PD-1 antibody or the antigen binding fragment thereof comprises one heavy chain comprising a hole mutation, one heavy chain comprising a knob mutation, and the two identical light chains.

In one specific embodiment of the present invention, the IL-21 mutant protein fusion protein described herein comprises the following structure:
chain A: a light chain of the antibody; and
chain B: the IL-21 mutant protein of the present invention linked to the C-terminus of a heavy chain of the antibody via a linker or directly.

In one embodiment, the IL-21 mutant protein in chain B is linked to the C-terminus of the heavy chain of the antibody via a linker.

In one embodiment, the mutant protein fusion protein described herein comprises two identical chains A and two identical chains B.

In one embodiment, the mutant protein fusion protein described herein has a structure of Format 2 as shown in FIG. 2.

In one embodiment of the present invention, the antibody from which chain A and chain B are derived is an antibody directed against PD-1, PD-L1, or PD-L2. In some embodiments of the present invention, the antibody from which chain A and chain B are derived is an anti-PD-L1 antibody disclosed in CN201680040482.0. In some embodiments of the present invention, the antibody from which chain A and chain B are derived comprises 6 CDRs of an anti-PD-1 antibody disclosed in CN201680040482.0, or comprises a VH and/or a VL of the antibody. In some embodiments of the present invention, the antibody from which chain A and chain B are derived comprises a heavy chain constant region of IgG1, e.g., IgG1 with an LALA mutation.

In some embodiments, chain A comprises LCDR1, LCDR2, and/or LCDR3 of a light chain variable region of the anti-PD-1 antibody or the antigen binding fragment thereof described above. In some embodiments, chain A comprises a light chain variable region of the anti-PD-1 antibody or the antigen binding fragment thereof described above. In some embodiments, chain A comprises a light chain constant region of the anti-PD-1 antibody or the antigen binding fragment thereof described above. In some embodiments, chain A comprises or is a light chain of the anti-PD-1 antibody or the antigen binding fragment thereof described above. In one embodiment, chain A comprises an amino acid sequence set forth in SEQ ID NO: 34, or an amino acid sequence having at least 85%, 90%, 91%, 92%, 93%, 94%, 95%, 96%, 97%, 98%, 99%, or 100% identity thereto.

In some embodiments, the antibody heavy chain in chain B comprises HCDR1, HCDR2, and/or HCDR3 of a heavy chain variable region of the anti-PD-1 antibody or the antigen binding fragment thereof described above. In some embodiments, the antibody heavy chain in chain B comprises a heavy chain variable region of the anti-PD-1 antibody or the antigen binding fragment thereof described above. In some embodiments, the antibody heavy chain in chain B comprises a heavy chain constant region of the anti-PD-1 antibody or the antigen binding fragment thereof described above. In some embodiments, the antibody heavy chain in chain B comprises a heavy chain of the anti-PD-1 antibody or the antigen binding fragment thereof described above. In one embodiment, the antibody heavy chain comprised in chain B comprises or consists of an amino acid sequence set forth in SEQ ID NO: 71, or an amino acid sequence having at least 85%, 90%, 91%, 92%, 93%, 94%, 95%, 96%, 97%, 98%, 99%, or 100% identity thereto.

In some embodiments, the IL-21 mutant protein comprised in chain B comprises or consists of an amino acid sequence set forth in any one of SEQ ID NOs: 75-105, or an amino acid sequence having at least 85%, 90%, 91%, 92%, 93%, 94%, 95%, 96%, 97%, 98%, 99%, or 100% identity thereto, and comprises the mutation described herein.

In some embodiments, chain B comprises or consists of an amino acid sequence set forth in any one of SEQ ID NOs: 35-54, or an amino acid sequence having at least 85%, 90%, 91%, 92%, 93%, 94%, 95%, 96%, 97%, 98%, 99% or 100% identity thereto.

In one specific embodiment of the present invention, the IL-21 mutant protein fusion protein described herein comprises the following structure:
chain A: a light chain of the antibody;
chain B1: the IL-21 mutant protein linked to the C-terminus of one heavy chain of the antibody; and
chain B2: a heavy chain of the antibody.

In one embodiment, the IL-21 mutant protein fusion protein described herein comprises two chains A, one chain B1, and one chain B2.

In one embodiment, a knob-into-hole mutation is introduced in chain B1 and chain B2 to facilitate the formation of a heterodimer by chains B1 and B2. In one embodiment, the knob mutation is comprised in chain B1 and the hole mutation is comprised in chain B2. In one embodiment, the knob mutation is a mutation of S354C and T366W and/or the hole mutation is Y349C & T366S & L368A & Y407V In one embodiment, the IL-21 mutant protein fusion protein described herein has a structure of Format 3 as shown in FIG. 2.

In one embodiment of the present invention, the antibody from which chain A and chains B1 and B2 are derived is an antibody directed against PD-1, PD-L1, or PD-L2. In some embodiments of the present invention, the antibody from which chain A and chains B1 and B2 are derived is an anti-PD-L1 antibody disclosed in CN201680040482.0. In some embodiments of the present invention, the antibody from which chain A and chains B 1 and B2 are derived comprises 6 CDRs of an anti-PD-1 antibody disclosed in CN201680040482.0, or comprises a VH and/or a VL of the antibody. In some embodiments of the present invention, the antibody from which chain A and chain B are derived comprises a heavy chain constant region of IgG1, e.g., IgG1 with an LALA mutation.

In some embodiments, chain A comprises LCDR1, LCDR2, and/or LCDR3 of a light chain variable region of the anti-PD-1 antibody or the antigen binding fragment thereof described above. In some embodiments, chain A comprises a light chain variable region of the anti-PD-1 antibody or the antigen binding fragment thereof described above. In some embodiments, chain A comprises a light chain constant region of the anti-PD-1 antibody or the antigen binding fragment thereof described above. In some embodiments, chain A comprises or is a light chain of the anti-PD-1 antibody or the antigen binding fragment thereof described above. In one embodiment, chain A comprises an amino acid sequence set forth in SEQ ID NO: 34, or an amino acid sequence having at least 85%, 90%, 91%, 92%, 93%, 94%, 95%, 96%, 97%, 98%, 99%, or 100% identity thereto.

In some embodiments, chain B1 or chain B2 each comprises HCDR1, HCDR2, and/or HCDR3 of a heavy chain variable region of the anti-PD-1 antibody or the antigen binding fragment thereof described above. In some embodiments, chain B 1 or chain B2 each comprises a heavy chain variable region of the anti-PD-1 antibody or the antigen binding fragment thereof described above. In some embodiments, chain B1 or chain B2 each comprises a heavy chain constant region of the anti-PD-1 antibody or the antigen binding fragment thereof described above. In some embodiments, chain B1 comprises a heavy chain comprising a knob mutation of the anti-PD-1 antibody or the antigen binding fragment thereof described above. In some embodiments, chain B2 comprises chain B1 comprises a heavy chain comprising a hole mutation of the anti-PD-1 antibody or the antigen binding fragment thereof described above.

In one embodiment, the antibody heavy chain comprised in chain B1 comprises an amino acid sequence set forth in SEQ ID NO: 73, or an amino acid sequence having at least 85%, 90%, 91%, 92%, 93%, 94%, 95%, 96%, 97%, 98%, 99%, or 100% identity thereto. In some embodiments, the IL-21 mutant protein comprised in chain B1 comprises or consists of an amino acid sequence set forth in any one of SEQ ID NOs: 75-105, or an amino acid sequence having at least 85%, 90%, 91%, 92%, 93%, 94%, 95%, 96%, 97%, 98%, 99%, or 100% identity thereto, and comprises the mutation described herein.

In some embodiments, chain B1 comprises or consists of an amino acid sequence set forth in any one of SEQ ID NOs: 55-67, or an amino acid sequence having at least 85%, 90%, 91%, 92%, 93%, 94%, 95%, 96%, 97%, 98%, 99%, or 100% identity thereto.

In one embodiment, chain B2 comprises an amino acid sequence set forth in SEQ ID NO: 33, or an amino acid sequence having at least 85%, 90%, 91%, 92%, 93%, 94%, 95%, 96%, 97%, 98%, 99%, or 100% identity thereto.

As will be understood by those skilled in the art, linkers suitable for linking the IL-21 mutant protein to the Fc fragment or the antibody in the fusion of the present invention may be any linker known in the art. In some embodiments, the linker may comprise a linker sequence selected from the following: (GS)n, (GSGGS)n, (GGGGS)n, and (GGGS)n, wherein n is an integer of at least 1, preferably, the linker comprises (G4S)₂ or (G4S)₃.

### III. Polynucleotide, Vector, and Host

The present invention provides a nucleic acid encoding any one of the above IL-21 mutant proteins or fusions. The polynucleotide sequence encoding the mutant protein of the present invention can be generated by *de novo* solid phase DNA synthesis or by PCR mutagenesis of an existing sequence encoding the wild-type IL-21 using methods well known in the art. In addition, the polynucleotide and the nucleic acid of the present invention may comprise a segment encoding a secretion signal peptide and are operably linked to a segment encoding the mutant protein of the present invention, so that secretory expression of the mutant protein of the present invention can be directed.

The present invention also provides a vector comprising the nucleic acid of the present invention. In one embodiment, the vector is an expression vector, e.g., a eukaryotic expression vector. The vector includes, but is not limited to, a virus, a plasmid, a cosmid, a λ phage or a yeast artificial chromosome (YAC). In a preferred embodiment, the expression vector of the present invention is a pcDNA3.1 expression vector.

The present invention also provides a host cell comprising the nucleic acid or the vector. Host cells suitable for replicating and supporting the expression of the IL-21 mutant protein or the fusion are well known in the art. Such cells can be transfected or transduced with a particular expression vector, and a large number of cells comprising vectors can be cultivated for inoculation in large-scale fermenters, so as to obtain sufficient IL-21 mutant proteins or fusions for clinical application. In one embodiment, the host cell is eukaryotic. In another embodiment, the host cell is selected from a yeast cell and a mammalian cell (e.g., a CHO cell or a 293 cell). Examples of available mammalian host cell lines include SV40 transformed monkey kidney CV1 line (COS-7); human embryonic kidney line (293 or 293T cells, e.g., Expi293 cells), baby hamster kidney cells (BHK), mouse Sertoli cells (TM4 cells), monkey kidney cells (CV1), African green monkey kidney cells (VERO-76), human cervical cancer cells (HELA), canine kidney cells (MDCK), buffalo rat liver cells (BRL3A), human lung cells (W138), human liver cells (HepG2), mouse mammary tumor cells (MMT060562), TRI cells, MRC5 cells, and FS4 cells. Other available mammalian host cell lines include Chinese hamster ovary (CHO) cells, including dhfr-CHO cells, and myeloma cell lines such as YO, NS0, P3X63, and Sp2/0. In one embodiment, the host cell is a eukaryotic cell, preferably a mammalian cell such as an HEK293 cell (Expi293), a Chinese hamster ovary (CHO) cell, a human embryonic kidney (HEK) cell, or a lymphocyte (e.g., Y0, NS0, or Sp20 cell).

### IV. Preparation Method

In a further aspect, the present invention provides a method for preparing the IL-21 mutant protein or the fusion of the present invention, wherein the method comprises culturing a host cell comprising a nucleic acid encoding the protein or the fusion under conditions suitable for expression of the IL-21 mutant protein or the fusion, as provided above, and optionally isolating the protein or the fusion from the host cell (or the host cell culture medium).

### V. Assay

The IL-21 mutant protein or the fusion thereof provided herein can be identified, screened, or characterized for its physical/chemical properties and/or biological activities through a variety of assays known in the art.

In one aspect, the IL-21 mutant protein or the fusion thereof of the present invention can be tested for its binding activity, e.g., affinity, for the IL-21 receptor. For example, the binding to human IL-21R can be determined by methods known in the art, such as ELISA and Western blot, or by exemplary methods disclosed in the examples herein. Alternatively, the binding of the mutant protein to the receptor, including binding kinetics (e.g., K_{D} value), can be determined by using an IL-21-Fc fusion protein or a fusion protein of IL-21 and an antibody in a BLI assay.

In a further aspect, the ability of the IL-21 mutant protein or the fusion thereof to bind to the IL-21 receptor can be measured indirectly by measuring the signaling and/or immune activation downstream of receptor binding.

Thus, in some embodiments, an assay for identifying mutant IL-21 proteins or fusions thereof having a biological activity is provided. Biological activity can include, for example, the ability to induce IL-21 signaling in T cells having IL-21 receptors. The present invention also provides a mutant IL-21 protein or a fusion thereof having such biological activity *in vivo* and/or *in vitro.*

A variety of methods known in the art can be used for determining the biological activity of the IL-21 or the fusion thereof, for example, for testing the ability of the IL-21 mutant protein or the fusion thereof of the present invention to induce STAT3 phosphorylation.

In a further aspect, the druggability (e.g., product purity) of the mutant protein or the fusion thereof of the present invention can be characterized by methods known in the art. For the determination of the product purity, the purity can be determined after one-step purification of the collected culture supernatant of the production cells to determine the purification performance of the mutant protein. Preferably, the mutant protein of the present invention, after being purified, has significantly superior purity to that of the wild-type protein, indicating that the mutant protein of the present invention has better purification performance. The purity determination method can be any conventional method known in the art, including but not limited to, the SEC-HPLC method.

In a further aspect, the pharmacokinetic properties, e.g., half-life, of the mutant protein or the fusion thereof of the present invention can be characterized by methods known in the art.

### VI. Method for Engineering IL-21 Protein

In a further aspect, the present invention provides a method for obtaining an IL-21 mutant protein having improved properties and the IL-21 mutant protein obtained by this method.

In one embodiment, the method of the present invention comprises the following steps:
(a) obtaining an amino acid sequence of an IL-21 mutant protein by one or more of the following mutations of the present invention;
   1. an IL-21/IL-4 chimera mutation;
   2. an IL-21 glycosylation mutation;
   3. an IL-21 interface amino acid mutation; and/or
   4. an IL-21 truncation mutation;
   wherein optionally, the method also comprises linking the IL-21 mutant protein to other proteins or polypeptides, e.g., an Fc fragment or an antibody or an antigen binding fragment thereof, to obtain an IL-21 mutant protein fusion;
(b) synthesizing a nucleic acid molecule encoding the IL-21 mutant protein, or synthesizing a nucleic acid molecule encoding strands of the IL-21 mutant protein fusion; and
(c) introducing the nucleic acid molecule from (b) into a mammalian cell (e.g., an HEK293 cell or a CHO cell) to express the IL-21 mutant protein or the fusion thereof. In one embodiment, the method of the present invention also comprises the step of identifying the following properties of the mutant protein or the fusion thereof after the steps (a)-(c): (i) protein purity after purification (e.g., purity after one-step affinity chromatography as detected by SEC-HPLC); (ii) reduced binding, and optionally (iii) a prolonged half-life.

In one embodiment, the method comprises: before performing the combination of mutations in steps (a)-(c), identifying activation of cells (e.g., T cells) by the mutant protein or the fusion thereof, e.g., binding to the JAK-STAT signaling pathway, e.g., STAT3 phosphorylation.

### VII. Pharmaceutical Composition and Pharmaceutical Preparation

The present invention also comprises a composition (including a pharmaceutical composition or a pharmaceutical preparation) comprising the IL-21 mutant protein or the fusion thereof, and a composition comprising the polynucleotide encoding the IL-21 mutant protein or the fusion thereof. Such compositions can also optionally comprise suitable pharmaceutical auxiliary materials, such as pharmaceutical carriers and pharmaceutical excipients known in the art, including buffers.

### VIII. Combination Product

In one aspect, the present invention also provides a combination product comprising the mutant protein or the fusion thereof of the present invention, and one or more additional therapeutic agents (e.g., a chemotherapeutic agent, other antibodies, a cytotoxic agent, a vaccine, an anti-infective active agent, and the like). The combination product of the present invention can be used in the therapeutic method of the present invention.

In some embodiments, the combination product is used for preventing or treating cancer.

### IX. Therapeutic Method and Use

In one aspect, the present invention relates to a method for preventing or treating a disease, such as cancer, in a subject, wherein the method comprises administering to the subject an effective amount of any of the IL-21 mutant proteins or the fusions thereof described herein. The cancer may be at an early, intermediate, or advanced stage, or may be a metastatic cancer. In some embodiments, the cancer may be a solid tumor or a hematological tumor.

The IL-21 mutant protein or the fusion thereof of the present invention (and the pharmaceutical composition comprising the same, and optionally an additional therapeutic agent) can be administered by any suitable method, including parenteral administration, intrapulmonary administration, intranasal administration, and, if required by locoregional treatment, intralesional administration. Parenteral infusion includes intramuscular, intravenous, intra-arterial, intraperitoneal, or subcutaneous administration. The administration may be performed by any suitable means, such as injection, e.g., intravenous or subcutaneous injection, to some extent depending on whether the treatment is short-term or long-term. Various administration schedules are encompassed herein, including but not limited to, single administration or multiple administrations at multiple time points, bolus injection, and pulse infusion.

In order to prevent or treat a disease, the appropriate dosage of the IL-21 mutant protein of the present invention (used alone or in combination with one or more additional therapeutic agents) will depend on the type of the disease to be treated, the type of the antibody, severity and progression of the disease, the purpose for which the antibody is administered (prevention or treatment), previous treatments, clinical history of a patient, responses to the antibody, and the discretion of an attending physician. The antibody is suitably administered to a patient through a single treatment or through a series of treatments.

In a further aspect, the present invention also provides use of the IL-21 mutant protein, the composition, and the fusion of the present invention in preparing a medicament for use in the method described above (e.g., for treatment).

The following examples are described to assist in understanding the present invention. The examples are not intended to be and should not be explained in any way as limiting the protection scope of the present invention.

These and other aspects and embodiments of the present invention are illustrated in the drawings (brief description of the drawings follows) and in the following detailed description of the present invention and are described in the following examples. Any or all of the features described above and throughout the present application may be combined in various embodiments of the present invention. The following examples further illustrate the present invention. However, it should be understood that the examples are described by way of illustration rather than limitation, and various modifications may be made by those skilled in the art.

### Examples

### Example 1: Design and construction of IL-21 mutants

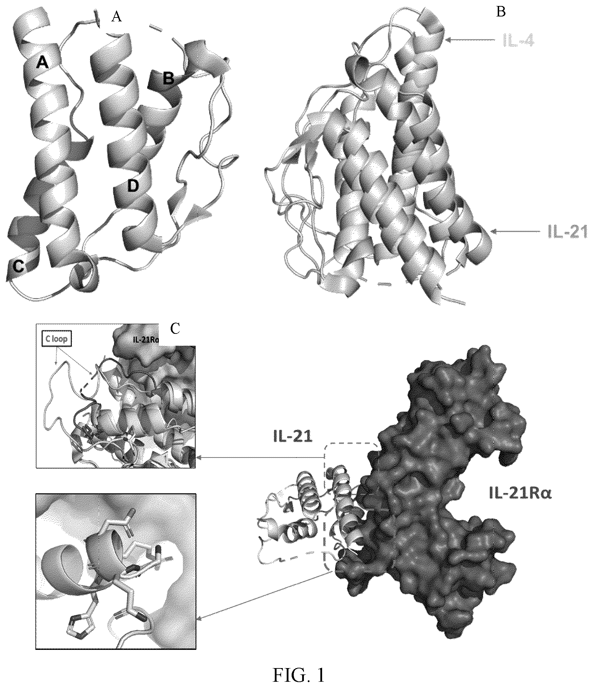

Interleukin 21 (IL-21) is a cytokine of a barrel structure consisting of 4 α helix chains (FIG. 1A), and is mainly secreted by activated CD4+ Th cells and NKT cells *in vivo.* It has high homology to IL-2, IL-4, and IL-15, all belonging to the γc receptor family of cytokines. The IL-21 receptor consists of two chains: IL-21Rα and IL-2Ryc. The binding interface of IL-21 with IL-21Rα is helix A, helix C, and a portion of CD Loop (FIG. 1C). To weaken the binding of IL-21 to IL-21Rα, we designed 4 classes of IL-21 mutants for wild-type IL-21 (uniprot: Q9HBE4, SEQ ID NO: 74) by reducing or weakening the interacting residues of IL-21 with the receptor, and validated them using 3 molecular forms (Formats). Specifically, the designed mutations mainly include the following mutant forms:
1) an IL-21/IL-4 chimera, obtained by partial secondary structure replacement of IL-21 with IL-4, i.e., replacement of the N-terminus of IL-21 with N-terminal amino acids of IL-4, on the basis of the structural homology of IL-21 to IL-4;
2) an IL-21 glycosylation mutant, obtained by introducing N-glycosylation site mutations on the interface of IL-21 with a receptor thereof;
3) an IL-21 interface amino acid mutant;
4) an IL-21 truncation mutant; and
5) a combinatorial mutant, including at least any two or three or all four of the above.

The wild-type protein and the mutant proteins of IL-21 described above were each linked to Fc (from IgG1, LALA mutation, SEQ ID NO: 70) via a linker (G4S)₂ (SEQ ID NO: 69), and two chains were connected by disulfide bonds to construct a fusion protein form of Format I; the wild-type protein and the mutant proteins of IL-21 were each directly linked to the C-terminus of a heavy chain of an anti-PD-1 antibody (from CN201680040482.0, the sequence of a heavy chain HC is set forth in SEQ ID NO: 71, and the sequence of a light chain LC is set forth in SEQ ID NO: 34) to construct a fusion protein form of Format II; or the wild-type protein and the mutant proteins of IL-21 were each linked to the C-terminus of a knob heavy chain of an anti-PD-1 antibody with knob-into-hole (from CN201680040482.0, the sequence of a light chain LC is set forth in SEQ ID NO: 34, the sequence of a knob heavy chain (namely HC-knob, IgG1 form, with an LALA mutation) is set forth in SEQ ID NO: 73, and the sequence of a hole heavy chain (namely HC-hole, IgG1 form, with an LALA mutation) is set forth in SEQ ID NO: 33) via a linker (G₄S)₃ (SEQ ID NO: 72) to construct a fusion protein form of Format III. The fusion protein forms of Formats I, II, and III are shown in FIG. 2.

The details of the mutations and Formats described above are as follows:

### Design of IL-21/IL-4 chimera mutation

The NMR structure of IL-21 (PDB: 2OQP) showed that the N-terminus of IL-21 was more active and did not have a relatively stable conformation. By structural comparison, we found that IL-4 had a higher structural similarity to IL-21 and had a more stable N-terminal conformation (FIG. 1B). Thus, we designed to replace N-terminal amino acids of IL-21 with N-terminal amino acids of IL-4 to obtain a more stable N-terminal conformation of IL-21, and optimized the druggability of molecules.

The specific design is shown in Table 1 below:

**Table 1: Engineering and sequence information on IL-21 mutant proteins**

| Molecule-I D | SEQ ID NO | Engineering information |
|---|---|---|
| IL-21-001 | 74 | Wild type |
| IL-21-002 | 75 | Replacement of 1-5 (QGQDR) of IL-21 with 1-5 & C3S (HKSDI) of IL-4 |
| IL-21-004 | 77 | Replacement of 1-9 (QGQDRHMIR) of IL-21 with 1-9 & C3S (HKSDITLQE) of IL-4 |
| IL-21-005 | 78 | Replacement of 1-15 (QGQDRHMIRMRQLID) of IL-21 with 1-15 & C3S (HKSDITLQEIIKTLN) of IL-4 |
| IL-21-006 | 79 | Replacement of 1-12 (QGQDRHMIRMRQ) of IL-21 with 1-12 & C3S (HKSDITLQEIIK) of IL-4 |
| IL-21-007 | 80 | Replacement of 1-11 (QGQDRHMIRMR) of IL-21 with 1-11 & C3S (HKSDITLQEII) of IL-4 |
| IL-21-008 | 81 | Replacement of 1-9 (QGQDRHMIR) of IL-21 with 1-11 & C3S (HKSDITLQEII) of IL-4 |

**Table 2. Information on fusion protein molecules of different Format forms**

| Molecule-I D | Format | Sequence information* | IL-21 applied |
|---|---|---|---|
| 001 | Format 1 | SEQ ID NO:1 | IL-21-001 |
| 002 | Format 1 | SEQ ID NO:2 | IL-21-002 |
| 004 | Format 1 | SEQ ID NO:4 | IL-21-004 |
| 005 | Format 1 | SEQ ID NO:5 | IL-21-005 |
| 006 | Format 1 | SEQ ID NO:6 | IL-21-006 |
| 007 | Format 1 | SEQ ID NO:7 | IL-21-007 |
| 008 | Format 1 | SEQ ID NO:8 | IL-21-008 |
| 033 | Format2 | Heavy chain: SEQ ID NO: 35; light chain: SEQ ID NO: 34 | IL-21-001 |
| 034 | Format2 | Heavy chain: SEQ ID NO: 36; light chain: SEQ ID NO: 34 | IL-21-002 |
| 035 | Format2 | Heavy chain: SEQ ID NO: 37; light chain: SEQ ID NO: 34 | IL-21-003 |
| 049 | Format2 | Heavy chain: SEQ ID NO: 51; light chain: SEQ ID NO: 34 | IL-21-004 |
| 053 | Format3 | Heavy chain knob: SEQ ID NO: 55; heavy chain hole: SEQ ID NO: 33; light chain: SEQ ID NO: 34 | IL-21-004 |
| 054 | Format3 | Heavy chain knob: SEQ ID NO: 56; heavy chain hole: SEQ ID NO: 33; light chain: SEQ ID NO: 34 | IL-21-005 |
| 055 | Format3 | Heavy chain knob: SEQ ID NO: 57; heavy chain hole: SEQ ID NO: 33; light chain: SEQ ID NO: 34 | IL-21-006 |
| 056 | Format3 | Heavy chain knob: SEQ ID NO: 58; heavy chain hole: SEQ ID NO: 33; light chain: SEQ ID NO: 34 | IL-21-007 |
| 062 | Format3 | Heavy chain knob: SEQ ID NO: 64; heavy chain hole: SEQ ID NO: 33; light chain: SEQ ID NO: 34 | IL-21-008 |

| | | | |
|---|---|---|---|
| * In Format 1, the sequence of only one chain is listed here, and the other chain has the same sequence; in Format 2, the heavy chain refers to a heavy chain comprising the IL-21 mutant protein and an antibody heavy chain, and the light chain refers to an antibody light chain; and in Format 3, the heavy chain knob refers to a heavy chain comprising the IL-21 mutant protein and an antibody heavy chain comprising the Knob mutation, the heavy chain hole refers to an antibody heavy chain comprising the Hole mutation, and the light chain refers to an antibody light chain. | | | |

### IL-21 glycosylation mutant

IL-21 has a short half-life *in vivo* due to adsorption of IL-21R to the cell surface (sink effect). The inventors found that the introduction of glycosylation sites at the binding interface of IL-21 with a receptor thereof to introduce glycosylation exerted a repulsive effect on the mutual binding between the IL-21 and the receptor thereof, so that the affinity of the IL-21 for the receptor thereof was reduced, and the half-life of the IL-21 *in vivo* was prolonged; meanwhile, the introduction of glycosylation on the surface of IL-21 could improve the aggregation state of molecules, so that the druggability of the molecules was optimized.

**Table 3: Engineering and sequence information on IL-21 mutant proteins**

| | SEQ ID NO: | Engineering information |
|---|---|---|
| IL-21-009 | 82 | IL21.glycan1 (D4N & H6T) |
| IL-21-010 | 83 | IL21.glycan2 (R5N & M7T) |
| IL-21-011 | 84 | IL21.glycan3 (D15N & V17T) |
| IL-21-012 | 85 | IL21.glycan4 (Q19N & K21T) |
| IL-21-013 | 86 | IL21.glycan5 (R76N & P78T) |
| IL-21-014 | 87 | IL21.glycan6 (K77N & P78S & P79T) |
| IL-21-015 | 88 | IL21.glycan7 (P79N) |
| IL-21-016 | 89 | IL21.glycan8 (S80N & N82T) |
| IL-21-017 | 90 | IL21.glycan9 (G84T) |
| IL-21-018 | 91 | IL21.glycan10 (H120N & H122T) |
| IL-21-019 | 92 | IL21.glycan11 (D37N & E39T) |

**Table 4. Information on fusion protein molecules of different Format forms**

| Molecule-ID | Format | Sequence information* | IL-21 applied |
|---|---|---|---|
| 009 | Format1 | SEQ ID NO:9 | IL-21-009 |
| 010 | Format1 | SEQ ID NO: 10 | IL-21-010 |
| 011 | Format1 | SEQ ID NO:11 | IL-21-011 |
| 012 | Format1 | SEQ ID NO:12 | IL-21-012 |
| 013 | Format1 | SEQ ID NO: 13 | IL-21-013 |
| 014 | Format1 | SEQ ID NO:14 | IL-21-014 |
| 015 | Format1 | SEQ ID NO:15 | IL-21-015 |
| 016 | Format1 | SEQ ID NO:16 | IL-21-016 |
| 017 | Format1 | SEQ ID NO:17 | IL-21-017 |
| 018 | Format1 | SEQ ID NO:18 | IL-21-018 |
| 019 | Format1 | SEQ ID NO:19 | IL-21-019 |
| 036 | Format2 | Heavy chain: SEQ ID NO: 38; light chain: SEQ ID NO: 34 | IL-21-009 |
| 037 | Format2 | Heavy chain: SEQ ID NO: 39; light chain: SEQ ID NO: 34 | IL-21-010 |
| 038 | Format2 | Heavy chain: SEQ ID NO: 40; light chain: SEQ ID NO: 34 | IL-21-011 |
| 039 | Format2 | Heavy chain: SEQ ID NO: 41; light chain: SEQ ID NO: 34 | IL-21-012 |
| 040 | Format2 | Heavy chain: SEQ ID NO: 42; light chain: SEQ ID NO: 34 | IL-21-013 |
| 041 | Format2 | Heavy chain: SEQ ID NO: 43; light chain: SEQ ID NO: 34 | IL-21-014 |
| 042 | Format2 | Heavy chain: SEQ ID NO: 44; light chain: SEQ ID NO: 34 | IL-21-015 |
| 043 | Format2 | Heavy chain: SEQ ID NO: 45; light chain: SEQ ID NO: 34 | IL-21-016 |
| 044 | Format2 | Heavy chain: SEQ ID NO: 46; light chain: SEQ ID NO: 34 | IL-21-017 |
| 045 | Format2 | Heavy chain: SEQ ID NO: 47; light chain: SEQ ID NO: 34 | IL-21-018 |
| 046 | Format2 | Heavy chain: SEQ ID NO: 48; light chain: SEQ ID NO: 34 | IL-21-019 |

| | | | |
|---|---|---|---|
| * In Format 1, the sequence of only one chain is listed here, and the other chain has the same sequence; and in Format 2, the heavy chain refers to a heavy chain comprising the IL-21 mutant protein and an antibody heavy chain, and the light chain refers to an antibody light chain. | | | |

### IL-21 interface amino acid mutant

At the binding interface of IL-21 with IL-21R, amino acids at the corresponding positions were mutated by amino acids with similar charge or hydrophilicity and hydrophobicity, so that the affinity of IL-21 for a receptor thereof was reduced, and the druggability of molecules was optimized.

**Table 5: Engineering and sequence information on IL-21 mutant proteins**

| | SEQ ID NO: | Engineering information |
|---|---|---|
| IL-21-020 | 93 | IL21.K72E |
| IL-21-021 | 94 | IL21.I8Q & P79E |
| IL-21-022 | 95 | IL21.K77D & P78A |

**Table 6. Information on fusion protein molecules of different Format forms**

| Molecule -ID | Format | Sequence information* | IL-21 applied |
|---|---|---|---|
| 020 | Format1 | SEQ ID NO:20 | IL-21-020 |
| 021 | Format1 | SEQ ID NO:21 | IL-21-021 |
| 022 | Format1 | SEQ ID NO:22 | IL-21-022 |
| 050 | Format2 | Heavy chain: SEQ ID NO: 52; light chain: SEQ ID NO: 34 | IL-21-020 |
| 051 | Format2 | Heavy chain: SEQ ID NO: 53; light chain: SEQ ID NO: 34 | IL-21-021 |
| 052 | Format2 | Heavy chain: SEQ ID NO: 54; light chain: SEQ ID NO: 34 | IL-21-022 |

| | | | |
|---|---|---|---|
| * In Format 1, the sequence of only one chain is listed here, and the other chain has the same sequence; and in Format 2, the heavy chain refers to a heavy chain comprising the IL-21 mutant protein and an antibody heavy chain, and the light chain refers to an antibody light chain. | | | |

### IL-21 truncation mutant

By truncating Helix A, CD loop, or C loop, a more stable local structure was obtained, so that the stability of IL-21 could be increased, and the druggability (e.g., SEC purity) of molecules was improved.

**Table 7: Engineering and sequence information on IL-21 mutant proteins**

| | SEQ ID NO: | Engineering information |
|---|---|---|
| IL-21-023 | SEQ ID NO:96 | IL21 truncation 85-87 (RRQ) |
| IL-21-024 | SEQ ID NO:97 | IL21 truncation 78-79 (PP) |
| IL-21-025 | SEQ ID NO:98 | IL-21 truncation 1-9 (QGQDRHMIR) |
| IL-21-026 | SEQ ID NO:99 | IL-21 truncation 1-9 (QGQDRHMIR) & truncation 78-79 (PP) |

**Table 8. Information on fusion protein molecules of different Format forms**

| Molec ule-ID | Format | Sequence information* | IL-21 applied |
|---|---|---|---|
| 023 | Format1 | SEQ ID NO:23 | IL-21-0 23 |
| 024 | Format1 | SEQ ID NO:24 | IL-21-0 24 |
| 025 | Format1 | SEQ ID NO:25 | IL-21-0 25 |
| 026 | Format1 | SEQ ID NO:26 | IL-21-0 26 |
| 047 | Format2 | Heavy chain: SEQ ID NO: 49; light chain: SEQ ID NO: 34 | IL-21-0 23 |
| 048 | Format2 | Heavy chain: SEQ ID NO: 50; light chain: SEQ ID NO: 34 | IL-21-0 24 |
| 058 | Format3 | Heavy chain knob: SEQ ID NO: 60; heavy chain hole: SEQ ID NO: 33; light chain: SEQ ID NO: 34 | IL-21-0 25 |
| 060 | Format3 | Heavy chain knob: SEQ ID NO: 62; heavy chain hole: SEQ ID NO: 33; light chain: SEQ ID NO: 34 | IL-21-0 26 |

| | | | |
|---|---|---|---|
| * In Format 1, the sequence of only one chain is listed here, and the other chain has the same sequence; in Format 2, the heavy chain refers to a heavy chain comprising the IL-21 mutant protein and an antibody heavy chain, and the light chain refers to an antibody light chain; and in Format 3, the heavy chain knob refers to a heavy chain comprising the IL-21 mutant protein and an antibody heavy chain comprising the Knob mutation, the heavy chain hole refers to an antibody heavy chain comprising the Hole mutation, and the light chain refers to an antibody light chain. | | | |

### Combinatorial mutant

The inventors further combined the above mutations to obtain combinatorial mutants with better stability and/or lower affinity, so that the druggability or activity of molecules was improved.

**Table 9: Engineering and sequence information on IL-21 mutant proteins**

| | SEQ ID NO: | Engineering information |
|---|---|---|
| IL-21-02 7 | 100 | IL-21 truncation 1-9 (QGQDRHMIR) & Q19N & K21T |
| IL-21-02 8 | 101 | IL-21 truncation 84-91 (GRRQKHRL) & C42A & C93T & Q19N & K21T |
| IL-21-02 9 | 102 | Replacement of 1-9 (QGQDRHMIR) of IL-21 with 1-9 & C3S (HKSDITLQE) of IL-4 & truncation 78-79 (PP) |
| IL-21-03 0 | 103 | Replacement of 1-9 (QGQDRHMIR) of IL-21 with 1-9 & C3S (HKSDITLQE) of IL-4 & K117N & I119T |
| IL-21-03 1 | 104 | Replacement of 1-9 (QGQDRHMIR) of IL-21 with 1-9 & C3S (HKSDITLQE) of IL-4 & D37N & E39T |
| IL-21-03 2 | 105 | Replacement of 1-9 (QGQDRHMIR) of IL-21 with 1-9 & C3S (HKSDITLQE) of IL-4 & D15N & V17T |
| IL-21-00 3 | 76 | Replacement of 1-5 (QGQDR) of IL-21 with 1-5 (HKCDI) of IL-4 & L123C |

**Table 10. Information on fusion protein molecules of different Format forms**

| Molecule-ID | Format | Sequence information* | IL-21 applied |
|---|---|---|---|
| 027 | Format 1 | SEQ ID NO:27 | IL-21-027 |
| 028 | Format 1 | SEQ ID NO:28 | IL-21-028 |
| 029 | Format 1 | SEQ ID NO:29 | IL-21-029 |
| 030 | Format 1 | SEQ ID NO:30 | IL-21-030 |
| 031 | Format 1 | SEQ ID NO:31 | IL-21-031 |
| 032 | Format 1 | SEQ ID NO:32 | IL-21-032 |
| 059 | Format3 | Heavy chain knob: SEQ ID NO: 61; heavy chain hole: SEQ ID NO: 33; light chain: SEQ ID NO: 34 | IL-21-027 |
| 061 | Format3 | Heavy chain knob: SEQ ID NO: 63; heavy chain hole: SEQ ID NO: 33; light chain: SEQ ID NO: 34 | IL-21-028 |
| 057 | Format3 | Heavy chain knob: SEQ ID NO: 59; heavy chain hole: SEQ ID NO: 33; light chain: SEQ ID NO: 34 | IL-21-029 |
| 063 | Format3 | Heavy chain knob: SEQ ID NO: 65; heavy chain hole: SEQ ID NO: 33; light chain: SEQ ID NO: 34 | IL-21-030 |
| 064 | Format3 | Heavy chain knob: SEQ ID NO: 66; heavy chain hole: SEQ ID NO: 33; light chain: SEQ ID NO: 34 | IL-21-031 |
| 065 | Format3 | Heavy chain knob: SEQ ID NO: 67; heavy chain hole: SEQ ID NO: 33; light chain: SEQ ID NO: 34 | IL-21-032 |
| 003 | Format 1 | SEQ ID NO:3 | IL-21-003 |

| | | | |
|---|---|---|---|
| * In Format 1, the sequence of only one chain is listed here, and the other chain has the same sequence; and in Format 3, the heavy chain knob refers to a heavy chain comprising the IL-21 mutant protein and an antibody heavy chain comprising the Knob mutation, the heavy chain hole refers to an antibody heavy chain comprising the Hole mutation, and the light chain refers to an antibody light chain. | | | |

Example 2: Expression and purification of IL-21 mutants

### Construction of expression plasmids

The sequences of chains of the fusion protein molecules 001-065 were synthesized by Genewiz and separately constructed into pCDNA3.1 template plasmids. A certain number of plasmids were prepared for transient expression of cells.

Expi293 cells (Thermo) were passaged according to a desired transfection volume. The cell density was adjusted to 1.5 × 10⁶ cells/mL the day before transfection. The cell density on the day of transfection was approximately 3 × 10⁶ cells/mL. 1/10 (v/v) of the final volume of Opti-MEM medium (Gibco, Catalog No. 31985-070) was taken as a transfection buffer. The expression plasmids constructed described above were added. The mixture was mixed homogeneously and filtered through a 0.22 µm filter for later use. An appropriate amount of polyethylenimine (PEI) (Polysciences, 23966) was added to the plasmids from the previous step (the mass ratio of plasmids to PEI was 1:3). The mixture was mixed homogeneously and incubated at room temperature for 10 min to give a DNA/PEI mixture. The DNA/PEI mixture was gently poured into Expi293 cells (for the fusion protein molecules in the Format I form, the plasmid pCNDA3.1 comprising the chains was transfected into the cells; and for the fusion protein molecules in the forms of Formats II and III, the plasmid pCNDA3.1 comprising the heavy chain and the plasmid pCNDA3.1 comprising the light chain were co-transfected into the cells). The mixture was mixed homogeneously and incubated at 37 °C with 8% CO₂ for 24 h. Then, VPA (Sigma; Catalog No. P4543-100G) was added to reach a final concentration of 2 mM, followed by 2% (v/v) Feed (1 g/L Phytone Peptone + 1 g/L Difco Select Phytone). The mixture was incubated for another 6 days.

Protein purification: the cell supernatant was collected by centrifugation. The supernatant was purified using a pre-packed column Hitrap Mabselect Sure (GE, 11-0034-95) according to the manufacturer's instructions. The procedures were as follows: the packing column was equilibrated with a 5-fold column volume of equilibration buffer (20 mM Tris, 150 mM NaCl, pH 7.2) before purification; the collected supernatant was passed through the column, and then the column was washed with a 10-fold column volume of equilibration buffer to remove non-specific binding proteins; and the packing was washed with a 5-fold column volume of elution buffer (100 mM sodium citrate, pH 3.5), and the eluent was collected. 80 µL of Tris (2M Tris) was added per 1 mL of eluent, and the mixture was buffer-exchanged into PBS (Gibco, Catalog No. 70011-044) using an ultrafiltration concentration tube (MILLIPORE, Catalog No. UFC901096), and then the concentration was determined. 100 µg of purified protein was taken with its concentration adjusted to 1 mg/mL. The protein purity was determined by a gel filtration column SW3000 (TOSOH, Catalog No. 18675). The results are shown in Table 11.

**Table 11: SEC purity of IL-21 mutants**

| Molecule-ID | SEC |
|---|---|
| 033 control | 61% |
| 034 | 90.09% |
| 035 | 68.94% |
| 036 | 72.77% |
| 037 | 92.07% |
| 038 | 61.12% |
| 039 | 98.75% |
| 040 | 63.47% |
| 041 | 65.44% |
| 042 | 74.62% |
| 043 | 95.03% |
| 044 | 93.73% |
| 045 | 70.85% |
| 046 | 95.11% |
| 047 | 90.75% |
| 048 | 92.28% |
| 049 | 90.57% |
| 050 | 98.35% |
| 051 | 79.47% |
| 052 | 65.27% |
| 053 | 99.33% |
| 054 | 97.02% |
| 055 | 99.10% |
| 056 | 97.74% |
| 057 | 98.81% |
| 058 | 88.29% |
| 059 | 86.37% |
| 060 | 89.94% |
| 061 | 76.95% |
| 062 | 94.47% |
| 063 | 85.94% |
| 064 | 83.94% |
| 065 | 97.76% |

As can be seen from Table 11, the wild-type IL-21(033 control) only had 61% purity after one-step affinity purification; while the IL-21 mutants of the present invention all had purity superior to that of the wild type, and there were 21 molecules with 85% or higher purity among 034-065, including 034, 037, 039, 043-044, 046-050, 053-060, 062-063, and 065. In addition, the SEC purity of all or part of the molecules among 001-032 was similarly determined, and the results showed that these molecules all had SEC purity higher than that of the wild-type molecule 001 (data not shown). The results suggest that compared to the wild-type IL-21, the mutants of the present invention are more stable and less prone to aggregation, can achieve higher purity, and have improved druggability.

### Example 3: Affinity assay of IL-21 mutants for receptors thereof

The equilibrium dissociation constant (K_{D}) of the IL-21 mutants of the present invention for binding to human IL-21R (R&D systems) was determined using the bio-layer interferometry (BLI) technique. A BLI affinity assay was performed according to the existing method (Estep, P et al., High throughput solution based measurement of antibody-antigen affinity and epitope binding. *MAbs,* 2013.5(2): p. 270-8).

Half an hour before the experiment, an appropriate number of AHC (ForteBio, 18-5060) sensors were taken according to the number of samples and soaked in an SD buffer (PBS 1×, BSA 0.1%, and Tween-20 0.05%).

100 µL of SD buffer, IL-21 fusion protein (the molecules 033-065, wherein the molecule 033 is a wild-type IL-21 fusion protein as a control), and IL-21R (R&D systems, 9249-R2-100) were each added to 96-well black polystyrene half-area plate (Greiner, 675076). The sensors were arranged according to the positions of the samples. The instrument settings were as follows: the operation procedures were Baseline, Loading ~1 nm, Baseline, Association, and Dissociation; the run time of each procedure was dependent on the rates of association and dissociation of samples; and the rotation speed was 400 rpm, and the temperature was 30 °C. The K_{D} values were analyzed by ForteBio analysis software. The results are shown in Table 12.

**Table 12. Affinity of IL-21 mutants for IL-21 receptors**

| Molecule-ID | Affinity for IL-21R K_{D} (M) |
|---|---|
| 033 control | 8.29E-10 |
| 034 | 1.52E-08 |
| 035 | 4.54E-07 |
| 036 | 1.41E-09 |
| 037 | 2.89E-07 |
| 038 | 3.73E-06 |
| 039 | 1.48E-08 |
| 040 | N.B. |
| 041 | 2.25E-07 |
| 042 | 1.39E-08 |
| 043 | 4.57E-09 |
| 044 | 4.67E-09 |
| 045 | 2.84E-09 |
| 046 | 3.26E-09 |
| 047 | 2.07E-09 |
| 048 | 1.05E-05 |
| 049 | N.B. |
| 050 | 2.79E-09 |
| 051 | 2.57E-08 |
| 052 | 1.16E-08 |
| 053 | N.B. |
| 054 | N.B. |
| 055 | N.B. |
| 056 | N.B. |
| 057 | N.B. |
| 058 | N.B. |
| 059 | N.B. |
| 060 | N.B. |
| 061 | N.B. |
| 062 | N.B. |
| 063 | N.B. |
| 064 | N.B. |
| 065 | N.B. |

The data for the affinity of the IL-21 mutants for receptors thereof are shown in Table 12. The affinity K_{D} of wild-type IL-21 (033) for IL-21R was 8.29E-10M, while the IL-21 mutants obtained in this study all had a weak monovalent affinity for IL-21R, in which the molecules 002-032 had a K**_{D}** value of lower than 3.74E-10 or lower than the detection range of the instrument (shown as no binding (N.B.), data not shown), and part of the molecules 034-065 had a K**_{D}** value of lower than the detection range of the instrument (shown as no binding (N.B.) here) (Table 12).

IL-21 has a short half-life *in vivo.* One of the main reasons is that IL-21 has a high affinity for a cell surface receptor, which makes it susceptible to endocytosis and degradation. Thus, IL-21 mutants with a weakened receptor binding ability will have a longer half-life *in vivo.*

### Example 4: In vitro functional assay

### Experimental method:

I. Preparation of HuT78 PD1-positive cells
> The day before transfection, 293T cells (ATCC, CRL-3216) in logarithmic growth phase were selected and seeded into a T175 square flask (NUNC, 159910).
> The medium was discarded 1 h before transfection, and 18 mL of serum-free DMEM medium (HyClone, SH30022.01B) was added. The flask was put into an incubator (Thermo, 4111) at 37 °C with 5% CO₂.
> 1 mL of Opti-MEM (Gibco, 31985-070) and 105 µL of PEIpro (Polyplus, 115-100) were added to a centrifuge tube 1. The mixture was gently mixed homogeneously and incubated at room temperature for 5 min. 1 mL of Opti-MEM, 20 µg of lentiviral plasmids (hPD1-kozaka-cds in pLVX-IRES-EGFP) (Genewiz, Suzhou), 10 µg of pSPAX2 (Genewiz, Suzhou), and 5 µg of pMG2.G (Genewiz, Suzhou) were added to a centrifuge tube 2. The mixture was gently mixed homogeneously and incubated at room temperature for 5 min.
> The mixture in the two centrifuge tubes was mixed together, gently mixed, and incubated at room temperature for 20 min.
> The well mixed transfection mixture was added to the culture flask. The flask was gently shaken to homogenize the mixture and put into an incubator at 37 °C with 5% CO₂ for culturing.
> After 4-6 h of transfection, the medium in the culture flask was removed by pipetting, and 25 mL of DMEM medium containing 10% serum (HyClone, SH30406.05) and 1% bispecific antibody (Gibco, 15140-122) was added. The flask was put into an incubator at 37 °C with 5% CO₂ for culturing.
> After 48 h of transfection, the cell supernatant was collected and stored in a refrigerator at 4 °C, and 25 mL of DMEM medium containing 10% serum and 1% bispecific antibody was added to the original flask. The flask was put into an incubator at 37 °C with 5% CO₂ for culturing.
> After 72 h of transfection, the cell supernatant was collected again and mixed with the cell supernatant collected after 48 h of transfection.
> The mixed cell supernatant was centrifuged with a refrigerated centrifuge (Thermo Scientific, SA40R) at 2000 g for 10 min at 4 °C, and cell debris was removed.
> After centrifugation, the supernatant was filtered through a 0.22 µm low-protein binding filter membrane (sartorius, stedim, 16541-k).
> One third of the supernatant volume of concentration reagent (Takara, 631232) was added. The mixture was gently mixed homogeneously and left to stand overnight in a refrigerator at 4 °C.
> The mixture was centrifuged at 1500 g for 45 min at 4 °C and the supernatant was carefully discarded (be careful not to discard the viral pellet) to obtain the lentiviral pellet.
> The lentiviral pellet obtained from the previous step was resuspended with 1 mL of the desired infection cell medium (RPMI1640 (Hyclone, SH30809.01), 10% FBS (HyClone, SH30406.05), and 1% Pen Strep (Gibco, 15140-122)). The pellet was gently suspended without pipetting. The suspension was left to stand at 4 °C for 4 h to dissolve the virus. The lentivirus solution was obtained, subpackaged at 100 µL/tube, and cryopreserved at -80 °C.
> The day before infection, HuT78 cells (ATCC, TIB-161) in logarithmic growth phase were selected and seeded into a 6-well plate (NEST, 703001) at 3×10⁵ cells/3 mL/well.
> 100 µL of freeze-thawed lentivirus solution was added to each well. The mixture was gently mixed homogeneously, and the plate was put into an incubator at 37 °C with 5% CO₂ for 3 days to obtain PD-1-GFP positive cells, which were named HuT78-GFP-PD1.
> HuT78 and HuT78-GFP-PD1 cells were taken and centrifuged at 300 g for 5 min at room temperature, and the supernatant was removed.
> The positive rates for GFP and PD1 were determined by a flow cytometer (BD, FACS Symphony).

II. STAT3 assay on IL-21 mutants in HuT78 and HuT78-GFP-PD1 cells
> HuT78 and HuT78-GFP-PD1 cells were adjusted for the concentrations to 5×10⁶ cells/mL and mixed according to a ratio of 3:7 (HuT78:HuT78-GFP-PD1). The mixture was centrifuged at 300 g for 5min at room temperature, and the supernatant was removed.
> An equal volume of DCM diluent (L34963) was added to the cells from which the supernatant was removed. The plate was incubated at room temperature for 10 min in the dark.
> Three-fold volume of FACS buffer (500 mL of PBS (Gibco, 0020000034), 50 mL of FBS, and 5 mL of EDTA (invitrogen)) was added. The mixture was centrifuged at 300 g for 5 min at room temperature, and the supernatant was removed. An equal volume of medium (RPMI1640 (Hyclone, SH30809.01), 10% FBS (HyClone, SH30406.05), and 1% Pen Strep (Gibco, 15140-122)) was added for resuspension to obtain a cell mixture.
> 100 µL of the cell mixture prepared from the previous step, 100 µL of rhIL-21 (R&D systems, 8879-IL-050), or the molecules 033-065 of the present invention were added to each well. The mixture was mixed homogeneously, and the plate was incubated in a water bath at 37 °C for 20 min.
> Then, 50 µL of fixation solution (Pierce^{™} 16% formaldehyde (w/v), Methanol-free, 28906) diluted to 10% with PBS was added to each well, and the plate was incubated in a water bath at 37 °C for 10 min.
> The mixture was centrifuged at 600 g for 5 min at room temperature, and the supernatant was removed.
> 50 µL of Triton diluted to 0.05% with PBS was added to each well from which the supernatant was removed, and the plate was incubated on ice for 5 min for perforation.
> 200 µL of FACS buffer was added to each well. The mixture was centrifuged at 600 g for 5 min at room temperature, and the supernatant was removed.
> 200 µL of FACS buffer was added to each well. The mixture was centrifuged at 500 g for 4 min at room temperature, and the supernatant was removed.
> 200 µL of methanol (Sinopharm Chemical Reagent Co., Ltd., 10014118) was added to each well. The mixture was mixed homogeneously and left to stand overnight at -20 °C.
> The mixture was centrifuged at 600 g for 5 min at room temperature, and the supernatant was removed.
> 200 µL of FACS buffer was added to each well. The mixture was centrifuged at 600 g for 5 min at room temperature, and the supernatant was removed.
> 50 µL of STAT3 stain solution (FACS buffer + PE-conjugated pSTAT3 antibody) was added to each well for resuspension, and the plate was incubated at room temperature for 60 min in the dark.

| Target | Channel | Company | Catalog No. | Dilution ratio |
|---|---|---|---|---|
| DCM (dead cell marker) | PacB | Invitrogen | L34963 | 1:1000 |
| STAT3 | PE | BD | 562077 | 1:25 |

> 200 µL of FACS buffer was added to each well. The mixture was centrifuged at 600 g for 5 min at room temperature, and the supernatant was removed.
> 100 µL of FACS buffer was added to each well for resuspension, and STAT3+ positive cells were detected by a flow cytometer (BD, FACS Symphony).

### Experimental results:

The binding of IL-21 to an IL-21 receptor on the surface of T cells will activate the JAK-STAT signaling pathway in T lymphocytes, resulting in phosphorylation of STATS. Thus, the level of phosphorylated STAT3 (pSTAT3) was used to determine the degree of activation of this signaling pathway.

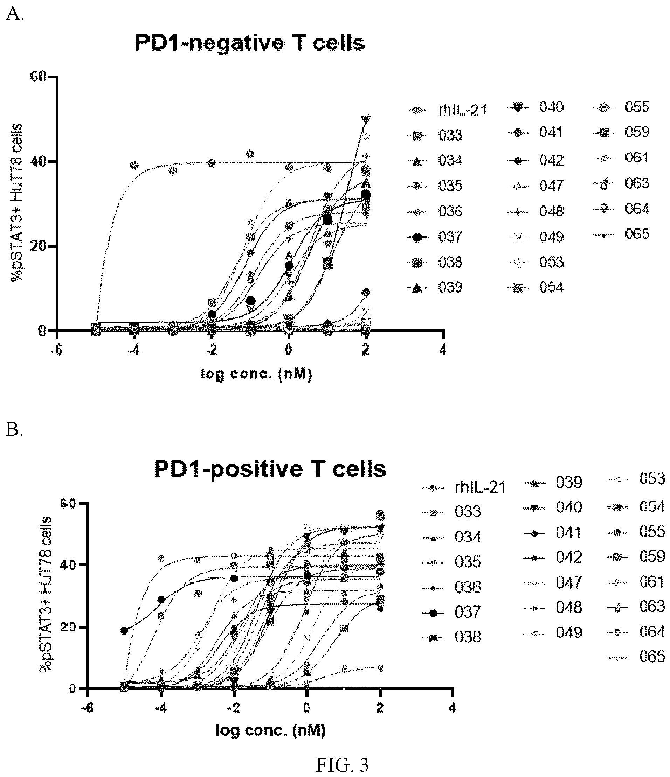

The following results were obtained (Table 13): IL21 in rhIL-21 and 033 was wild-type IL-21, which showed strong activation activity for the signaling pathway in PD1-negative HuT78 cells, with EC₅₀ of -1.61E-009 nM and 0.046 nM, respectively; while the mutants in the study all had an EC₅₀ value greater than that of the wild-type IL-21, that is, they showed lower activation activity for PD1-negative cells. For example, 041, 049, 053-055, 059, 061, and 063-064 showed very weak activation activity at 100 nM, and even no detectable activity. The results are shown in FIG. 3A.

In contrast, in PD1-positive HuT78 cells (HuT78-GFP-PD1), the activity EC₅₀ was -1.59E-009 nM for rhIL-21 and 6.3E-05 nM for 033; and the activation activity EC₅₀ values of the mutant fusion protein molecules 053, 054, 055, 061, and 063 in the study were 0.034 nM, 0.043 nM, 0.092 nM, 0.831 nM, and 0.8217 nM, respectively, which showed that the activity of the IL-21 mutants of the present invention on PD1-positive T cells was greatly improved compared to the activity of these IL-21 mutants on PD-1-negative cells, although it was weaker than that of rhIL-21. The results are shown in FIG. 3B.

As can be seen from the above results, the mutants of the present invention had very weak activity on PD1-negative HuT78 cells, but had very strong activity on PD1-positive HuT78-GFP-PD1 cells, and most of the mutants had activity EC₅₀ less than 0.1 nM, and at most less than 5.5 nM, which showed that the IL-21 mutants obtained in the study had a very large therapeutic window on both cells and could selectively activate PD1-positive T cells.

**Table 13: Stat3 activity of IL-21 mutants on HuT78 PD1- and PD1+ cells**

| ID | EC₅₀ (nM) (PD1-negative HuT78 cells) | EC₅₀ (nM) (PD1-Positive HuT78-GFP-PD1 cells) |
|---|---|---|
| rhIL-21 | ∼1.610e-009 | ∼ 1.593e-009 |
| 033 | 0.04584 | 0.00006302 |
| 034 | 0.1638 | 0.003616 |
| 035 | 1.034 | 0.01347 |
| 036 | 0.1384 | 0.001319 |
| 037 | 1.167 | 8.41E-05 |
| 038 | 12.91 | 0.08134 |
| 039 | 3.317 | 0.01637 |
| 040 | 32.04 | 0.1111 |
| 041 | ∼ 98091 | 2.492 |
| 042 | 0.07625 | 0.003994 |
| 047 | 0.07039 | 0.0021 |
| 048 | 3.763 | 0.04881 |
| 049 | ∼ 4376 | 1.528 |
| 053 | N/A | 0.03393 |
| 054 | N/A | 0.04313 |
| 055 | N/A | 0.09215 |
| 059 | N/A | 5.33 |
| 061 | N/A | 0.831 |
| 063 | N/A | 0.8217 |
| 064 | N/A | 3.042 |

| | | |
|---|---|---|
| N/A: the molecule has no activity or very weak activity at 100 nM and the EC₅₀ value cannot be demonstrated. | | |

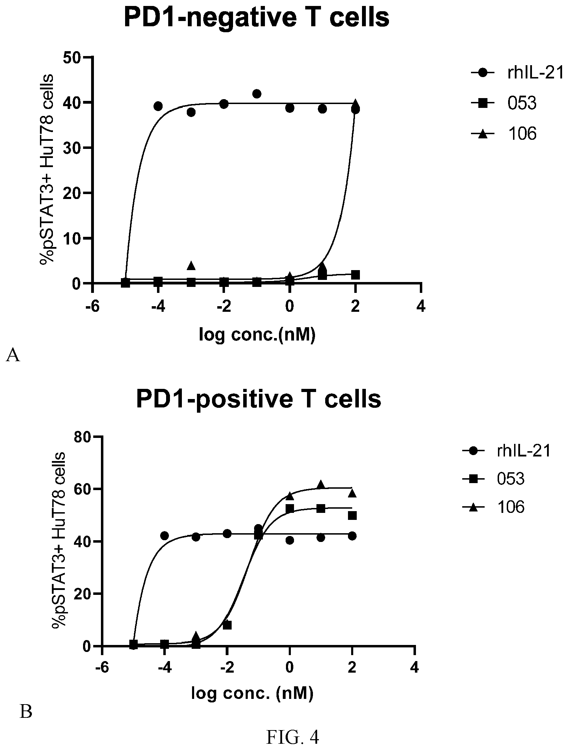

The inventors further compared the signaling pathway activating activity of the fusion protein molecule 053 of the present invention with that of a control molecule (encoded as 106 in the present application, wherein the engineering information on the IL-21 mutant protein is derived from an IL-21 mutant protein with the sequence No. 241 disclosed in the patent application US20190046611A1, which is the anti-PD1 antibody-fusion protein of the present invention, wherein the sequences of the anti-PD1 antibody are as follows: heavy chain-Knob: SEQ ID NO: 68, heavy chain-Hole: SEQ ID NO: 33, and light chain: SEQ ID NO: 034, prepared as described in Example 2). The results are shown in FIG. 4. The results showed that the fusion protein molecule 053 of the present invention had significantly weaker activity on PD1-negative HuT78 cells than that of the molecule 106 (FIG. 4A), but had comparable activity on PD1-positive HuT78-GFP-PD1 cells to that of the molecule 106 (FIG. 4B), which further showed that the IL-21 mutants obtained by the present invention had a larger activity window between PD-1-negative and -positive cells than that of the known mutants.

| | | |
|---|---|---|
| | | KTHTCPPCPAPEAAGGPSVFLFPPKPKDTLMISRTPEVTCVVVDVSHE DPEVKFNWYVDGVEVHNAKTKPREEQYNSTYRVVSVLTVLHQDWL NGKEYKCKVSNKALPAPIEKTISKAKGQPREPQVYTLPPSREEMTKN QVSLTCLVKGFYPSDIAVEWESNGQPENNYKTTPPVLDSDGSFFLYSK LTVDKSRWQQGNVFSCSVMHEALHNHYTQKSLSLSPGK |
| 6 | 006 | HKSDITLQEIIKLIDIVDQLKNYVNDLVPEFLPAPEDVETNCEWSAFSC FQKAQLKSANTGNNERIINVSIKKLKRKPPSTNAGRRQKHRLTCPSCD SYEKKPPKEFLERFKSLLQKMIHQHLSSRTHGSEDSGGGGSGGGGSD KTHTCPPCPAPEAAGGPSVFLFPPKPKDTLMISRTPEVTCVVVDVSHE DPEVKFNWYVDGVEVHNAKTKPREEQYNSTYRVVSVLTVLHQDWL NGKEYKCKVSNKALPAPIEKTISKAKGQPREPQVYTLPPSREEMTKN QVSLTCLVKGFYPSDIAVEWESNGQPENNYKTTPPVLDSDGSFFLYSK LTVDKSRWQQGNVFSCSVMHEALHNHYTQKSLSLSPGK |
| 7 | 007 | HKSDITLQEIIQLIDIVDQLKNYVNDLVPEFLPAPEDVETNCEWSAFSC FQKAQLKSANTGNNERIINVSIKKLKRKPPSTNAGRRQKHRLTCPSCD SYEKKPPKEFLERFKSLLQKMIHQHLSSRTHGSEDSGGGGSGGGGSD KTHTCPPCPAPEAAGGPSVFLFPPKPKDTLMISRTPEVTCVVVDVSHE DPEVKFNWYVDGVEVHNAKTKPREEQYNSTYRVVSVLTVLHQDWL NGKEYKCKVSNKALPAPIEKTISKAKGQPREPQVYTLPPSREEMTKN QVSLTCLVKGFYPSDIAVEWESNGQPENNYKTTPPVLDSDGSFFLYSK LTVDKSRWQQGNVFSCSVMHEALHNHYTQKSLSLSPGK |
| 8 | 008 | HKSDITLQEIIMRQLIDIVDQLKNYVNDLVPEFLPAPEDVETNCEWSAF SCFQKAQLKSANTGNNERIINVSIKKLKRKPPSTNAGRRQKHRLTCPS CDSYEKKPPKEFLERFKSLLQKMIHQHLSSRTHGSEDSGGGGSGGGG SDKTHTCPPCPAPEAAGGPSVFLFPPKPKDTLMISRTPEVTCVVVDVS HEDPEVKFNWYVDGVEVHNAKTKPREEQYNSTYRVVSVLTVLHQD WLNGKEYKCKVSNKALPAPIEKTISKAKGQPREPQVYTLPPSREEMT KNQVSLTCLVKGFYPSDIAVEWESNGQPENNYKTTPPVLDSDGSFFLY SKLTVDKSRWQQGNVFSCSVMHEALHNHYTQKSLSLSPGK |
| 9 | 009 | QGQNRTMIRMRQLIDIVDQLKNYVNDLVPEFLPAPEDVETNCEWSAF SCFQKAQLKSANTGNNERIINVSIKKLKRKPPSTNAGRRQKHRLTCPS CDSYEKKPPKEFLERFKSLLQKMIHQHLSSRTHGSEDSGGGGSGGGG SDKTHTCPPCPAPEAAGGPSVFLFPPKPKDTLMISRTPEVTCVVVDVS HEDPEVKFNWYVDGVEVHNAKTKPREEQYNSTYRVVSVLTVLHQD WLNGKEYKCKVSNKALPAPIEKTISKAKGQPREPQVYTLPPSREEMT KNQVSLTCLVKGFYPSDIAVEWESNGQPENNYKTTPPVLDSDGSFFLY SKLTVDKSRWQQGNVFSCSVMHEALHNHYTQKSLSLSPGK |
| 10 | 010 | QGQDNHTIRMRQLIDIVDQLKNYVNDLVPEFLPAPEDVETNCEWSAF |
| | | SCFQKAQLKSANTGNNERIINVSIKKLKRKPPSTNAGRRQKHRLTCPS CDSYEKKPPKEFLERFKSLLQKMIHQHLSSRTHGSEDSGGGGSGGGG SDKTHTCPPCPAPEAAGGPSVFLFPPKPKDTLMISRTPEVTCVVVDVS HEDPEVKFNWYVDGVEVHNAKTKPREEQYNSTYRVVSVLTVLHQD WLNGKEYKCKVSNKALPAPIEKTISKAKGQPREPQVYTLPPSREEMT KNQVSLTCLVKGFYPSDIAVEWESNGQPENNYKTTPPVLDSDGSFFLY SKLTVDKSRWQQGNVFSCSVMHEALHNHYTQKSLSLSPGK |
| 11 | 011 | QGQDRHMIRMRQLINITDQLKNYVNDLVPEFLPAPEDVETNCEWSAF SCFQKAQLKSANTGNNERIINVSIKKLKRKPPSTNAGRRQKHRLTCPS CDSYEKKPPKEFLERFKSLLQKMIHQHLSSRTHGSEDSGGGGSGGGG SDKTHTCPPCPAPEAAGGPSVFLFPPKPKDTLMISRTPEVTCVVVDVS HEDPEVKFNWYVDGVEVHNAKTKPREEQYNSTYRVVSVLTVLHQD WLNGKEYKCKVSNKALPAPIEKTISKAKGQPREPQVYTLPPSREEMT KNQVSLTCLVKGFYPSDIAVEWESNGQPENNYKTTPPVLDSDGSFFLY SKLTVDKSRWQQGNVFSCSVMHEALHNHYTQKSLSLSPGK |
| 12 | 012 | QGQDRHMIRMRQLIDIVDNLTNYVNDLVPEFLPAPEDVETNCEWSAF SCFQKAQLKSANTGNNERIINVSIKKLKRKPPSTNAGRRQKHRLTCPS CDSYEKKPPKEFLERFKSLLQKMIHQHLSSRTHGSEDSGGGGSGGGG SDKTHTCPPCPAPEAAGGPSVFLFPPKPKDTLMISRTPEVTCVVVDVS HEDPEVKFNWYVDGVEVHNAKTKPREEQYNSTYRVVSVLTVLHQD WLNGKEYKCKVSNKALPAPIEKTISKAKGQPREPQVYTLPPSREEMT KNQVSLTCLVKGFYPSDIAVEWESNGQPENNYKTTPPVLDSDGSFFLY SKLTVDKSRWQQGNVFSCSVMHEALHNHYTQKSLSLSPGK |
| 13 | 013 | QGQDRHMIRMRQLIDIVDQLKNYVNDLVPEFLPAPEDVETNCEWSAF SCFQKAQLKSANTGNNERIINVSIKKLKNKTPSTNAGRRQKHRLTCPS CDSYEKKPPKEFLERFKSLLQKMIHQHLSSRTHGSEDSGGGGSGGGG SDKTHTCPPCPAPEAAGGPSVFLFPPKPKDTLMISRTPEVTCVVVDVS HEDPEVKFNWYVDGVEVHNAKTKPREEQYNSTYRVVSVLTVLHQD WLNGKEYKCKVSNKALPAPIEKTISKAKGQPREPQVYTLPPSREEMT KNQVSLTCLVKGFYPSDIAVEWESNGQPENNYKTTPPVLDSDGSFFLY SKLTVDKSRWQQGNVFSCSVMHEALHNHYTQKSLSLSPGK |
| 14 | 014 | QGQDRHMIRMRQLIDIVDQLKNYVNDLVPEFLPAPEDVETNCEWSAF SCFQKAQLKSANTGNNERIINVSIKKLKRNSTSTNAGRRQKHRLTCPS CDSYEKKPPKEFLERFKSLLQKMIHQHLSSRTHGSEDSGGGGSGGGG SDKTHTCPPCPAPEAAGGPSVFLFPPKPKDTLMISRTPEVTCVVVDVS HEDPEVKFNWYVDGVEVHNAKTKPREEQYNSTYRVVSVLTVLHQD WLNGKEYKCKVSNKALPAPIEKTISKAKGQPREPQVYTLPPSREEMT KNQVSLTCLVKGFYPSDIAVEWESNGQPENNYKTTPPVLDSDGSFFLY |
| | | SKLTVDKSRWQQGNVFSCSVMHEALHNHYTQKSLSLSPGK |
| 15 | 015 | QGQDRHMIRMRQLIDIVDQLKNYVNDLVPEFLPAPEDVETNCEWSAF SCFQKAQLKSANTGNNERIINVSIKKLKRKPNSTNAGRRQKHRLTCPS CDSYEKKPPKEFLERFKSLLQKMIHQHLSSRTHGSEDSGGGGSGGGG SDKTHTCPPCPAPEAAGGPSVFLFPPKPKDTLMISRTPEVTCVVVDVS HEDPEVKFNWYVDGVEVHNAKTKPREEQYNSTYRVVSVLTVLHQD WLNGKEYKCKVSNKALPAPIEKTISKAKGQPREPQVYTLPPSREEMT KNQVSLTCLVKGFYPSDIAVEWESNGQPENNYKTTPPVLDSDGSFFLY SKLTVDKSRWQQGNVFSCSVMHEALHNHYTQKSLSLSPGK |
| 16 | 016 | QGQDRHMIRMRQLIDIVDQLKNYVNDLVPEFLPAPEDVETNCEWSAF SCFQKAQLKSANTGNNERIINVSIKKLKRKPPNTTAGRRQKHRLTCPS CDSYEKKPPKEFLERFKSLLQKMIHQHLSSRTHGSEDSGGGGSGGGG SDKTHTCPPCPAPEAAGGPSVFLFPPKPKDTLMISRTPEVTCVVVDVS HEDPEVKFNWYVDGVEVHNAKTKPREEQYNSTYRVVSVLTVLHQD WLNGKEYKCKVSNKALPAPIEKTISKAKGQPREPQVYTLPPSREEMT KNQVSLTCLVKGFYPSDIAVEWESNGQPENNYKTTPPVLDSDGSFFLY SKLTVDKSRWQQGNVFSCSVMHEALHNHYTQKSLSLSPGK |
| 17 | 017 | QGQDRHMIRMRQLIDIVDQLKNYVNDLVPEFLPAPEDVETNCEWSAF SCFQKAQLKSANTGNNERIINVSIKKLKRKPPSTNATRRQKHRLTCPS CDSYEKKPPKEFLERFKSLLQKMIHQHLSSRTHGSEDSGGGGSGGGG SDKTHTCPPCPAPEAAGGPSVFLFPPKPKDTLMISRTPEVTCVVVDVS HEDPEVKFNWYVDGVEVHNAKTKPREEQYNSTYRVVSVLTVLHQD WLNGKEYKCKVSNKALPAPIEKTISKAKGQPREPQVYTLPPSREEMT KNQVSLTCLVKGFYPSDIAVEWESNGQPENNYKTTPPVLDSDGSFFLY SKLTVDKSRWQQGNVFSCSVMHEALHNHYTQKSLSLSPGK |
| 18 | 018 | QGQDRHMIRMRQLIDIVDQLKNYVNDLVPEFLPAPEDVETNCEWSAF SCFQKAQLKSANTGNNERIINVSIKKLKRKPPSTNAGRRQKHRLTCPS CDSYEKKPPKEFLERFKSLLQKMINQTLSSRTHGSEDSGGGGSGGGG SDKTHTCPPCPAPEAAGGPSVFLFPPKPKDTLMISRTPEVTCVVVDVS HEDPEVKFNWYVDGVEVHNAKTKPREEQYNSTYRVVSVLTVLHQD WLNGKEYKCKVSNKALPAPIEKTISKAKGQPREPQVYTLPPSREEMT KNQVSLTCLVKGFYPSDIAVEWESNGQPENNYKTTPPVLDSDGSFFLY SKLTVDKSRWQQGNVFSCSVMHEALHNHYTQKSLSLSPGK |
| 19 | 019 | QGQDRHMIRMRQLIDIVDQLKNYVNDLVPEFLPAPENVTTNCEWSAF SCFQKAQLKSANTGNNERIINVSIKKLKRKPPSTNAGRRQKHRLTCPS CDSYEKKPPKEFLERFKSLLQKMIHQHLSSRTHGSEDSGGGGSGGGG SDKTHTCPPCPAPEAAGGPSVFLFPPKPKDTLMISRTPEVTCVVVDVS HEDPEVKFNWYVDGVEVHNAKTKPREEQYNSTYRVVSVLTVLHQD |
| | | WLNGKEYKCKVSNKALPAPIEKTISKAKGQPREPQVYTLPPSREEMT KNQVSLTCLVKGFYPSDIAVEWESNGQPENNYKTTPPVLDSDGSFFLY SKLTVDKSRWQQGNVFSCSVMHEALHNHYTQKSLSLSPGK |
| 20 | 020 | QGQDRHMIRMRQLIDIVDQLKNYVNDLVPEFLPAPEDVETNCEWSAF SCFQKAQLKSANTGNNERIINVSIEKLKRKPPSTNAGRRQKHRLTCPS CDSYEKKPPKEFLERFKSLLQKMIHQHLSSRTHGSEDSGGGGSGGGG SDKTHTCPPCPAPEAAGGPSVFLFPPKPKDTLMISRTPEVTCVVVDVS HEDPEVKFNWYVDGVEVHNAKTKPREEQYNSTYRVVSVLTVLHQD WLNGKEYKCKVSNKALPAPIEKTISKAKGQPREPQVYTLPPSREEMT KNQVSLTCLVKGFYPSDIAVEWESNGQPENNYKTTPPVLDSDGSFFLY SKLTVDKSRWQQGNVFSCSVMHEALHNHYTQKSLSLSPGK |
| 21 | 021 | QGQDRHMQRMRQLIDIVDQLKNYVNDLVPEFLPAPEDVETNCEWSA FSCFQKAQLKSANTGNNERIINVSIKKLKRKPESTNAGRRQKHRLTCP SCDSYEKKPPKEFLERFKSLLQKMIHQHLSSRTHGSEDSGGGGSGGG GSDKTHTCPPCPAPEAAGGPSVFLFPPKPKDTLMISRTPEVTCVVVDV SHEDPEVKFNWYVDGVEVHNAKTKPREEQYNSTYRVVSVLTVLHQD WLNGKEYKCKVSNKALPAPIEKTISKAKGQPREPQVYTLPPSREEMT KNQVSLTCLVKGFYPSDIAVEWESNGQPENNYKTTPPVLDSDGSFFLY SKLTVDKSRWQQGNVFSCSVMHEALHNHYTQKSLSLSPGK |
| 22 | 022 | QGQDRHMIRMRQLIDIVDQLKNYVNDLVPEFLPAPEDVETNCEWSAF SCFQKAQLKSANTGNNERIINVSIKKLKRDAPSTNAGRRQKHRLTCPS CDSYEKKPPKEFLERFKSLLQKMIHQHLSSRTHGSEDSGGGGSGGGG SDKTHTCPPCPAPEAAGGPSVFLFPPKPKDTLMISRTPEVTCVVVDVS HEDPEVKFNWYVDGVEVHNAKTKPREEQYNSTYRVVSVLTVLHQD WLNGKEYKCKVSNKALPAPIEKTISKAKGQPREPQVYTLPPSREEMT KNQVSLTCLVKGFYPSDIAVEWESNGQPENNYKTTPPVLDSDGSFFLY SKLTVDKSRWQQGNVFSCSVMHEALHNHYTQKSLSLSPGK |
| 23 | 023 | QGQDRHMIRMRQLIDIVDQLKNYVNDLVPEFLPAPEDVETNCEWSAF SCFQKAQLKSANTGNNERIINVSIKKLKRKPPSTNAGKHRLTCPSCDS YEKKPPKEFLERFKSLLQKMIHQHLSSRTHGSEDSGGGGSGGGGSDK THTCPPCPAPEAAGGPSVFLFPPKPKDTLMISRTPEVTCVVVDVSHED PEVKFNWYVDGVEVHNAKTKPREEQYNSTYRVVSVLTVLHQDWLN GKEYKCKVSNKALPAPIEKTISKAKGQPREPQVYTLPPSREEMTKNQ VSLTCLVKGFYPSDIAVEWESNGQPENNYKTTPPVLDSDGSFFLYSKL TVDKSRWQQGNVFSCSVMHEALHNHYTQKSLSLSPGK |
| 24 | 024 | QGQDRHMIRMRQLIDIVDQLKNYVNDLVPEFLPAPEDVETNCEWSAF SCFQKAQLKSANTGNNERIINVSIKKLKRKSTNAGRRQKHRLTCPSCD SYEKKPPKEFLERFKSLLQKMIHQHLSSRTHGSEDSGGGGSGGGGSD |
| | | KTHTCPPCPAPEAAGGPSVFLFPPKPKDTLMISRTPEVTCVVVDVSHE DPEVKFNWYVDGVEVHNAKTKPREEQYNSTYRVVSVLTVLHQDWL NGKEYKCKVSNKALPAPIEKTISKAKGQPREPQVYTLPPSREEMTKN QVSLTCLVKGFYPSDIAVEWESNGQPENNYKTTPPVLDSDGSFFLYSK LTVDKSRWQQGNVFSCSVMHEALHNHYTQKSLSLSPGK |
| 25 | 025 | MRQLIDIVDQLKNYVNDLVPEFLPAPEDVETNCEWSAFSCFQKAQLK SANTGNNERIINVSIKKLKRKPPSTNAGRRQKHRLTCPSCDSYEKKPP KEFLERFKSLLQKMIHQHLSSRTHGSEDSGGGGSGGGGSDKTHTCPP CPAPEAAGGPSVFLFPPKPKDTLMISRTPEVTCVVVDVSHEDPEVKFN WYVDGVEVHNAKTKPREEQYNSTYRVVSVLTVLHQDWLNGKEYKC KVSNKALPAPIEKTISKAKGQPREPQVYTLPPSREEMTKNQVSLTCLV KGFYPSDIAVEWESNGQPENNYKTTPPVLDSDGSFFLYSKLTVDKSR WQQGNVFSCSVMHEALHNHYTQKSLSLSPGK |
| 26 | 026 | MRQLIDIVDQLKNYVNDLVPEFLPAPEDVETNCEWSAFSCFQKAQLK SANTGNNERIINVSIKKLKRKSTNAGRRQKHRLTCPSCDSYEKKPPKE FLERFKSLLQKMIHQHLSSRTHGSEDSGGGGSGGGGSDKTHTCPPCP APEAAGGPSVFLFPPKPKDTLMISRTPEVTCVVVDVSHEDPEVKFNW YVDGVEVHNAKTKPREEQYNSTYRVVSVLTVLHQDWLNGKEYKCK VSNKALPAPIEKTISKAKGQPREPQVYTLPPSREEMTKNQVSLTCLVK GFYPSDIAVEWESNGQPENNYKTTPPVLDSDGSFFLYSKLTVDKSRW QQGNVFSCSVMHEALHNHYTQKSLSLSPGK |
| 27 | 027 | MRQLIDIVDNLTNYVNDLVPEFLPAPEDVETNCEWSAFSCFQKAQLK SANTGNNERIINVSIKKLKRKPPSTNAGRRQKHRLTCPSCDSYEKKPP KEFLERFKSLLQKMIHQHLSSRTHGSEDSGGGGSGGGGSDKTHTCPP CPAPEAAGGPSVFLFPPKPKDTLMISRTPEVTCVVVDVSHEDPEVKFN WYVDGVEVHNAKTKPREEQYNSTYRVVSVLTVLHQDWLNGKEYKC KVSNKALPAPIEKTISKAKGQPREPQVYTLPPSREEMTKNQVSLTCLV KGFYPSDIAVEWESNGQPENNYKTTPPVLDSDGSFFLYSKLTVDKSR WQQGNVFSCSVMHEALHNHYTQKSLSLSPGK |
| 28 | 028 | QGQDRHMIRMRQLIDIVDNLTNYVNDLVPEFLPAPEDVETNAEWSAF SCFQKAQLKSANTGNNERIINVSIKKLKRKPPSTNATTPSCDSYEKKPP KEFLERFKSLLQKMIHQHLSSRTHGSEDSGGGGSGGGGSDKTHTCPP CPAPEAAGGPSVFLFPPKPKDTLMISRTPEVTCVVVDVSHEDPEVKFN WYVDGVEVHNAKTKPREEQYNSTYRVVSVLTVLHQDWLNGKEYKC KVSNKALPAPIEKTISKAKGQPREPQVYTLPPSREEMTKNQVSLTCLV KGFYPSDIAVEWESNGQPENNYKTTPPVLDSDGSFFLYSKLTVDKSR WQQGNVFSCSVMHEALHNHYTQKSLSLSPGK |
| 29 | 029 | HKSDITLQEMRQLIDIVDQLKNYVNDLVPEFLPAPEDVETNCEWSAFS |
| | | CFQKAQLKSANTGNNERIINVSIKKLKRKSTNAGRRQKHRLTCPSCDS YEKKPPKEFLERFKSLLQKMIHQHLSSRTHGSEDSGGGGSGGGGSDK THTCPPCPAPEAAGGPSVFLFPPKPKDTLMISRTPEVTCVVVDVSHED PEVKFNWYVDGVEVHNAKTKPREEQYNSTYRVVSVLTVLHQDWLN GKEYKCKVSNKALPAPIEKTISKAKGQPREPQVYTLPPSREEMTKNQ VSLTCLVKGFYPSDIAVEWESNGQPENNYKTTPPVLDSDGSFFLYSKL TVDKSRWQQGNVFSCSVMHEALHNHYTQKSLSLSPGK |
| 30 | 030 | HKSDITLQEMRQLIDIVDQLKNYVNDLVPEFLPAPEDVETNCEWSAFS CFQKAQLKSANTGNNERIINVSIKKLKRKPPSTNAGRRQKHRLTCPSC DSYEKKPPKEFLERFKSLLQNMTHQHLSSRTHGSEDSGGGGSGGGGS DKTHTCPPCPAPEAAGGPSVFLFPPKPKDTLMISRTPEVTCVVVDVSH EDPEVKFNWYVDGVEVHNAKTKPREEQYNSTYRVVSVLTVLHQDW LNGKEYKCKVSNKALPAPIEKTISKAKGQPREPQVYTLPPSREEMTK NQVSLTCLVKGFYPSDIAVEWESNGQPENNYKTTPPVLDSDGSFFLYS KLTVDKSRWQQGNVFSCSVMHEALHNHYTQKSLSLSPGK |
| 31 | 031 | HKSDITLQEMRQLIDIVDQLKNYVNDLVPEFLPAPENVTTNCEWSAFS CFQKAQLKSANTGNNERIINVSIKKLKRKPPSTNAGRRQKHRLTCPSC DSYEKKPPKEFLERFKSLLQKMIHQHLSSRTHGSEDSGGGGSGGGGS DKTHTCPPCPAPEAAGGPSVFLFPPKPKDTLMISRTPEVTCVVVDVSH EDPEVKFNWYVDGVEVHNAKTKPREEQYNSTYRVVSVLTVLHQDW LNGKEYKCKVSNKALPAPIEKTISKAKGQPREPQVYTLPPSREEMTK NQVSLTCLVKGFYPSDIAVEWESNGQPENNYKTTPPVLDSDGSFFLYS KLTVDKSRWQQGNVFSCSVMHEALHNHYTQKSLSLSPGK |
| 32 | 032 | HKSDITLQEMRQLINITDQLKNYVNDLVPEFLPAPEDVETNCEWSAFS CFQKAQLKSANTGNNERIINVSIKKLKRKPPSTNAGRRQKHRLTCPSC DSYEKKPPKEFLERFKSLLQKMIHQHLSSRTHGSEDSGGGGSGGGGS DKTHTCPPCPAPEAAGGPSVFLFPPKPKDTLMISRTPEVTCVVVDVSH EDPEVKFNWYVDGVEVHNAKTKPREEQYNSTYRVVSVLTVLHQDW LNGKEYKCKVSNKALPAPIEKTISKAKGQPREPQVYTLPPSREEMTK NQVSLTCLVKGFYPSDIAVEWESNGQPENNYKTTPPVLDSDGSFFLYS KLTVDKSRWQQGNVFSCSVMHEALHNHYTQKSLSLSPGK |
| 33 | Heavy chain of anti-PD-1 antibody comprising a hole mutation, in the IgG1 form, with an LALA mutation (chain B2 in fusion protein) | QVQLVQSGAEVKKPGSSVKVSCKASGGTFSSYAISWVRQAPGQGLE WMGLIIPMFDTAGYAQKFQGRVAITVDESTSTAYMELSSLRSEDTAVY YCARAEHSSTGTFDYWGQGTLVTVSSASTKGPSVFPLAPSSKSTSGGT AALGCLVKDYFPEPVTVSWNSGALTSGVHTFPAVLQSSGLYSLSSVVT VPSSSLGTQTYICNVNHKPSNTKVDKKVEPKSCDKTHTCPPCPAPEA AGGPSVFLFPPKPKDTLMISRTPEVTCVVVDVSHEDPEVKFNWYVDG VEVHNAKTKPREEQYNSTYRVVSVLTVLHQDWLNGKEYKCKVSNK |
| | | ALPAPIEKTISKAKGQPREPQVCTLPPSRDELTKNQVSLSCAVKGFYPS DIAVEWESNGQPENNYKTTPPVLDSDGSFFLVSKLTVDKSRWQQGNV FSCSVMHEALHNHYTQKSLSLSPGK |
| 34 | Light chain of anti-PD-1 antibody (chain A in fusion protein) | DIQMTQSPSSVSASVGDRVTITCRASQGISSWLAWYQQKPGKAPKLLI SAASSLQSGVPSRFSGSGSGTDFTLTISSLQPEDFATYYCQQANHLPFT FGGGTKVEIKRTVAAPSVFIFPPSDEQLKSGTASVVCLLNNFYPREAK VQWKVDNALQSGNSQESVTEQDSKDSTYSLSSTLTLSKADYEKHKV YACEVTHQGLSSPVTKSFNRGEC |
| 35 | Chain B in fusion protein | QVQLVQSGAEVKKPGSSVKVSCKASGGTFSSYAISWVRQAPGQGLE WMGLIIPMFDTAGYAQKFQGRVAITVDESTSTAYMELSSLRSEDTAVY YCARAEHSSTGTFDYWGQGTLVTVSSASTKGPSVFPLAPSSKSTSGGT AALGCLVKDYFPEPVTVSWNSGALTSGVHTFPAVLQSSGLYSLSSVVT VPSSSLGTQTYICNVNHKPSNTKVDKKVEPKSCDKTHTCPPCPAPEA AGGPSVFLFPPKPKDTLMISRTPEVTCVVVDVSHEDPEVKFNWYVDG VEVHNAKTKPREEQYNSTYRVVSVLTVLHQDWLNGKEYKCKVSNK ALPAPIEKTISKAKGQPREPQVYTLPPSREEMTKNQVSLTCLVKGFYPS DIAVEWESNGQPENNYKTTPPVLDSDGSFFLYSKLTVDKSRWQQGNV FSCSVMHEALHNHYTQKSLSLSPGQGQDRHMIRMRQLIDIVDQLKN YVNDLVPEFLPAPEDVETNCEWSAFSCFQKAQLKSANTGNNERIINVS IKKLKRKPPSTNAGRRQKHRLTCPSCDSYEKKPPKEFLERFKSLLQKM IHQHLSSRTHGSEDS |
| 36 | Chain B in fusion protein | QVQLVQSGAEVKKPGSSVKVSCKASGGTFSSYAISWVRQAPGQGLE WMGLIIPMFDTAGYAQKFQGRVAITVDESTSTAYMELSSLRSEDTAVY YCARAEHSSTGTFDYWGQGTLVTVSS ASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDK KVEPKSCDKTHTCPPCPAPEAAGGPSVFLFPPKPKDTLMISRTPEVTC VVVDVSHEDPEVKFNWYVDGVEVHNAKTKPREEQYNSTYRVVSVL TVLHQDWLNGKEYKCKVSNKALPAPIEKTISKAKGQPREPQVYTLPP SREEMTKNQVSLTCLVKGFYPSDIAVEWESNGQPENNYKTTPPVLDS DGSFFLYSKLTVDKSRWQQGNVFSCSVMHEALHNHYTQKSLSLSPG HKSDIHMIRMRQLIDIVDQLKNYVNDLVPEFLPAPEDVETNCEWSAFS CFQKAQLKSANTGNNERIINVSIKKLKRKPPSTNAGRRQKHRLTCPSC DSYEKKPPKEFLERFKSLLQKMIHQHLSSRTHGSEDS |
| 37 | Chain B in fusion protein | QVQLVQSGAEVKKPGSSVKVSCKASGGTFSSYAISWVRQAPGQGLE WMGLIIPMFDTAGYAQKFQGRVAITVDESTSTAYMELSSLRSEDTAVY YCARAEHSSTGTFDYWGQGTLVTVSS ASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDK KVEPKSCDKTHTCPPCPAPEAAGGPSVFLFPPKPKDTLMISRTPEVTC VVVDVSHEDPEVKFNWYVDGVEVHNAKTKPREEQYNSTYRVVSVL TVLHQDWLNGKEYKCKVSNKALPAPIEKTISKAKGQPREPQVYTLPP SREEMTKNQVSLTCLVKGFYPSDIAVEWESNGQPENNYKTTPPVLDS DGSFFLYSKLTVDKSRWQQGNVFSCSVMHEALHNHYTQKSLSLSPG HKCDIHMIRMRQLIDIVDQLKNYVNDLVPEFLPAPEDVETNCEWSAF SCFQKAQLKSANTGNNERIINVSIKKLKRKPPSTNAGRRQKHRLTCPS CDSYEKKPPKEFLERFKSLLQKMIHQHCSSRTHGSEDS |
| 38 | Chain B in fusion protein | QVQLVQSGAEVKKPGSSVKVSCKASGGTFSSYAISWVRQAPGQGLE WMGLIIPMFDTAGYAQKFQGRVAITVDESTSTAYMELSSLRSEDTAVY YCARAEHSSTGTFDYWGQGTLVTVSS ASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDK KVEPKSCDKTHTCPPCPAPEAAGGPSVFLFPPKPKDTLMISRTPEVTC VVVDVSHEDPEVKFNWYVDGVEVHNAKTKPREEQYNSTYRVVSVL TVLHQDWLNGKEYKCKVSNKALPAPIEKTISKAKGQPREPQVYTLPP SREEMTKNQVSLTCLVKGFYPSDIAVEWESNGQPENNYKTTPPVLDS DGSFFLYSKLTVDKSRWQQGNVFSCSVMHEALHNHYTQKSLSLSPG QGQNRTMIRMRQLIDIVDQLKNYVNDLVPEFLPAPEDVETNCEWSAF SCFQKAQLKSANTGNNERIINVSIKKLKRKPPSTNAGRRQKHRLTCPS CDSYEKKPPKEFLERFKSLLQKMIHQHLSSRTHGSEDS |
| 39 | Chain B in fusion protein | QVQLVQSGAEVKKPGSSVKVSCKASGGTFSSYAISWVRQAPGQGLE WMGLIIPMFDTAGYAQKFQGRVAITVDESTSTAYMELSSLRSEDTAVY YCARAEHSSTGTFDYWGQGTLVTVSS ASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDK KVEPKSCDKTHTCPPCPAPEAAGGPSVFLFPPKPKDTLMISRTPEVTC VVVDVSHEDPEVKFNWYVDGVEVHNAKTKPREEQYNSTYRVVSVL TVLHQDWLNGKEYKCKVSNKALPAPIEKTISKAKGQPREPQVYTLPP SREEMTKNQVSLTCLVKGFYPSDIAVEWESNGQPENNYKTTPPVLDS DGSFFLYSKLTVDKSRWQQGNVFSCSVMHEALHNHYTQKSLSLSPG QGQDNHTIRMRQLIDIVDQLKNYVNDLVPEFLPAPEDVETNCEWSAF SCFQKAQLKSANTGNNERIINVSIKKLKRKPPSTNAGRRQKHRLTCPS |
| | | CDSYEKKPPKEFLERFKSLLQKMIHQHLSSRTHGSEDS |
| 40 | Chain B in fusion protein | QVQLVQSGAEVKKPGSSVKVSCKASGGTFSSYAISWVRQAPGQGLE WMGLIIPMFDTAGYAQKFQGRVAITVDESTSTAYMELSSLRSEDTAVY YCARAEHSSTGTFDYWGQGTLVTVSS ASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDK KVEPKSCDKTHTCPPCPAPEAAGGPSVFLFPPKPKDTLMISRTPEVTC VVVDVSHEDPEVKFNWYVDGVEVHNAKTKPREEQYNSTYRVVSVL TVLHQDWLNGKEYKCKVSNKALPAPIEKTISKAKGQPREPQVYTLPP SREEMTKNQVSLTCLVKGFYPSDIAVEWESNGQPENNYKTTPPVLDS DGSFFLYSKLTVDKSRWQQGNVFSCSVMHEALHNHYTQKSLSLSPG QGQDRHMIRMRQLINITDQLKNYVNDLVPEFLPAPEDVETNCEWSAF SCFQKAQLKSANTGNNERIINVSIKKLKRKPPSTNAGRRQKHRLTCPS CDSYEKKPPKEFLERFKSLLQKMIHQHLSSRTHGSEDS |
| 41 | Chain B in fusion protein | QVQLVQSGAEVKKPGSSVKVSCKASGGTFSSYAISWVRQAPGQGLE WMGLIIPMFDTAGYAQKFQGRVAITVDESTSTAYMELSSLRSEDTAVY YCARAEHSSTGTFDYWGQGTLVTVSS ASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDK KVEPKSCDKTHTCPPCPAPEAAGGPSVFLFPPKPKDTLMISRTPEVTC VVVDVSHEDPEVKFNWYVDGVEVHNAKTKPREEQYNSTYRVVSVL TVLHQDWLNGKEYKCKVSNKALPAPIEKTISKAKGQPREPQVYTLPP SREEMTKNQVSLTCLVKGFYPSDIAVEWESNGQPENNYKTTPPVLDS DGSFFLYSKLTVDKSRWQQGNVFSCSVMHEALHNHYTQKSLSLSPG QGQDRHMIRMRQLIDIVDNLTNYVNDLVPEFLPAPEDVETNCEWSAF SCFQKAQLKSANTGNNERIINVSIKKLKRKPPSTNAGRRQKHRLTCPS CDSYEKKPPKEFLERFKSLLQKMIHQHLSSRTHGSEDS |
| 42 | Chain B in fusion protein | QVQLVQSGAEVKKPGSSVKVSCKASGGTFSSYAISWVRQAPGQGLE WMGLIIPMFDTAGYAQKFQGRVAITVDESTSTAYMELSSLRSEDTAVY YCARAEHSSTGTFDYWGQGTLVTVSS ASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDK KVEPKSCDKTHTCPPCPAPEAAGGPSVFLFPPKPKDTLMISRTPEVTC VVVDVSHEDPEVKFNWYVDGVEVHNAKTKPREEQYNSTYRVVSVL TVLHQDWLNGKEYKCKVSNKALPAPIEKTISKAKGQPREPQVYTLPP SREEMTKNQVSLTCLVKGFYPSDIAVEWESNGQPENNYKTTPPVLDS DGSFFLYSKLTVDKSRWQQGNVFSCSVMHEALHNHYTQKSLSLSPG QGQDRHMIRMRQLIDIVDQLKNYVNDLVPEFLPAPEDVETNCEWSAF |
| | | SCFQKAQLKSANTGNNERIINVSIKKLKNKTPSTNAGRRQKHRLTCPS CDSYEKKPPKEFLERFKSLLQKMIHQHLSSRTHGSEDS |
| 43 | Chain B in fusion protein | QVQLVQSGAEVKKPGSSVKVSCKASGGTFSSYAISWVRQAPGQGLE WMGLIIPMFDTAGYAQKFQGRVAITVDESTSTAYMELSSLRSEDTAVY YCARAEHSSTGTFDYWGQGTLVTVSS ASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDK KVEPKSCDKTHTCPPCPAPEAAGGPSVFLFPPKPKDTLMISRTPEVTC VVVDVSHEDPEVKFNWYVDGVEVHNAKTKPREEQYNSTYRVVSVL TVLHQDWLNGKEYKCKVSNKALPAPIEKTISKAKGQPREPQVYTLPP SREEMTKNQVSLTCLVKGFYPSDIAVEWESNGQPENNYKTTPPVLDS DGSFFLYSKLTVDKSRWQQGNVFSCSVMHEALHNHYTQKSLSLSPG QGQDRHMIRMRQLIDIVDQLKNYVNDLVPEFLPAPEDVETNCEWSAF SCFQKAQLKSANTGNNERIINVSIKKLKRNSTSTNAGRRQKHRLTCPS CDSYEKKPPKEFLERFKSLLQKMIHQHLSSRTHGSEDS |
| 44 | Chain B in fusion protein | QVQLVQSGAEVKKPGSSVKVSCKASGGTFSSYAISWVRQAPGQGLE WMGLIIPMFDTAGYAQKFQGRVAITVDESTSTAYMELSSLRSEDTAVY YCARAEHSSTGTFDYWGQGTLVTVSS ASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDK KVEPKSCDKTHTCPPCPAPEAAGGPSVFLFPPKPKDTLMISRTPEVTC VVVDVSHEDPEVKFNWYVDGVEVHNAKTKPREEQYNSTYRVVSVL TVLHQDWLNGKEYKCKVSNKALPAPIEKTISKAKGQPREPQVYTLPP SREEMTKNQVSLTCLVKGFYPSDIAVEWESNGQPENNYKTTPPVLDS DGSFFLYSKLTVDKSRWQQGNVFSCSVMHEALHNHYTQKSLSLSPG QGQDRHMIRMRQLIDIVDQLKNYVNDLVPEFLPAPEDVETNCEWSAF SCFQKAQLKSANTGNNERIINVSIKKLKRKPNSTNAGRRQKHRLTCPS CDSYEKKPPKEFLERFKSLLQKMIHQHLSSRTHGSEDS |
| 45 | Chain B in fusion protein | QVQLVQSGAEVKKPGSSVKVSCKASGGTFSSYAISWVRQAPGQGLE WMGLIIPMFDTAGYAQKFQGRVAITVDESTSTAYMELSSLRSEDTAVY YCARAEHSSTGTFDYWGQGTLVTVSS ASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDK KVEPKSCDKTHTCPPCPAPEAAGGPSVFLFPPKPKDTLMISRTPEVTC VVVDVSHEDPEVKFNWYVDGVEVHNAKTKPREEQYNSTYRVVSVL TVLHQDWLNGKEYKCKVSNKALPAPIEKTISKAKGQPREPQVYTLPP SREEMTKNQVSLTCLVKGFYPSDIAVEWESNGQPENNYKTTPPVLDS DGSFFLYSKLTVDKSRWQQGNVFSCSVMHEALHNHYTQKSLSLSPG |
| | | QGQDRHMIRMRQLIDIVDQLKNYVNDLVPEFLPAPEDVETNCEWSAF SCFQKAQLKSANTGNNERIINVSIKKLKRKPPNTTAGRRQKHRLTCPS CDSYEKKPPKEFLERFKSLLQKMIHQHLSSRTHGSEDS |
| 46 | Chain B in fusion protein | QVQLVQSGAEVKKPGSSVKVSCKASGGTFSSYAISWVRQAPGQGLE WMGLIIPMFDTAGYAQKFQGRVAITVDESTSTAYMELSSLRSEDTAVY YCARAEHSSTGTFDYWGQGTLVTVSS ASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDK KVEPKSCDKTHTCPPCPAPEAAGGPSVFLFPPKPKDTLMISRTPEVTC VVVDVSHEDPEVKFNWYVDGVEVHNAKTKPREEQYNSTYRVVSVL TVLHQDWLNGKEYKCKVSNKALPAPIEKTISKAKGQPREPQVYTLPP SREEMTKNQVSLTCLVKGFYPSDIAVEWESNGQPENNYKTTPPVLDS DGSFFLYSKLTVDKSRWQQGNVFSCSVMHEALHNHYTQKSLSLSPG QGQDRHMIRMRQLIDIVDQLKNYVNDLVPEFLPAPEDVETNCEWSAF SCFQKAQLKSANTGNNERIINVSIKKLKRKPPSTNATRRQKHRLTCPS CDSYEKKPPKEFLERFKSLLQKMIHQHLSSRTHGSEDS |
| 47 | Chain B in fusion protein | QVQLVQSGAEVKKPGSSVKVSCKASGGTFSSYAISWVRQAPGQGLE WMGLIIPMFDTAGYAQKFQGRVAITVDESTSTAYMELSSLRSEDTAVY YCARAEHSSTGTFDYWGQGTLVTVSS ASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDK KVEPKSCDKTHTCPPCPAPEAAGGPSVFLFPPKPKDTLMISRTPEVTC VVVDVSHEDPEVKFNWYVDGVEVHNAKTKPREEQYNSTYRVVSVL TVLHQDWLNGKEYKCKVSNKALPAPIEKTISKAKGQPREPQVYTLPP SREEMTKNQVSLTCLVKGFYPSDIAVEWESNGQPENNYKTTPPVLDS DGSFFLYSKLTVDKSRWQQGNVFSCSVMHEALHNHYTQKSLSLSPG QGQDRHMIRMRQLIDIVDQLKNYVNDLVPEFLPAPEDVETNCEWSAF SCFQKAQLKSANTGNNERIINVSIKKLKRKPPSTNAGRRQKHRLTCPS CDSYEKKPPKEFLERFKSLLQKMINQTLSSRTHGSEDS |
| 48 | Chain B in fusion protein | QVQLVQSGAEVKKPGSSVKVSCKASGGTFSSYAISWVRQAPGQGLE WMGLIIPMFDTAGYAQKFQGRVAITVDESTSTAYMELSSLRSEDTAVY YCARAEHSSTGTFDYWGQGTLVTVSS ASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDK KVEPKSCDKTHTCPPCPAPEAAGGPSVFLFPPKPKDTLMISRTPEVTC VVVDVSHEDPEVKFNWYVDGVEVHNAKTKPREEQYNSTYRVVSVL TVLHQDWLNGKEYKCKVSNKALPAPIEKTISKAKGQPREPQVYTLPP SREEMTKNQVSLTCLVKGFYPSDIAVEWESNGQPENNYKTTPPVLDS |
| | | DGSFFLYSKLTVDKSRWQQGNVFSCSVMHEALHNHYTQKSLSLSPG QGQDRHMIRMRQLIDIVDQLKNYVNDLVPEFLPAPENVTTNCEWSAF SCFQKAQLKSANTGNNERIINVSIKKLKRKPPSTNAGRRQKHRLTCPS CDSYEKKPPKEFLERFKSLLQKMIHQHLSSRTHGSEDS |
| 49 | Chain B in fusion protein | QVQLVQSGAEVKKPGSSVKVSCKASGGTFSSYAISWVRQAPGQGLE WMGLIIPMFDTAGYAQKFQGRVAITVDESTSTAYMELSSLRSEDTAVY YCARAEHSSTGTFDYWGQGTLVTVSS ASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDK KVEPKSCDKTHTCPPCPAPEAAGGPSVFLFPPKPKDTLMISRTPEVTC VVVDVSHEDPEVKFNWYVDGVEVHNAKTKPREEQYNSTYRVVSVL TVLHQDWLNGKEYKCKVSNKALPAPIEKTISKAKGQPREPQVYTLPP SREEMTKNQVSLTCLVKGFYPSDIAVEWESNGQPENNYKTTPPVLDS DGSFFLYSKLTVDKSRWQQGNVFSCSVMHEALHNHYTQKSLSLSPG QGQDRHMIRMRQLIDIVDQLKNYVNDLVPEFLPAPEDVETNCEWSAF SCFQKAQLKSANTGNNERIINVSIKKLKRKPPSTNAGKHRLTCPSCDS YEKKPPKEFLERFKSLLQKMIHQHLSSRTHGSEDS |
| 50 | Chain B in fusion protein | QVQLVQSGAEVKKPGSSVKVSCKASGGTFSSYAISWVRQAPGQGLE WMGLIIPMFDTAGYAQKFQGRVAITVDESTSTAYMELSSLRSEDTAVY YCARAEHSSTGTFDYWGQGTLVTVSS ASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDK KVEPKSCDKTHTCPPCPAPEAAGGPSVFLFPPKPKDTLMISRTPEVTC VVVDVSHEDPEVKFNWYVDGVEVHNAKTKPREEQYNSTYRVVSVL TVLHQDWLNGKEYKCKVSNKALPAPIEKTISKAKGQPREPQVYTLPP SREEMTKNQVSLTCLVKGFYPSDIAVEWESNGQPENNYKTTPPVLDS DGSFFLYSKLTVDKSRWQQGNVFSCSVMHEALHNHYTQKSLSLSPG QGQDRHMIRMRQLIDIVDQLKNYVNDLVPEFLPAPEDVETNCEWSAF SCFQKAQLKSANTGNNERIINVSIKKLKRKSTNAGRRQKHRLTCPSCD SYEKKPPKEFLERFKSLLQKMIHQHLSSRTHGSEDS |
| 51 | Chain B in fusion protein | QVQLVQSGAEVKKPGSSVKVSCKASGGTFSSYAISWVRQAPGQGLE WMGLIIPMFDTAGYAQKFQGRVAITVDESTSTAYMELSSLRSEDTAVY YCARAEHSSTGTFDYWGQGTLVTVSS ASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDK KVEPKSCDKTHTCPPCPAPEAAGGPSVFLFPPKPKDTLMISRTPEVTC VVVDVSHEDPEVKFNWYVDGVEVHNAKTKPREEQYNSTYRVVSVL TVLHQDWLNGKEYKCKVSNKALPAPIEKTISKAKGQPREPQVYTLPP |
| | | SREEMTKNQVSLTCLVKGFYPSDIAVEWESNGQPENNYKTTPPVLDS DGSFFLYSKLTVDKSRWQQGNVFSCSVMHEALHNHYTQKSLSLSPG HKSDITLQEMRQLIDIVDQLKNYVNDLVPEFLPAPEDVETNCEWSAFS CFQKAQLKSANTGNNERIINVSIKKLKRKPPSTNAGRRQKHRLTCPSC DSYEKKPPKEFLERFKSLLQKMIHQHLSSRTHGSEDS |
| 52 | Chain B in fusion protein | QVQLVQSGAEVKKPGSSVKVSCKASGGTFSSYAISWVRQAPGQGLE WMGLIIPMFDTAGYAQKFQGRVAITVDESTSTAYMELSSLRSEDTAVY YCARAEHSSTGTFDYWGQGTLVTVSS ASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDK KVEPKSCDKTHTCPPCPAPEAAGGPSVFLFPPKPKDTLMISRTPEVTC VVVDVSHEDPEVKFNWYVDGVEVHNAKTKPREEQYNSTYRVVSVL TVLHQDWLNGKEYKCKVSNKALPAPIEKTISKAKGQPREPQVYTLPP SREEMTKNQVSLTCLVKGFYPSDIAVEWESNGQPENNYKTTPPVLDS DGSFFLYSKLTVDKSRWQQGNVFSCSVMHEALHNHYTQKSLSLSPG QGQDRHMIRMRQLIDIVDQLKNYVNDLVPEFLPAPEDVETNCEWSAF SCFQKAQLKSANTGNNERIINVSIEKLKRKPPSTNAGRRQKHRLTCPS CDSYEKKPPKEFLERFKSLLQKMIHQHLSSRTHGSEDS |
| 53 | Chain B in fusion protein | QVQLVQSGAEVKKPGSSVKVSCKASGGTFSSYAISWVRQAPGQGLE WMGLIIPMFDTAGYAQKFQGRVAITVDESTSTAYMELSSLRSEDTAVY YCARAEHSSTGTFDYWGQGTLVTVSS ASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDK KVEPKSCDKTHTCPPCPAPEAAGGPSVFLFPPKPKDTLMISRTPEVTC VVVDVSHEDPEVKFNWYVDGVEVHNAKTKPREEQYNSTYRVVSVL TVLHQDWLNGKEYKCKVSNKALPAPIEKTISKAKGQPREPQVYTLPP SREEMTKNQVSLTCLVKGFYPSDIAVEWESNGQPENNYKTTPPVLDS DGSFFLYSKLTVDKSRWQQGNVFSCSVMHEALHNHYTQKSLSLSPG QGQDRHMQRMRQLIDIVDQLKNYVNDLVPEFLPAPEDVETNCEWSA FSCFQKAQLKSANTGNNERIINVSIKKLKRKPESTNAGRRQKHRLTCP SCDSYEKKPPKEFLERFKSLLQKMIHQHLSSRTHGSEDS |
| 54 | Chain B in fusion protein | QVQLVQSGAEVKKPGSSVKVSCKASGGTFSSYAISWVRQAPGQGLE WMGLIIPMFDTAGYAQKFQGRVAITVDESTSTAYMELSSLRSEDTAVY YCARAEHSSTGTFDYWGQGTLVTVSSASTKGPSVFPLAPSSKSTSGGT AALGCLVKDYFPEPVTVSWNSGALTSGVHTFPAVLQSSGLYSLSSVVT VPSSSLGTQTYICNVNHKPSNTKVDKKVEPKSCDKTHTCPPCPAPEA AGGPSVFLFPPKPKDTLMISRTPEVTCVVVDVSHEDPEVKFNWYVDG VEVHNAKTKPREEQYNSTYRVVSVLTVLHQDWLNGKEYKCKVSNK |
| | | ALPAPIEKTISKAKGQPREPQVYTLPPSREEMTKNQVSLTCLVKGFYPS DIAVEWESNGQPENNYKTTPPVLDSDGSFFLYSKLTVDKSRWQQGNV FSCSVMHEALHNHYTQKSLSLSPGQGQDRHMIRMRQLIDIVDQLKN YVNDLVPEFLPAPEDVETNCEWSAFSCFQKAQLKSANTGNNERIINVS IKKLKRDAPSTNAGRRQKHRLTCPSCDSYEKKPPKEFLERFKSLLQK MIHQHLSSRTHGSEDS |
| 55 | Chain B1 in fusion protein | QVQLVQSGAEVKKPGSSVKVSCKASGGTFSSYAISWVRQAPGQGLE WMGLIIPMFDTAGYAQKFQGRVAITVDESTSTAYMELSSLRSEDTAVY YCARAEHSSTGTFDYWGQGTLVTVSSASTKGPSVFPLAPSSKSTSGGT AALGCLVKDYFPEPVTVSWNSGALTSGVHTFPAVLQSSGLYSLSSVVT VPSSSLGTQTYICNVNHKPSNTKVDKKVEPKSCDKTHTCPPCPAPEA AGGPSVFLFPPKPKDTLMISRTPEVTCVVVDVSHEDPEVKFNWYVDG VEVHNAKTKPREEQYNSTYRVVSVLTVLHQDWLNGKEYKCKVSNK ALPAPIEKTISKAKGQPREPQVYTLPPCRDELTKNQVSLWCLVKGFYP SDIAVEWESNGQPENNYKTTPPVLDSDGSFFLYSKLTVDKSRWQQGN VFSCSVMHEALHNHYTQKSLSLSPGGGGGSGGGGSGGGGSHKSDITL QEMRQLIDIVDQLKNYVNDLVPEFLPAPEDVETNCEWSAFSCFQKAQ LKSANTGNNERIINVSIKKLKRKPPSTNAGRRQKHRLTCPSCDSYEKK PPKEFLERFKSLLQKMIHQHLSSRTHGSEDS |
| 56 | Chain B1 in fusion protein | QVQLVQSGAEVKKPGSSVKVSCKASGGTFSSYAISWVRQAPGQGLE WMGLIIPMFDTAGYAQKFQGRVAITVDESTSTAYMELSSLRSEDTAVY YCARAEHSSTGTFDYWGQGTLVTVSSASTKGPSVFPLAPSSKSTSGGT AALGCLVKDYFPEPVTVSWNSGALTSGVHTFPAVLQSSGLYSLSSVVT VPSSSLGTQTYICNVNHKPSNTKVDKKVEPKSCDKTHTCPPCPAPEA AGGPSVFLFPPKPKDTLMISRTPEVTCVVVDVSHEDPEVKFNWYVDG VEVHNAKTKPREEQYNSTYRVVSVLTVLHQDWLNGKEYKCKVSNK ALPAPIEKTISKAKGQPREPQVYTLPPCRDELTKNQVSLWCLVKGFYP SDIAVEWESNGQPENNYKTTPPVLDSDGSFFLYSKLTVDKSRWQQGN VFSCSVMHEALHNHYTQKSLSLSPGGGGGSGGGGSGGGGSHKSDITL QEIIKTLNIVDQLKNYVNDLVPEFLPAPEDVETNCEWSAFSCFQKAQL KSANTGNNERIINVSIKKLKRKPPSTNAGRRQKHRLTCPSCDSYEKKP PKEFLERFKSLLQKMIHQHLSSRTHGSEDS |
| 57 | Chain B1 in fusion protein | QVQLVQSGAEVKKPGSSVKVSCKASGGTFSSYAISWVRQAPGQGLE WMGLIIPMFDTAGYAQKFQGRVAITVDESTSTAYMELSSLRSEDTAVY YCARAEHSSTGTFDYWGQGTLVTVSSASTKGPSVFPLAPSSKSTSGGT AALGCLVKDYFPEPVTVSWNSGALTSGVHTFPAVLQSSGLYSLSSVVT VPSSSLGTQTYICNVNHKPSNTKVDKKVEPKSCDKTHTCPPCPAPEA AGGPSVFLFPPKPKDTLMISRTPEVTCVVVDVSHEDPEVKFNWYVDG |
| | | VEVHNAKTKPREEQYNSTYRVVSVLTVLHQDWLNGKEYKCKVSNK ALPAPIEKTISKAKGQPREPQVYTLPPCRDELTKNQVSLWCLVKGFYP SDIAVEWESNGQPENNYKTTPPVLDSDGSFFLYSKLTVDKSRWQQGN VFSCSVMHEALHNHYTQKSLSLSPGGGGGSGGGGSGGGGSHKSDITL QEIIKLIDIVDQLKNYVNDLVPEFLPAPEDVETNCEWSAFSCFQKAQL KSANTGNNERIINVSIKKLKRKPPSTNAGRRQKHRLTCPSCDSYEKKP PKEFLERFKSLLQKMIHQHLSSRTHGSEDS |
| 58 | Chain B1 in fusion protein | QVQLVQSGAEVKKPGSSVKVSCKASGGTFSSYAISWVRQAPGQGLE WMGLIIPMFDTAGYAQKFQGRVAITVDESTSTAYMELSSLRSEDTAVY YCARAEHSSTGTFDYWGQGTLVTVSSASTKGPSVFPLAPSSKSTSGGT AALGCLVKDYFPEPVTVSWNSGALTSGVHTFPAVLQSSGLYSLSSVVT VPSSSLGTQTYICNVNHKPSNTKVDKKVEPKSCDKTHTCPPCPAPEA AGGPSVFLFPPKPKDTLMISRTPEVTCVVVDVSHEDPEVKFNWYVDG VEVHNAKTKPREEQYNSTYRVVSVLTVLHQDWLNGKEYKCKVSNK ALPAPIEKTISKAKGQPREPQVYTLPPCRDELTKNQVSLWCLVKGFYP SDIAVEWESNGQPENNYKTTPPVLDSDGSFFLYSKLTVDKSRWQQGN VFSCSVMHEALHNHYTQKSLSLSPGGGGGSGGGGSGGGGSHKSDITL QEIIQLIDIVDQLKNYVNDLVPEFLPAPEDVETNCEWSAFSCFQKAQL KSANTGNNERIINVSIKKLKRKPPSTNAGRRQKHRLTCPSCDSYEKKP PKEFLERFKSLLQKMIHQHLSSRTHGSEDS |
| 59 | Chain B1 in fusion protein | QVQLVQSGAEVKKPGSSVKVSCKASGGTFSSYAISWVRQAPGQGLE WMGLIIPMFDTAGYAQKFQGRVAITVDESTSTAYMELSSLRSEDTAVY YCARAEHSSTGTFDYWGQGTLVTVSSASTKGPSVFPLAPSSKSTSGGT AALGCLVKDYFPEPVTVSWNSGALTSGVHTFPAVLQSSGLYSLSSVVT VPSSSLGTQTYICNVNHKPSNTKVDKKVEPKSCDKTHTCPPCPAPEA AGGPSVFLFPPKPKDTLMISRTPEVTCVVVDVSHEDPEVKFNWYVDG VEVHNAKTKPREEQYNSTYRVVSVLTVLHQDWLNGKEYKCKVSNK ALPAPIEKTISKAKGQPREPQVYTLPPCRDELTKNQVSLWCLVKGFYP SDIAVEWESNGQPENNYKTTPPVLDSDGSFFLYSKLTVDKSRWQQGN VFSCSVMHEALHNHYTQKSLSLSPGGGGGSGGGGSGGGGSHKSDITL QEMRQLIDIVDQLKNYVNDLVPEFLPAPEDVETNCEWSAFSCFQKAQ LKSANTGNNERIINVSIKKLKRKSTNAGRRQKHRLTCPSCDSYEKKPP KEFLERFKSLLQKMIHQHLSSRTHGSEDS |
| 60 | Chain B1 in fusion protein | QVQLVQSGAEVKKPGSSVKVSCKASGGTFSSYAISWVRQAPGQGLE WMGLIIPMFDTAGYAQKFQGRVAITVDESTSTAYMELSSLRSEDTAVY YCARAEHSSTGTFDYWGQGTLVTVSSASTKGPSVFPLAPSSKSTSGGT AALGCLVKDYFPEPVTVSWNSGALTSGVHTFPAVLQSSGLYSLSSVVT VPSSSLGTQTYICNVNHKPSNTKVDKKVEPKSCDKTHTCPPCPAPEA |
| | | AGGPSVFLFPPKPKDTLMISRTPEVTCVVVDVSHEDPEVKFNWYVDG VEVHNAKTKPREEQYNSTYRVVSVLTVLHQDWLNGKEYKCKVSNK ALPAPIEKTISKAKGQPREPQVYTLPPCRDELTKNQVSLWCLVKGFYP SDIAVEWESNGQPENNYKTTPPVLDSDGSFFLYSKLTVDKSRWQQGN VFSCSVMHEALHNHYTQKSLSLSPGGGGGSGGGGSGGGGSMRQLIDI VDQLKNYVNDLVPEFLPAPEDVETNCEWSAFSCFQKAQLKSANTGN NERIINVSIKKLKRKPPSTNAGRRQKHRLTCPSCDSYEKKPPKEFLERF KSLLQKMIHQHLSSRTHGSEDS |
| 61 | Chain B1 in fusion protein | QVQLVQSGAEVKKPGSSVKVSCKASGGTFSSYAISWVRQAPGQGLE WMGLIIPMFDTAGYAQKFQGRVAITVDESTSTAYMELSSLRSEDTAVY YCARAEHSSTGTFDYWGQGTLVTVSSASTKGPSVFPLAPSSKSTSGGT AALGCLVKDYFPEPVTVSWNSGALTSGVHTFPAVLQSSGLYSLSSVVT VPSSSLGTQTYICNVNHKPSNTKVDKKVEPKSCDKTHTCPPCPAPEA AGGPSVFLFPPKPKDTLMISRTPEVTCVVVDVSHEDPEVKFNWYVDG VEVHNAKTKPREEQYNSTYRVVSVLTVLHQDWLNGKEYKCKVSNK ALPAPIEKTISKAKGQPREPQVYTLPPCRDELTKNQVSLWCLVKGFYP SDIAVEWESNGQPENNYKTTPPVLDSDGSFFLYSKLTVDKSRWQQGN VFSCSVMHEALHNHYTQKSLSLSPGGGGGSGGGGSGGGGSMRQLIDI VDNLTNYVNDLVPEFLPAPEDVETNCEWSAFSCFQKAQLKSANTGNN ERIINVSIKKLKRKPPSTNAGRRQKHRLTCPSCDSYEKKPPKEFLERFK SLLQKMIHQHLSSRTHGSEDS |
| 62 | Chain B1 in fusion protein | QVQLVQSGAEVKKPGSSVKVSCKASGGTFSSYAISWVRQAPGQGLE WMGLIIPMFDTAGYAQKFQGRVAITVDESTSTAYMELSSLRSEDTAVY YCARAEHSSTGTFDYWGQGTLVTVSSASTKGPSVFPLAPSSKSTSGGT AALGCLVKDYFPEPVTVSWNSGALTSGVHTFPAVLQSSGLYSLSSVVT VPSSSLGTQTYICNVNHKPSNTKVDKKVEPKSCDKTHTCPPCPAPEA AGGPSVFLFPPKPKDTLMISRTPEVTCVVVDVSHEDPEVKFNWYVDG VEVHNAKTKPREEQYNSTYRVVSVLTVLHQDWLNGKEYKCKVSNK ALPAPIEKTISKAKGQPREPQVYTLPPCRDELTKNQVSLWCLVKGFYP SDIAVEWESNGQPENNYKTTPPVLDSDGSFFLYSKLTVDKSRWQQGN VFSCSVMHEALHNHYTQKSLSLSPGGGGGSGGGGSGGGGSMRQLIDI VDQLKNYVNDLVPEFLPAPEDVETNCEWSAFSCFQKAQLKSANTGN NERIINVSIKKLKRKSTNAGRRQKHRLTCPSCDSYEKKPPKEFLERFKS LLQKMIHQHLSSRTHGSEDS |
| 63 | Chain B1 in fusion protein | QVQLVQSGAEVKKPGSSVKVSCKASGGTFSSYAISWVRQAPGQGLE WMGLIIPMFDTAGYAQKFQGRVAITVDESTSTAYMELSSLRSEDTAVY YCARAEHSSTGTFDYWGQGTLVTVSSASTKGPSVFPLAPSSKSTSGGT AALGCLVKDYFPEPVTVSWNSGALTSGVHTFPAVLQSSGLYSLSSVVT |
| | | VPSSSLGTQTYICNVNHKPSNTKVDKKVEPKSCDKTHTCPPCPAPEA AGGPSVFLFPPKPKDTLMISRTPEVTCVVVDVSHEDPEVKFNWYVDG VEVHNAKTKPREEQYNSTYRVVSVLTVLHQDWLNGKEYKCKVSNK ALPAPIEKTISKAKGQPREPQVYTLPPCRDELTKNQVSLWCLVKGFYP SDIAVEWESNGQPENNYKTTPPVLDSDGSFFLYSKLTVDKSRWQQGN VFSCSVMHEALHNHYTQKSLSLSPGGGGGSGGGGSGGGGSQGQDRH MIRMRQLIDIVDNLTNYVNDLVPEFLPAPEDVETNAEWSAFSCFQKA QLKSANTGNNERIINVSIKKLKRKPPSTNATTPSCDSYEKKPPKEFLER FKSLLQKMIHQHLSSRTHGSEDS |
| 64 | Chain B1 in fusion protein | QVQLVQSGAEVKKPGSSVKVSCKASGGTFSSYAISWVRQAPGQGLE WMGLIIPMFDTAGYAQKFQGRVAITVDESTSTAYMELSSLRSEDTAVY YCARAEHSSTGTFDYWGQGTLVTVSSASTKGPSVFPLAPSSKSTSGGT AALGCLVKDYFPEPVTVSWNSGALTSGVHTFPAVLQSSGLYSLSSVVT VPSSSLGTQTYICNVNHKPSNTKVDKKVEPKSCDKTHTCPPCPAPEA AGGPSVFLFPPKPKDTLMISRTPEVTCVVVDVSHEDPEVKFNWYVDG VEVHNAKTKPREEQYNSTYRVVSVLTVLHQDWLNGKEYKCKVSNK ALPAPIEKTISKAKGQPREPQVYTLPPCRDELTKNQVSLWCLVKGFYP SDIAVEWESNGQPENNYKTTPPVLDSDGSFFLYSKLTVDKSRWQQGN VFSCSVMHEALHNHYTQKSLSLSPGGGGGSGGGGSGGGGSHKSDITL QEIIMRQLIDIVDQLKNYVNDLVPEFLPAPEDVETNCEWSAFSCFQKA QLKSANTGNNERIINVSIKKLKRKPPSTNAGRRQKHRLTCPSCDSYEK KPPKEFLERFKSLLQKMIHQHLSSRTHGSEDS |
| 65 | Chain B1 in fusion protein | QVQLVQSGAEVKKPGSSVKVSCKASGGTFSSYAISWVRQAPGQGLE WMGLIIPMFDTAGYAQKFQGRVAITVDESTSTAYMELSSLRSEDTAVY YCARAEHSSTGTFDYWGQGTLVTVSSASTKGPSVFPLAPSSKSTSGGT AALGCLVKDYFPEPVTVSWNSGALTSGVHTFPAVLQSSGLYSLSSVVT VPSSSLGTQTYICNVNHKPSNTKVDKKVEPKSCDKTHTCPPCPAPEA AGGPSVFLFPPKPKDTLMISRTPEVTCVVVDVSHEDPEVKFNWYVDG VEVHNAKTKPREEQYNSTYRVVSVLTVLHQDWLNGKEYKCKVSNK ALPAPIEKTISKAKGQPREPQVYTLPPCRDELTKNQVSLWCLVKGFYP SDIAVEWESNGQPENNYKTTPPVLDSDGSFFLYSKLTVDKSRWQQGN VFSCSVMHEALHNHYTQKSLSLSPGGGGGSGGGGSGGGGSHKSDITL QEMRQLIDIVDQLKNYVNDLVPEFLPAPEDVETNCEWSAFSCFQKAQ LKSANTGNNERIINVSIKKLKRKPPSTNAGRRQKHRLTCPSCDSYEKK PPKEFLERFKSLLQNMTHQHLSSRTHGSEDS |
| 66 | Chain B1 in fusion protein | QVQLVQSGAEVKKPGSSVKVSCKASGGTFSSYAISWVRQAPGQGLE WMGLIIPMFDTAGYAQKFQGRVAITVDESTSTAYMELSSLRSEDTAVY YCARAEHSSTGTFDYWGQGTLVTVSSASTKGPSVFPLAPSSKSTSGGT |
| | | AALGCLVKDYFPEPVTVSWNSGALTSGVHTFPAVLQSSGLYSLSSVVT VPSSSLGTQTYICNVNHKPSNTKVDKKVEPKSCDKTHTCPPCPAPEA AGGPSVFLFPPKPKDTLMISRTPEVTCVVVDVSHEDPEVKFNWYVDG VEVHNAKTKPREEQYNSTYRVVSVLTVLHQDWLNGKEYKCKVSNK ALPAPIEKTISKAKGQPREPQVYTLPPCRDELTKNQVSLWCLVKGFYP SDIAVEWESNGQPENNYKTTPPVLDSDGSFFLYSKLTVDKSRWQQGN VFSCSVMHEALHNHYTQKSLSLSPGGGGGSGGGGSGGGGSHKSDITL QEMRQLIDIVDQLKNYVNDLVPEFLPAPENVTTNCEWSAFSCFQKAQ LKSANTGNNERIINVSIKKLKRKPPSTNAGRRQKHRLTCPSCDSYEKK PPKEFLERFKSLLQKMIHQHLSSRTHGSEDS |
| 67 | Chain B1 in fusion protein | QVQLVQSGAEVKKPGSSVKVSCKASGGTFSSYAISWVRQAPGQGLE WMGLIIPMFDTAGYAQKFQGRVAITVDESTSTAYMELSSLRSEDTAVY YCARAEHSSTGTFDYWGQGTLVTVSSASTKGPSVFPLAPSSKSTSGGT AALGCLVKDYFPEPVTVSWNSGALTSGVHTFPAVLQSSGLYSLSSVVT VPSSSLGTQTYICNVNHKPSNTKVDKKVEPKSCDKTHTCPPCPAPEA AGGPSVFLFPPKPKDTLMISRTPEVTCVVVDVSHEDPEVKFNWYVDG VEVHNAKTKPREEQYNSTYRVVSVLTVLHQDWLNGKEYKCKVSNK ALPAPIEKTISKAKGQPREPQVYTLPPCRDELTKNQVSLWCLVKGFYP SDIAVEWESNGQPENNYKTTPPVLDSDGSFFLYSKLTVDKSRWQQGN VFSCSVMHEALHNHYTQKSLSLSPGGGGGSGGGGSGGGGSHKSDITL QEMRQLINITDQLKNYVNDLVPEFLPAPEDVETNCEWSAFSCFQKAQ LKSANTGNNERIINVSIKKLKRKPPSTNAGRRQKHRLTCPSCDSYEKK PPKEFLERFKSLLQKMIHQHLSSRTHGSEDS |
| 68 | Chain B1 in control fusion protein | QVQLVQSGAEVKKPGSSVKVSCKASGGTFSSYAISWVRQAPGQGLE WMGLIIPMFDTAGYAQKFQGRVAITVDESTSTAYMELSSLRSEDTAVY YCARAEHSSTGTFDYWGQGTLVTVSSASTKGPSVFPLAPSSKSTSGGT AALGCLVKDYFPEPVTVSWNSGALTSGVHTFPAVLQSSGLYSLSSVVT VPSSSLGTQTYICNVNHKPSNTKVDKKVEPKSCDKTHTCPPCPAPEA AGGPSVFLFPPKPKDTLMISRTPEVTCVVVDVSHEDPEVKFNWYVDG VEVHNAKTKPREEQYNSTYRVVSVLTVLHQDWLNGKEYKCKVSNK ALPAPIEKTISKAKGQPREPQVYTLPPCRDELTKNQVSLWCLVKGFYP SDIAVEWESNGQPENNYKTTPPVLDSDGSFFLYSKLTVDKSRWQQGN VFSCSVMHEALHNHYTQKSLSLSPGQGQDQHMIRMRQLIDIVDQLK NYVNDLVPEFLPAPEDVETNCEWSAFSCFQKAQLKSANTGNNERIIN VSIKKLKEKPPSTNAGRRQKHRLTCPSCDSYEKKPPKEFLERFKSLLQ KMIHQHLSSRTHGSEDS |
| 69 | Linker, (G₄S)₂ | GGGGSGGGGS |
| 70 | Fc fragment of IgG1, with an LALA mutation | DKTHTCPPCPAPEAAGGPSVFLFPPKPKDTLMISRTPEVTCVVVDVSH EDPEVKFNWYVDGVEVHNAKTKPREEQYNSTYRVVSVLTVLHQDW LNGKEYKCKVSNKALPAPIEKTISKAKGQPREPQVYTLPPSREEMTK NQVSLTCLVKGFYPSDIAVEWESNGQPENNYKTTPPVLDSDGSFFLYS KLTVDKSRWQQGNVFSCSVMHEALHNHYTQKSLSLSPGK |
| 71 | Heavy chain of anti-PD-1 antibody, in the IgG1 form, with an LALA mutation | QVQLVQSGAEVKKPGSSVKVSCKASGGTFSSYAISWVRQAPGQGLE WMGLIIPMFDTAGYAQKFQGRVAITVDESTSTAYMELSSLRSEDTAVY YCARAEHSSTGTFDYWGQGTLVTVSSASTKGPSVFPLAPSSKSTSGGT AALGCLVKDYFPEPVTVSWNSGALTSGVHTFPAVLQSSGLYSLSSVVT VPSSSLGTQTYICNVNHKPSNTKVDKKVEPKSCDKTHTCPPCPAPEA AGGPSVFLFPPKPKDTLMISRTPEVTCVVVDVSHEDPEVKFNWYVDG VEVHNAKTKPREEQYNSTYRVVSVLTVLHQDWLNGKEYKCKVSNK ALPAPIEKTISKAKGQPREPQVYTLPPSREEMTKNQVSLTCLVKGFYPS DIAVEWESNGQPENNYKTTPPVLDSDGSFFLYSKLTVDKSRWQQGNV FSCSVMHEALHNHYTQKSLSLSPG |
| 72 | Linker, (G₄S)₃ | GGGGSGGGGSGGGGS |
| 73 | Heavy chain of anti-PD-1 antibody comprising a knob mutation, in the IgG1 form, with an LALA mutation | QVQLVQSGAEVKKPGSSVKVSCKASGGTFSSYAISWVRQAPGQGLE WMGLIIPMFDTAGYAQKFQGRVAITVDESTSTAYMELSSLRSEDTAVY YCARAEHSSTGTFDYWGQGTLVTVSSASTKGPSVFPLAPSSKSTSGGT AALGCLVKDYFPEPVTVSWNSGALTSGVHTFPAVLQSSGLYSLSSVVT VPSSSLGTQTYICNVNHKPSNTKVDKKVEPKSCDKTHTCPPCPAPEA AGGPSVFLFPPKPKDTLMISRTPEVTCVVVDVSHEDPEVKFNWYVDG VEVHNAKTKPREEQYNSTYRVVSVLTVLHQDWLNGKEYKCKVSNK ALPAPIEKTISKAKGQPREPQVYTLPPCRDELTKNQVSLWCLVKGFYP SDIAVEWESNGQPENNYKTTPPVLDSDGSFFLYSKLTVDKSRWQQGN VFSCSVMHEALHNHYTQKSLSLSPG |
| 74 | IL-21-001, wild type | |
| 75 | IL-21-002 | HKSDIHMIRMRQLIDIVDQLKNYVNDLVPEFLPAPEDVETNCEWSAFS CFQKAQLKSANTGNNERIINVSIKKLKRKPPSTNAGRRQKHRLTCPSC DSYEKKPPKEFLERFKSLLQKMIHQHLSSRTHGSEDS |
| 76 | IL-21-003 | HKCDIHMIRMRQLIDIVDQLKNYVNDLVPEFLPAPEDVETNCEWSAF SCFQKAQLKSANTGNNERIINVSIKKLKRKPPSTNAGRRQKHRLTCPS CDSYEKKPPKEFLERFKSLLQKMIHQHCSSRTHGSEDS |
| 77 | IL-21-004 | HKSDITLQEMRQLIDIVDQLKNYVNDLVPEFLPAPEDVETNCEWSAFS CFQKAQLKSANTGNNERIINVSIKKLKRKPPSTNAGRRQKHRLTCPSC |
| | | DSYEKKPPKEFLERFKSLLQKMIHQHLSSRTHGSEDS |
| 78 | IL-21-005 | HKSDITLQEIIKTLNIVDQLKNYVNDLVPEFLPAPEDVETNCEWSAFSC FQKAQLKSANTGNNERIINVSIKKLKRKPPSTNAGRRQKHRLTCPSCD SYEKKPPKEFLERFKSLLQKMIHQHLSSRTHGSEDS |
| 79 | IL-21-006 | HKSDITLQEIIKLIDIVDQLKNYVNDLVPEFLPAPEDVETNCEWSAFSC FQKAQLKSANTGNNERIINVSIKKLKRKPPSTNAGRRQKHRLTCPSCD SYEKKPPKEFLERFKSLLQKMIHQHLSSRTHGSEDS |
| 80 | IL-21-007 | HKSDITLQEIIQLIDIVDQLKNYVNDLVPEFLPAPEDVETNCEWSAFSC FQKAQLKSANTGNNERIINVSIKKLKRKPPSTNAGRRQKHRLTCPSCD SYEKKPPKEFLERFKSLLQKMIHQHLSSRTHGSEDS |
| 81 | IL-21-008 | HKSDITLQEIIMRQLIDIVDQLKNYVNDLVPEFLPAPEDVETNCEWSAF SCFQKAQLKSANTGNNERIINVSIKKLKRKPPSTNAGRRQKHRLTCPS CDSYEKKPPKEFLERFKSLLQKMIHQHLSSRTHGSEDS |
| 82 | IL-21-009 | QGQNRTMIRMRQLIDIVDQLKNYVNDLVPEFLPAPEDVETNCEWSAF SCFQKAQLKSANTGNNERIINVSIKKLKRKPPSTNAGRRQKHRLTCPS CDSYEKKPPKEFLERFKSLLQKMIHQHLSSRTHGSEDS |
| 83 | IL-21-010 | QGQDNHTIRMRQLIDIVDQLKNYVNDLVPEFLPAPEDVETNCEWSAF SCFQKAQLKSANTGNNERIINVSIKKLKRKPPSTNAGRRQKHRLTCPS CDSYEKKPPKEFLERFKSLLQKMIHQHLSSRTHGSEDS |
| 84 | IL-21-011 | QGQDRHMIRMRQLINITDQLKNYVNDLVPEFLPAPEDVETNCEWSAF SCFQKAQLKSANTGNNERIINVSIKKLKRKPPSTNAGRRQKHRLTCPS CDSYEKKPPKEFLERFKSLLQKMIHQHLSSRTHGSEDS |
| 85 | IL-21-012 | QGQDRHMIRMRQLIDIVDNLTNYVNDLVPEFLPAPEDVETNCEWSAF SCFQKAQLKSANTGNNERIINVSIKKLKRKPPSTNAGRRQKHRLTCPS CDSYEKKPPKEFLERFKSLLQKMIHQHLSSRTHGSEDS |
| 86 | IL-21-013 | QGQDRHMIRMRQLIDIVDQLKNYVNDLVPEFLPAPEDVETNCEWSAF SCFQKAQLKSANTGNNERIINVSIKKLKNKTPSTNAGRRQKHRLTCPS CDSYEKKPPKEFLERFKSLLQKMIHQHLSSRTHGSEDS |
| 87 | IL-21-014 | QGQDRHMIRMRQLIDIVDQLKNYVNDLVPEFLPAPEDVETNCEWSAF SCFQKAQLKSANTGNNERIINVSIKKLKRNSTSTNAGRRQKHRLTCPS CDSYEKKPPKEFLERFKSLLQKMIHQHLSSRTHGSEDS |
| 88 | IL-21-015 | QGQDRHMIRMRQLIDIVDQLKNYVNDLVPEFLPAPEDVETNCEWSAF SCFQKAQLKSANTGNNERIINVSIKKLKRKPNSTNAGRRQKHRLTCPS CDSYEKKPPKEFLERFKSLLQKMIHQHLSSRTHGSEDS |
| 89 | IL-21-016 | QGQDRHMIRMRQLIDIVDQLKNYVNDLVPEFLPAPEDVETNCEWSAF SCFQKAQLKSANTGNNERIINVSIKKLKRKPPNTTAGRRQKHRLTCPS CDSYEKKPPKEFLERFKSLLQKMIHQHLSSRTHGSEDS |
| 90 | IL-21-017 | QGQDRHMIRMRQLIDIVDQLKNYVNDLVPEFLPAPEDVETNCEWSAF |
| | | SCFQKAQLKSANTGNNERIINVSIKKLKRKPPSTNATRRQKHRLTCPS CDSYEKKPPKEFLERFKSLLQKMIHQHLSSRTHGSEDS |
| 91 | IL-21-018 | QGQDRHMIRMRQLIDIVDQLKNYVNDLVPEFLPAPEDVETNCEWSAF SCFQKAQLKSANTGNNERIINVSIKKLKRKPPSTNAGRRQKHRLTCPS CDSYEKKPPKEFLERFKSLLQKMINQTLSSRTHGSEDS |
| 92 | IL-21-019 | QGQDRHMIRMRQLIDIVDQLKNYVNDLVPEFLPAPENVTTNCEWSAF SCFQKAQLKSANTGNNERIINVSIKKLKRKPPSTNAGRRQKHRLTCPS CDSYEKKPPKEFLERFKSLLQKMIHQHLSSRTHGSEDS |
| 93 | IL-21-020 | QGQDRHMIRMRQLIDIVDQLKNYVNDLVPEFLPAPEDVETNCEWSAF SCFQKAQLKSANTGNNERIINVSIEKLKRKPPSTNAGRRQKHRLTCPS CDSYEKKPPKEFLERFKSLLQKMIHQHLSSRTHGSEDS |
| 94 | IL-21-021 | QGQDRHMQRMRQLIDIVDQLKNYVNDLVPEFLPAPEDVETNCEWSA FSCFQKAQLKSANTGNNERIINVSIKKLKRKPESTNAGRRQKHRLTCP SCDSYEKKPPKEFLERFKSLLQKMIHQHLSSRTHGSEDS |
| 95 | IL-21-022 | QGQDRHMIRMRQLIDIVDQLKNYVNDLVPEFLPAPEDVETNCEWSAF SCFQKAQLKSANTGNNERIINVSIKKLKRDAPSTNAGRRQKHRLTCPS CDSYEKKPPKEFLERFKSLLQKMIHQHLSSRTHGSEDS |
| 96 | IL-21-023 | QGQDRHMIRMRQLIDIVDQLKNYVNDLVPEFLPAPEDVETNCEWSAF SCFQKAQLKSANTGNNERIINVSIKKLKRKPPSTNAGKHRLTCPSCDS YEKKPPKEFLERFKSLLQKMIHQHLSSRTHGSEDS |
| 97 | IL-21-024 | QGQDRHMIRMRQLIDIVDQLKNYVNDLVPEFLPAPEDVETNCEWSAF SCFQKAQLKSANTGNNERIINVSIKKLKRKSTNAGRRQKHRLTCPSCD SYEKKPPKEFLERFKSLLQKMIHQHLSSRTHGSEDS |
| 98 | IL-21-025 | MRQLIDIVDQLKNY VNDLVPEFLPAPEDVETNCEWSAFSCFQKAQLK SANTGNNERIINVSIKKLKRKPPSTNAGRRQKHRLTCPSCDSYEKKPP KEFLERFKSLLQKMIHQHLSSRTHGSEDS |
| 99 | IL-21-026 | MRQLIDIVDQLKNYVNDLVPEFLPAPEDVETNCEWSAFSCFQKAQLK SANTGNNERIINVSIKKLKRKSTNAGRRQKHRLTCPSCDSYEKKPPKE FLERFKSLLQKMIHQHLSSRTHGSEDS |
| 100 | IL-21-027 | MRQLIDIVDNLTNYVNDLVPEFLPAPEDVETNCEWSAFSCFQKAQLK SANTGNNERIINVSIKKLKRKPPSTNAGRRQKHRLTCPSCDSYEKKPP KEFLERFKSLLQKMIHQHLSSRTHGSEDS |
| 101 | IL-21-028 | QGQDRHMIRMRQLIDIVDNLTNYVNDLVPEFLPAPEDVETNAEWSAF SCFQKAQLKSANTGNNERIINVSIKKLKRKPPSTNATTPSCDSYEKKPP KEFLERFKSLLQKMIHQHLSSRTHGSEDS |
| 102 | IL-21-029 | HKSDITLQEMRQLIDIVDQLKNYVNDLVPEFLPAPEDVETNCEWSAFS CFQKAQLKSANTGNNERIINVSIKKLKRKSTNAGRRQKHRLTCPSCDS YEKKPPKEFLERFKSLLQKMIHQHLSSRTHGSEDS |
| 103 | IL-21-030 | HKSDITLQEMRQLIDIVDQLKNYVNDLVPEFLPAPEDVETNCEWSAFS CFQKAQLKSANTGNNERIINVSIKKLKRKPPSTNAGRRQKHRLTCPSC DSYEKKPPKEFLERFKSLLQNMTHQHLSSRTHGSEDS |
| 104 | IL-21-031 | HKSDITLQEMRQLIDIVDQLKNYVNDLVPEFLPAPENVTTNCEWSAFS CFQKAQLKSANTGNNERIINVSIKKLKRKPPSTNAGRRQKHRLTCPSC DSYEKKPPKEFLERFKSLLQKMIHQHLSSRTHGSEDS |
| 105 | IL-21-032 | HKSDITLQEMRQLINITDQLKNYVNDLVPEFLPAPEDVETNCEWSAFS CFQKAQLKSANTGNNERIINVSIKKLKRKPPSTNAGRRQKHRLTCPSC DSYEKKPPKEFLERFKSLLQKMIHQHLSSRTHGSEDS |
| 106 | IL-21 (comprising a signal peptide) | MRSSPGNMERIVICLMVIFLGTLVHKSSSQGQDRHMIRMRQLIDIVDQ LKNYVNDLVPEFLPAPEDVETNEWSAFSCFQKAQLKSANTGNNERII NVSIKKLKRKPPSTNAGRRQKHRLTCPSCDSYEKKPPKEFLERFKSLL QKMIHQ HLSSRTHGSEDS |
| 107 | IL-21 native splice variant | MRSSPGNMERIVICLMVIFLGTLVHKSSSQGQDRHMIRMRQLIDIVDQ LKNYVNDLVPEFLPAPEDVETNCEWSAFSCFQKAQLKSANTGNNERII NVSIKKLKRKPPSTNAGRRQKHRLTCPSCDSYEKKPPKEFLERFKSLL QKVSTLSFI |
| 108 | IL-21 native splice variant (comprising a signal peptide) | QGQDRHMIRMRQLIDIVDQLKNYVNDLVPEFLPAPEDVETNCEWSAF SCFQKAQLKSANTGNNERIINVSIKKLKRKPPSTNAGRRQKHRLTCPS CDSYEKKPPKEFLERFKSLLQKVSTLSFI |
| 109 | anti PD-1 HCDR1 | KASGGTFSSYAIS |
| 110 | anti PD-1 HCDR2 | LIIPMFDTAGYAQKFQG |
| 111 | anti PD-1 HCDR3 | ARAEHSSTGTFDY |
| 112 | anti PD-1 LCDR1 | RASQGISSWLA |
| 113 | anti PD-1 LCDR2 | SAASSLQS |
| 114 | anti PD-1 LCDR3 | QQANHLPFT |
| 115 | anti PD-1 VH | QVQLVQSGAEVKKPGSSVKVSCKASGGTFSSYAISWVRQAPGQGLE WMGLIIPMFDTAGYAQKFQGRVAITVDESTSTAYMELSSLRSEDTAVY YCARAEHSSTGTFDYWGQGTLVTVSS |
| 116 | anti PD-1 VL | DIQMTQSPSSVSASVGDRVTITCRASQGISSWLAWYQQKPGKAPKLLI SAASSLQSGVPSRFSGSGSGTDFTLTISSLQPEDFATYYCQQANHLPFT FGGGTKVEIK |
| 117 | IL-4 | |

## Claims

1. An IL-21 mutant protein, wherein the mutant protein comprises one or more of the following mutations compared to wild-type IL-21 (preferably human IL-21, and more preferably IL-21 comprising a sequence of SEQ ID NO: 74):
(i) replacement of amino acids at positions 1-15 of IL-21 with amino acids at positions 1-15 or amino acids comprising CS3 at positions 1-15 of IL-4 (preferably human IL-4); replacement of amino acids at positions 1-14 of IL-21 with amino acids at positions 1-14 or amino acids comprising CS3 at positions 1-14 of IL-4; replacement of amino acids at positions 1-13 of IL-21 with amino acids at positions 1-13 or amino acids comprising CS3 at positions 1-13 of IL-4; replacement of amino acids at positions 1-12 of IL-21 with amino acids at positions 1-12 or amino acids comprising CS3 at positions 1-12 of IL-4; replacement of amino acids at positions 1-11 of IL-21 with amino acids at positions 1-11 or amino acids comprising CS3 at positions 1-11 of IL-4; replacement of amino acids at positions 1-10 of IL-21 with amino acids at positions 1-10 or amino acids comprising CS3 at positions 1-10 of IL-4; replacement of amino acids at positions 1-9 of IL-21 with amino acids at positions 1-9 or amino acids comprising CS3 at positions 1-9 of IL-4; replacement of amino acids at positions 1-8 of IL-21 with amino acids at positions 1-8 or amino acids comprising CS3 at positions 1-8 of IL-4; replacement of amino acids at positions 1-7 of IL-21 with amino acids at positions 1-7 or amino acids comprising CS3 at positions 1-7 of IL-4; replacement of amino acids at positions 1-6 of IL-21 with amino acids at positions 1-6 or amino acids comprising CS3 at positions 1-6 of IL-4; replacement of amino acids at positions 1-5 of IL-21 with amino acids at positions 1-5 or amino acids comprising CS3 at positions 1-5 of IL-4; replacement of amino acids at positions 1-4 of IL-21 with amino acids at positions 1-4 or amino acids comprising CS3 at positions 1-4 of IL-4; or replacement of amino acids at positions 1-9 of IL-21 with amino acids at positions 1-11 or amino acids comprising CS3 at positions 1-11 of IL-4;
(ii) mutations in at least 1, 2, 3, 4, or 5 positions selected from the following, resulting in glycosylation at the positions: D4, R5, H6, M7, D15, V17, Q19, K21, D37, E39, R76, K77, P78, P79, S80, N82, G84, H120, and/or H122;
(iii) substitutions at 1-5 positions, e.g., 1 or 2 positions, selected from the following: 18, K72, K77, P78, and/or P79, wherein the K can be substituted with D or E, the P can be substituted with E or A, or the I can be substituted with Q; and
(iv) a deletion of 1, 2, 3, 4, 5, 6, 7, 8, or 9 amino acids or a deletion of a segment comprising 1, 2, 3, 4, 5, 6, 7, 8, or 9 amino acids at the N-terminus of Helix A (e.g., positions 1-15), CD loop (e.g., positions 82-95 or 84-91), or C loop (e.g., positions 76-81);
wherein the amino acid positions are amino acid positions numbered according to SEQ ID NO: 74.

2. The mutant protein according to claim 1, wherein the amino acids at positions 1-15 of the N-terminus of IL-4 used for substitution in the mutation (i) are HKSDITLQEIIKTLN or HKCDITLQEIIKTLN;
more preferably, the mutation in the mutation (i) is selected from:
replacement of 1-5 (QGQDR) of IL-21 with 1-5 & C3S (HKSDI) of IL-4;
replacement of 1-9 (QGQDRHMIR) of IL-21 with 1-9 & C3S (HKSDITLQE) of IL-4;
replacement of 1-15 (QGQDRHMIRMRQLID) of IL-21 with 1-15 & C3S (HKSDITLQEIIKTLN) of IL-4;
replacement of 1-12 (QGQDRHMIRMRQ) of IL-21 with 1-12 & C3S (HKSDITLQEIIK) of IL-4;
replacement of 1-11 (QGQDRHMIRMR) of IL-21 with 1-11 & C3S (HKSDITLQEII) of IL-4; and
replacement of 1-9 (QGQDRHMIR) of IL-21 with 1-11 & C3S (HKSDITLQEII) of IL-4.

3. The IL-21 mutant protein according to claim 1, wherein the mutation in the mutation (ii) is substitutions of amino acids at the positions with N or T or S; preferably, the mutation (ii) comprises 1, 2, 3, 4, or 5 substitutions selected from the following:
D4N, R5N, H6T, M7T, D15N, V17T, Q19N, K21T, D37N, E39T, R76N, K77N, P78T/S, P79T/N, S80N, N82T, G82T, G84T, H120N, and/or H122T;
more preferably, the mutation (ii) is selected from a substitution or a combination of substitutions at the following position:
D4 & H6;
R5 & M7;
D15 & V17;
IQ19 & K21;
R76 & P78;
K77 & P78 & P79;
P79;
S80 & N82;
G84;
H120 & H122; or
D37 & E39;
and most preferably, the mutation (ii) is selected from the following substitutions or combinations of substitutions:
D4N & H6T;
R5N & M7T;
D15N & V17T;
IQ19N & K21T;
R76N & P78T;
K77N & P78S & P79T;
P79N;
S80N & N82T;
G84T;
H120N & H122T; and
D37N & E39T.

4. The IL-21 mutant protein according to claim 1, wherein the mutation in the mutation (iii) comprises 1-5, e.g., 1-2, of substitutions selected from the following:
I8Q, K72E, K77D, P78A, and/or P79E;
preferably, the mutation in the mutation (iii) is a substitution at the following position or combination of positions:
K72;
18 & P79; or
K77 & P78, wherein the K can be substituted with D or E, the P can be substituted with E or A, or the I can be substituted with Q;
preferably, the mutation in the mutation (iii) is selected from
K72E;
I8Q & P79E; and
K77D & P78A.

5. The IL-21 mutant protein according to claim 1, wherein the mutation in the mutation (iv) comprises a deletion of an amino acid segment at positions selected from the following: deletions at positions 1-9, positions 78-79, positions 85-87, and positions 84-91;
preferably, the mutation in the mutation (iv) is a deletion of an amino acid segment at the following position or combination of positions:
truncation 85-87;
truncation 78-79;
truncation 1-9;
truncation 84-91; or
truncation 1-9 & truncation 78-79;
preferably, the mutation in the mutation (iv) is selected from the following deletion of an amino acid segment:
truncation 85-87 (RRQ);
truncation 78-79 (PP);
truncation 1-9 (QGQDRHMIR);
truncation 84-91 (GRRQKHRL); or
truncation 1-9 (QGQDRHMIR) & truncation 78-79 (PP).

6. The IL-21 mutant protein according to claim 1, wherein the mutant protein comprises mutations selected from the following:
(i) truncation 1-9 (QGQDRHMIR) & Q19N & K21T;
(ii) truncation 84-91 (GRRQKHRL) & C42A & C93T & Q19N & K21T;
(iii) replacement of 1-9 (QGQDRHMIR) of IL-21 with 1-9 & C3S (HKSDITLQE) of IL-4 & truncation 78-79 (PP);
(iv) replacement of 1-9 (QGQDRHMIR) of IL-21 with 1-9 & C3S (HKSDITLQE) of IL-4 & K117N & I119T;
(v) replacement of 1-9 (QGQDRHMIR) of IL-21 with 1-9 & C3S (HKSDITLQE) of IL-4 & D37N & E39T;
(vi) replacement of 1-9 (QGQDRHMIR) of IL-21 with 1-9 & C3S (HKSDITLQE) of IL-4 & D15N & V17T; and
(vii) replacement of 1-5 (QGQDR) of IL-21 with 1-5 (HKCDI) of IL-4 & L123C.

7. The IL-21 mutant protein according to any one of claims 1-6, wherein the wild-type IL-21 comprises an amino acid sequence set forth in SEQ ID NO: 74 or SEQ ID NOs: 106-108, or an amino acid sequence having at least 95%-99% or more identity to the amino acid sequence or having no more than 1-10 or 1-5 amino acid conservative substitutions.

8. The IL-21 mutant protein according to any one of claims 1-7, wherein the mutant protein has one or more of the following improved properties compared to the wild-type protein:
(i) lower affinity for IL-21R;
(ii) higher stability;
(iii) improved druggability (e.g., longer half-life and/or improved purity, e.g., SEC purity); and/or
(iv) upon formation of a fusion protein with an antibody or an antigen binding fragment thereof directed against an antigen, increased selective activation of cells positive for the antigen.

9. The IL-21 mutant protein according to any one of claims 1-7, wherein the mutant protein comprises or consists of an amino acid sequence selected from SEQ ID NOs: 75-105, or comprises an amino acid sequence having at least 85%, 86%, 87%, 88%, or 89% identity, preferably 90% or more identity, preferably 95% but no more than 97%, and more preferably no more than 96% identity to the amino acid sequence selected from SEQ ID NOs: 75-105.

10. An IL-21 mutant protein fusion protein, comprising the IL2 mutant protein according to any one of claims 1-8.

11. The IL-21 mutant protein fusion protein according to claim 10, comprising the IL-21 mutant protein according to any one of claims 1-9 linked to an Fc fragment, or comprising the IL-21 mutant protein according to any one of claims 1-9 linked to an antibody or an antigen binding fragment thereof, wherein the linkage is achieved with or without a linker.

12. The mutant protein fusion protein according to claim 11, wherein the Fc fragment is human IgG Fc, e.g., human IgG1 Fc, human IgG2 Fc, or human IgG4 Fc, preferably, the Fc fragment is human IgG1 Fc, e.g., human IgG1 Fc comprising an L234A/L235A mutation, and more preferably, the Fc fragment comprises or consists of an amino acid sequence of SEQ ID NO: 70 or an amino acid sequence having at least 90% identity, e.g., 95%, 96%, 97%, 99% or more identity thereto.

13. The IL-21 mutant protein fusion protein according to claim 11, wherein an antigen against which the antibody is directed is PD-1, PD-L1, or PD-L2.

14. The IL-21 mutant protein fusion protein according to claim 12, wherein an antigen against which the antibody is directed is PD-1, and the antibody or the antigen binding fragment thereof comprises:
(1) three complementarity determining regions HCDR1, HCDR2, and HCDR3 comprised in a VH set forth in SEQ ID NO: 115, and three complementarity determining regions LCDR1, LCDR2, and LCDR3 comprised in a VL set forth in SEQ ID NO: 116; or
(2) HCDR1, HCDR2, and HCDR3 set forth in amino acid sequences of SEQ ID NOs: 109, 110, and 111, respectively, and LCDR1, LCDR2, and LCDR3 set forth in amino acid sequences of SEQ ID NOs: 112, 113, and 114, respectively.

15. The IL-21 mutant protein fusion protein according to claim 14, wherein the antibody or the antigen binding fragment thereof comprises:
a VH comprising or consisting of an amino acid sequence set forth in SEQ ID NO: 115 or an amino acid sequence having at least 90%, 91%, 92%, 93%, 94%, 95%, 96%, 97%, 98%, or 99% identity thereto, and a VL comprising or consisting of an amino acid sequence set forth in SEQ ID NO: 116 or an amino acid sequence having at least 90%, 91%, 92%, 93%, 94%, 95%, 96%, 97%, 98%, or 99% identity thereto.

16. The IL-21 mutant protein fusion protein according to any one of claims 10-12, comprising
chain A: the IL-21 mutant protein linked to the N-terminus of the Fc fragment via a linker or directly;
wherein preferably, the fusion protein comprises two identical chains A.

17. The mutant protein fusion protein according to claim 16, wherein the chain A comprises or consists of an amino acid sequence set forth in any one of SEQ ID NOs: 1-32.

18. The IL-21 mutant protein fusion protein according to any one of claims 10, 11, and 13-15, comprising the following structures:
chain A: a light chain of the antibody; and
chain B: the IL-21 mutant protein linked to the C-terminus of a heavy chain of the antibody;
wherein preferably, the mutant protein fusion protein comprises two identical chains A and two identical chains B.

19. The IL-21 mutant protein fusion protein according to claim 15, wherein chain A comprises an amino acid sequence set forth in SEQ ID NO: 34, or an amino acid sequence having at least 85%, 90%, 91%, 92%, 93%, 94%, 95%, 96%, 97%, 98%, 99%, or 100% identity thereto, and/or chain B comprises an amino acid sequence set forth in any one of SEQ ID NOs: 35-54, or an amino acid sequence having at least 85%, 90%, 91%, 92%, 93%, 94%, 95%, 96%, 97%, 98%, 99%, or 100% identity thereto.

20. The IL-21 mutant protein fusion protein according to any one of claims 10, 11, and 13-15, comprising the following structures:
chain A: a light chain of the antibody;
chain B1: the IL-21 mutant protein linked to the C-terminus of one heavy chain of the antibody; and
chain B2: a heavy chain of the antibody;
wherein preferably, the fusion protein comprises two identical chains A, one chain B1, and one chain B2; and preferably, the chain B1 comprises a knob mutation, e.g., S354C & T366W, and the chain B2 comprises a hole mutation, e.g., Y349C & T366S & L368A & Y407V.

21. The IL-21 mutant protein fusion protein according to claim 20, wherein the chain A comprises an amino acid sequence set forth in SEQ ID NO: 34, or an amino acid sequence having at least 85%, 90%, 91%, 92%, 93%, 94%, 95%, 96%, 97%, 98%, 99%, or 100% identity thereto; and/or the chain B1 comprises or consists of an amino acid sequence set forth in any one of SEQ ID NOs: 55-67, or an amino acid sequence having at least 85%, 90%, 91%, 92%, 93%, 94%, 95%, 96%, 97%, 98%, 99%, or 100% identity thereto; and/or the chain B2 comprises an amino acid sequence set forth in SEQ ID NO: 33, or an amino acid sequence having at least 85%, 90%, 91%, 92%, 93%, 94%, 95%, 96%, 97%, 98%, 99%, or 100% identity thereto.

22. The IL-21 mutant protein fusion protein according to any one of claims 10-21, wherein the linker comprises a linker sequence selected from the following: (GS)n, (GSGGS)n, (GGGGS)n, and (GGGS)n, wherein n is an integer of at least 1, preferably, the linker comprises (G4S)₂ or (G4S)₃.

23. An isolated polynucleotide, encoding a chain in the IL-21 mutant protein according to any one of claims 1-9 or the IL-21 mutant protein fusion protein according to any one of claims 10-22.

24. An expression vector, comprising the polynucleotide according to claim 23.

25. A host cell, comprising the polynucleotide according to claim 23 or the vector according to claim 24, wherein preferably, the host cell is a yeast cell or a mammalian cell, particularly an HEK293 cell or a CHO cell.

26. A method for producing the IL-21 mutant protein according to any one of claims 1-9 or the IL-21 mutant protein fusion protein according to any one of claims 10-22, comprising culturing the host cell according to claim 25 under conditions suitable for expression of the IL-21 mutant protein or the fusion protein.

27. A pharmaceutical composition, comprising the IL-21 mutant protein according to any one of claims 1-9 or the fusion protein according to any one of claims 10-12, and optionally a pharmaceutical auxiliary material.

28. Use of the IL-21 mutant protein according to any one of claims 1-9 or the fusion protein according to any one of claims 10-21 or the pharmaceutical composition according to claim 27 in preparing a medicament for the prevention and/or treatment of cancer, wherein preferably, the cancer is a solid tumor or a hematological tumor.

29. The use according to claim 28, wherein the pharmaceutical composition also comprises a second therapeutic agent.

30. A method for preventing and/or treating cancer in a subject, comprising administering to the subject the IL-21 mutant protein according to any one of claims 1-9 or the fusion protein according to any one of claims 10-21 or the pharmaceutical composition according to claim 27, wherein preferably, the cancer is a solid tumor or a hematological tumor.

31. The method according to claim 30, wherein the mutant protein, the fusion protein, or the pharmaceutical composition is administered in a combination therapy with a second therapeutic agent.
